# EUROPEAN PATENT APPLICATION

(11) **EP 4 428 238 A1**
(43) Date of publication of application: **11.09.2024**
(21) Application number: 22889979.5
(22) Date of filing: 02.11.2022
(51) Int. Cl.: C12N 15/113, C07K 14/00

(54) **LIGAND-BOUND NUCLEIC ACID COMPLEX**

(30) Priority: 02.11.2021 JP 2021179771
(71) Applicant: Rena Therapeutics Inc., Tokyo 100-0004 (JP)
(72) Inventor: TAKAGI Koh, Tokyo 100-0004 (JP); KIZAWA Hideki, Tokyo 100-0004 (JP); OKAMOTO Hiroshi, Tokyo 100-0004 (JP); KANEKO Keiko, Tokyo 100-0004 (JP); FUSE Nobuyuki, Tokyo 100-0004 (JP); OCHI Takashi, Tokyo 100-0004 (JP)
(74) Representative: J A Kemp LLP
(86) International application number: PCT/JP2022/040996
(87) International publication number: WO 2023/080159

(57) **Abstract**

This invention provides a PTH1 ligand-binding nucleic acid complex that is delivered to a target organ to regulate expression or editing of a target gene or a transcription or translation product thereof. The PTH1 ligand-binding nucleic acid complex in which a nucleic acid comprising an antisense strand comprising an oligonucleotide having a nucleotide sequence complementary to a target transcription product and consisting of 12 to 30 nucleotides is bound to a PTH1 ligand is delivered to an organ to regulate expression or editing of a target gene or a transcription or translation product thereof. Thus, such nucleic acid complex is useful for treatment of a disease caused by such gene. In addition, the ligand-binding nucleic acid complex can be delivered to an organ, tissue, or cell in which the PTH1 receptor is expressed, such as the kidney, bone, and subcutaneous fat tissue, or the vascular endothelial cells. Thus, such nucleic acid complex is useful for treatment of diseases in such organs.

## Description

### Technical Field

The present invention relates to a nucleic acid complex that regulates expression or editing of a target gene or a transcription or translation product thereof. More particularly, the present invention relates to a ligand-binding nucleic acid complex in which a ligand that can be delivered to a target organ, cell, or the like binds to a nucleic acid complex.

### Background Art

Unlike conventional low molecular medicine, nucleic acid medicine acts on the sequence of a transcription product of a disease-causing gene to regulate expression of the transcription product or a protein. Accordingly, development thereof as next-generation medicine has been in progress.

Examples of known nucleic acids that have heretofore been used for nucleic acid medicine include short-interfering nucleic acid (siNA), short-interfering RNA (siRNA), double-stranded RNA (dsRNA), micro-RNA (miRNA), a short-stranded hairpin RNA (shRNA) molecule that can mediate RNA interference (RNAi) to a target nucleic acid sequence, and chemically modified low molecular weight nucleic acids using the antisense technique, such as a single-stranded antisense oligonucleotide (ASO), an antisense double-stranded DNA oligonucleotide (ADO), a hetero-duplex oligonucleotide (HDO) consisting of an antisense strand of DNA and a complementary strand of RNA, and a single-stranded hetero-duplex oligonucleotide (ss-HDO).

HDO is composed of an antisense strand comprising a nucleic acid sequence capable of hybridizing to a target gene or target transcription product and a complementary strand, which is a nucleic acid strand complementary to the antisense strand (also referred to as a "sense strand"). A construct comprising an antisense strand annealed to a complementary strand is referred to as a "hetero-duplex oligonucleotide (HDO" (Patent Literature 1).

An ss-HDO construct is a single-stranded oligonucleotide when it is prepared, and such construct comprises an antisense strand consisting of a DNA nucleotide or DNA nucleotide analog, a linker sequence consisting of 3 to 10 nucleotides, and a sense strand consisting of an RNA nucleotide or RNA nucleotide analog complementary to the antisense strand. The single-stranded oligonucleotide consists of a X-L-Y structure (Patent Literature 2). The X-L-Y structure comprises a nucleotide X serving as an antisense strand, a nucleotide Y serving as a strand complementary to the antisense strand, and a nucleotide L serving as a linker. When such single-stranded oligonucleotide is used as a pharmaceutical composition, single-molecule annealing takes place between an antisense strand and a strand complementary to the antisense strand with a linker in physiological saline, a solvent used for an aqueous injection preparation, a non-aqueous injection preparation, a suspended injection preparation, or a solid injection preparation, the blood, or the plasma to form a double-stranded structure. When such nucleic acid complex acts as a pharmaceutical composition, it undergoes single-molecule annealing to form a double-stranded structure. Thus, this single-stranded oligonucleotide is a type of HDO.

As a technique of delivering a nucleic acid complex to a target organ, a technique of adding a ligand or the like that can be delivered to a target organ to the nucleic acid complex is known. Examples of a ligand or the like include molecules selected from among a lipid, a peptide, and a protein. A lipid can be selected from among cholesterol, fatty acid, fat-soluble vitamin, a glycolipid, and a glyceride. As a ligand or the like, it is also possible to select a ligand reacting with a receptor exposed on the target cell surface (Patent Literature 1).

When cholesterol is used as a ligand or the like, for example, a nucleic acid complex is integrated into a cell via an LDL receptor on a cell surface. Accordingly, a ligand or the like can deliver a nucleic acid complex to, in particular, the liver, which is the organ comprising cells with LDL receptors. However, many cells with LDL receptors are present in organs other than the liver. When an organ other than the liver is targeted, accordingly, a nucleic acid complex is delivered to other organs, disadvantageously. This necessitates examination of safety and side effects in other organs.

The ligand-binding nucleic acid complex used as nucleic acid medicine is required to deliver a nucleic acid complex of interest to an organ. In addition, the ligand-binding nucleic acid complex is required to be integrated into the organ tissue or a cell in the organ to exert antisense effects. When a nucleic acid molecule with a molecular weight larger than that of a ligand is bound, it is difficult to predict as to whether or not the ligand-binding nucleic acid complex would be integrated into a cell. It is accordingly difficult to attain the object with such technique. At present, ligand-binding nucleic acid complexes that can be delivered to various organs have not yet been sufficiently provided. At present, research and development of ligands that can be more specifically delivered to target organs have been continuously performed by bio-venture and pharmaceutical companies.

While the PTH1 receptor is distributed throughout the body, it is expressed at high levels in the kidney and the bone. To date, PTH1 type 1 to type 3 have been identified. The PTH1 receptor is a G-protein-coupled receptor. As *in vivo* ligands reacting with the PTH1 receptor, parathyroid hormone (PTH) and parathyroid hormone-related protein (PTHrP) are known.

PTH is a hormone secreted from the parathyroid gland, which is a peptide hormone consisting of 84 amino acids. PTH is involved in regulation of the calcium level in the blood, and such regulation is mostly performed in a system mediated by the PTH1 receptor of the kidney. While PTHrP is a 139- to 173-amino-acid protein with N-terminal homology to PTH, the N-terminus of PTHrP has high homology to that of PTH, and PTH and PTHrP bind to a common PTH receptor and act thereon. PTHrP is secreted from various cells in every organ, according to need, and physiological activity thereof primarily functions in a paracrine/autocrine fashion. Biological activity of PTH can be reproduced by the N-terminal fragment of 1 to 34 amino acids; i.e., hPTH (1 to 34) (Patent Literature 3). PTH (1 to 34) and PTHrP (1 to 34) are known to have two amphophilic alpha helical domains. PTH (1 to 34) and PTHrP (1 to 34) are known to have biological activity, such that they promote differentiation of osteoblastic progenitor cells or preosteoblasts and inhibit the apoptosis of osteoblasts, so as to increase the number of the osteoblasts and promote bone formation.

However, no attempt had been made to regulate expression or editing of a target gene or a transcription or translation product thereof with the aid of a nucleic acid molecule by binding a ligand reacting with the PTH1 receptor (hereafter referred to as the "PTH1 ligand") to a nucleic acid molecule that regulates expression or editing of a target gene or a transcription or translation product thereof, and integrating a nucleic acid molecule comprising an antisense strand to the target gene or a transcription product thereof into tissue or a cell using the PTH1 ligand so as to deliver the nucleic acid molecule to the organ, tissue, or cells.

### Citation List

### Patent Literature

Patent Literature 1: International Publication No. WO 2013/089283
Patent Literature 2: International Publication No. WO 2017/131124
Patent Literature 3: JP S62-28799 B (1987)

### Summary of Invention

### Technical Problem

The PTH1 receptor is a G-protein-coupled receptor (GPCR) and it has 7 transmembrane helices. While the PTH1 receptor is distributed throughout the body, it is likely to be expressed at high levels in, for example, organs and tissue, such as the kidney, bone, and subcutaneous fat tissue, and cells, such as the vascular endothelial cells. The PTH1 receptor is expressed at particularly high levels in the kidney among human and mouse organs.

The present invention is intended to deliver a ligand-binding nucleic acid complex to a subject's organ expressing the PTH1 receptor through the PTH1 receptor and regulate expression or editing of a target gene or a transcription or translation product thereof. Also, the present invention is intended to discover and obtain a ligand-binding nucleic acid complex that can be delivered to a subject's organ expressing the PTH1 receptor through the PTH1 receptor by intravenous or subcutaneous administration, which is a method of administration with less physical stress on a subject, and that can regulate expression or editing of a target gene or a transcription or translation product thereof.

The present inventors verified that a nucleic acid complex comprising, bound thereto, a ligand reacting with the PTH1 receptor expressed in the kidney and bone tissue of a human, mouse, or other subject would be delivered to an organ expressing the PTH1 receptor, so as to regulate expression or editing of a target gene or a transcription or translation product thereof. This has led to the completion of the present invention.

### Solution to Problem

The present inventors had conducted studies on a ligand reacting with the PTH1 receptor. As a result, they discovered that the PTH1 ligand-binding nucleic acid complex of the present invention would be delivered to a target organ and expression or editing of a target gene or a transcription or translation product thereof would be regulated. The ligand-binding nucleic acid complex of the present invention can be used for treatment of a disease in an organ comprising a cell expressing the PTH1 receptor.

Specifically, the present invention is as described below.
[1] A ligand-binding nucleic acid complex in which a nucleic acid molecule that regulates expression or editing of a target gene or a transcription or translation product thereof binds to a PTH1 ligand with or without a linker.
[2] The ligand-binding nucleic acid complex according to [1], wherein the PTH1 ligand is a peptide consisting of the amino acid sequence represented by the following formula:

[Chemical Formula 1] A₁-A₂-A₃-A₄-A₅-A₆-A₇-A₈-A₉-A₁₀-A₁₁-A₁₂-A₁₃-A₁₄-A₁₅-A₁₆-A₁₇-A₁₈-A₁₉-A₂₀-A₂₁-A₂₂-A₂₃-A₂₄-A₂₅-A₂₆-A₂₇-A₂₈-A₂₉-A₃₀-A₃₁-A₃₂-A₃₃-A₃₄
wherein the amino acid sequence is arranged in order from the N terminus toward the C terminus;
A1 represents Ala, Ser, or Dap or is deleted;
A2 represents Val or is deleted;
A3 represents Ser, Thr, or Aib or is deleted;
A4 represents Glu or is deleted;
A5 represents Leu, His, Nle, Ile, Cha, β-Nal, Trp, Pal, Ace, or Phep-X-Phe, wherein X represents OH, halogen, or CH3, or is deleted;
A6 represents Gin;
A7 represents Leu, Nle, Ile, Cha, β-Nal, Trp, Pal, Ace, or Phep-X-Phe, wherein X represents OH, halogen, or CH3, or is deleted;
A8 represents Met, Nva, Leu, Val, Ile, Cha, Ace, or Nle or is deleted;
A9 represents His or is deleted;
A10 represents Asp or Asn;
A11 represents Leu, Lys, Nle, Ile, Cha, β-Nal, Trp, Pal, Ace, Phe, or p-X-Phe, wherein X represents OH, halogen, or CH3;
A12 represents Gly, Ace, or Aib;
A13 represents Lys;
A14 represents Ser or His;
A15 represents Leu, Nle, Ile, Cha, β-Nal, Trp, Pal, Ace, Phe, or P-X-Phe, wherein X represents OH, halogen, or CH3;
A16 represents Ser, Gin, Asn, Ala, or Aib;
A17 represents Ser, Asp, Thr, or Aib;
A18 represents Met, Nva, Leu, Val, Ile, Nle, Ace, Cha, or Aib;
A19 represents Arg, Glu, or Aib;
A20 represents Arg;
A21 represents Arg, Val, Ace, Cha, or Met;
A22 represents Phe, Glu, Aib, Ace, or Cha;
A23 represents Phe, Trp, Leu, Lys, Ace, or Cha;
A24 represents Leu, Lys, Ace, or Cha;
A25 represents His, Arg, Lys, Aib, Ace, or Glu;
A26 represents His, Aib, Ace, or Lys;
A27 represents Lys, Aib, Leu, hArg, Gin, Ace, or Cha;
A28 represents Ile, Leu, Lys, Ace, or Cha;
A29 represents Ala, Glu, Ace, or Aib;
A30 represents Glu, Asp, Leu, Nle, Cha, Aib, Ace, or Lys;
A31 represents Ile, Val, Leu, Nle, Cha, Lys, or Acc;
A32 represents His;
A33 represents Thr, Asn, Lys, or Cys; and
A34 represents Phe, Ala, Tyr, Amp, or Aib.

[3] The ligand-binding nucleic acid complex according to [1], wherein the PTH1 ligand is a peptide consisting of a sequence selected from the group consisting of SEQ ID NO: 1 to SEQ ID NO: 168.
[4] The ligand-binding nucleic acid complex according to [2], wherein the PTH1 ligand is a peptide consisting of a sequence selected from the group consisting of SEQ ID NO: 1 to SEQ ID NO: 164.
[5] The ligand-binding nucleic acid complex according to [4], wherein the nucleic acid molecule binds to the PTH1 ligand with a linker.
[6] The ligand-binding nucleic acid complex according to [5], wherein the linker is a cleavable linker having a cleavable structure.
[7] The ligand-binding nucleic acid complex according to [5], wherein the linker has the structure shown below.

**[Table 1]**

| Linker type | Structural formula |
|---|---|
| Having a disulfide bond | 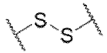 |
| Having an amide bond (n=0-9) | 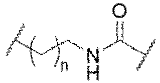 |
| Having a polyethylene glycol group (n=1-200) | 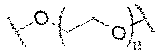 |
| Having a phosphate group | 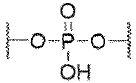 |
| Having a pyrrolidinyl group | 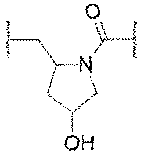 |
| Having a BCN group | 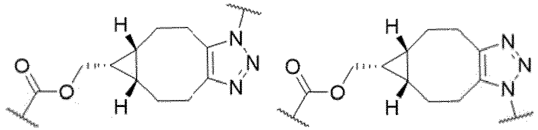 |
| Having a DBCO group | 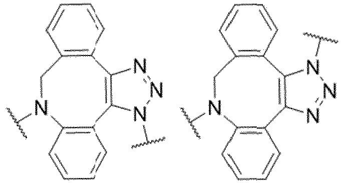 |
| Having a maleimide thioether group | 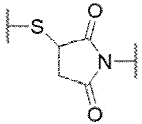 |

[8] The ligand-binding nucleic acid complex according to [1], wherein the nucleic acid molecule is selected from the group consisting of a nucleic acid molecule comprising an antisense strand consisting of an oligonucleotide having a nucleotide sequence complementary to a target gene or a transcription product thereof, an aptamer comprising a nucleotide sequence binding specifically to a target protein, and a decoy consisting of an oligonucleotide having a nucleic acid sequence complementary to a target transcription factor.
[9] The ligand-binding nucleic acid complex according to [1], wherein the nucleic acid molecule is selected from among ADO, ASO, HDO, and RNAi.
[10] The ligand-binding nucleic acid complex according to [9], wherein the antisense strand of the nucleic acid molecule consists of 12 to 30 continuous nucleotides.
[11] The ligand-binding nucleic acid complex according to [8], wherein the nucleic acid molecule is a decoy comprising a nucleic acid sequence complementary to a target transcription factor and consisting of 8 to 30 nucleotides.
[12] The ligand-binding nucleic acid complex according to [9], wherein the oligonucleotide is siRNA consisting of an antisense strand consisting of RNA and a nucleic acid strand complementary to the antisense strand.
[13] The ligand-binding nucleic acid complex according to [9], wherein the nucleic acid strand of the nucleic acid molecule comprises nucleotides, modified nucleotides, and/or nucleotide analogs.
[14] The ligand-binding nucleic acid complex according to [13], wherein the total number of nucleotides, modified nucleotides, and nucleotide analogs constituting the antisense strand of the nucleic acid molecule is 12 to 30.
[15] The ligand-binding nucleic acid complex according to [13], wherein the nucleic acid molecule is HDO and the total number of nucleotides, modified nucleotides, and nucleotide analogs constituting the antisense strand and that constituting the complementary strand of HDO are each 12 to 30.
[16] The ligand-binding nucleic acid complex according to [15], wherein the antisense strand is a gapmer and comprises a gap region comprising nucleotides and/or modified nucleotides and a wing region (or wing regions) comprising one or a plurality of nucleotide analogs and/or modified nucleotides provided on the 5' terminal side and/or 3' terminal side thereof.
[17] The ligand-binding nucleic acid complex according to [15], wherein the antisense strand is a non-gapmer and comprises nucleotides, modified nucleotides, and/or nucleotide analogs.
[18] The ligand-binding nucleic acid complex according to [17], wherein the complementary strand comprises nucleotides, modified nucleotides, and/or nucleotide analogs.
[19] The ligand-binding nucleic acid complex according to [16], wherein the complementary strand comprises a center region comprising nucleotides and/or modified nucleotides and a wing region (or wing regions) comprising one or a plurality of nucleotide analogs and/or modified nucleotides provided on the 5' terminal side and/or 3' terminal side thereof.
[20] The ligand-binding nucleic acid complex according to [13], wherein the modified nucleotides are nucleotides comprising a 2'-O-CH₃ group or a 2'-O-CH₂CH₂OCH₃ (MOE) group.
[21] The ligand-binding nucleic acid complex according to [13], wherein the nucleotide analogs include bridged nucleotides selected independently from the group consisting of LNA, cEt-BNA, amide BNA (AmNA), and cMOE-BNA.
[22] The ligand-binding nucleic acid complex according to [13], wherein the nucleotide analogs are selected independently from the group consisting ofPNA, GNA, TNA, cEt, tcDNA, morpholino nucleic acid, BNA, guanidine bridged nucleic acid (GuNA), and 2'-O,4'-C-spirocyclopropylene bridged nucleic acid (scpBNA).
[23] The ligand-binding nucleic acid complex according to [13], wherein at least one nucleotide or modified nucleotide in the nucleic acid molecule is phosphorothioated or boranophosphated.
[24] A ligand-binding nucleic acid complex comprising an antisense strand to lower an expression level of a target transcription product in a subject's organ expressing the PTH1 receptor and a PTH1 ligand to deliver the antisense strand to the subject's organ, wherein the antisense strand comprises a base sequence capable of hybridizing to at least a part of a target transcription product and having antisense effects on the target transcription product.
[25] The ligand-binding nucleic acid complex according to [24], wherein the nucleic acid complex is a double-stranded nucleic acid agent consisting of a first nucleic acid strand and a second nucleic acid strand, wherein the first nucleic acid strand comprises a base sequence capable of hybridizing to at least a part of a target transcription product and having antisense effects on the target transcription product, the second nucleic acid strand comprises a base sequence complementary to the first nucleic acid strand and binds to the PTH1 ligand, and the first nucleic acid strand is annealed to the second nucleic acid strand.
[26] The ligand-binding nucleic acid complex according to [25], which is used for intravenous administration.

The description incorporates the contents disclosed by JP Patent Application No. 2021-179771, based on which the priority of the present application claims.

### Advantageous Effects of Invention

The ligand-binding nucleic acid complex of the present invention can be used for treatment of diseases of organs comprising PTH1 receptor-expressing cells.

The ligand-binding nucleic acid complex of the present invention can be delivered in an organ- or cell-specific manner with the use of a PTH1 ligand, and expression or editing of a target gene or a transcription or translation product thereof can be regulated by the nucleic acid molecule. The nucleic acid complex targeting a disease-causing gene or a transcription or translation product thereof can be used as a therapeutic agent for a disease of a particular organ. Since the nucleic acid complex is likely to be delivered to the kidney expressing the PTH1 receptor at the highest level among various organs, the PTH1 ligand-binding nucleic acid complex can be used as a therapeutic agent for a kidney disease.

### Brief Description of Drawings

[Figure 1] Figure 1 shows changes in animal body weight between before and after administration of L001-HDO6.
[Figure 2A] Figure 2A shows changes in ALT activity in blood 3 days after administration of L001-HDO6 to mice.
[Figure 2B] Figure 2B shows changes in AST activity in blood 3 days after administration of L001-HDO6 to mice.
[Figure 3] Figure 3 shows changes in mMalat1 ncRNA expression levels in the mouse liver after administration of L001-HDO6.
[Figure 4] Figure 4 shows changes in mMalat1 ncRNA expression levels in the mouse kidney after administration of L001-HDO6.
[Figure 5] Figure 5 schematically shows fluorescence-labeled ASO and HDO used for the intrarenal distribution test.
[Figure 6] Figure 6 shows the immunostained images of the kidney obtained 10 minutes, 6 hours, 24 hours, and 72 hours after a single intravenous administration of L001-HDO6-Alexa488 in mice.
[Figure 7] Figure 7 shows changes in mMalat1 ncRNA expression levels in the mouse kidney after a single intravenous administration of HDO, L001-HDO6, L003-HDO6, L005-HDO6, and L010-HDO6.
[Figure 8] Figure 8 shows changes in mMalat1 ncRNA expression levels in the mouse kidney after subcutaneous administration of L031-HDO6 and L021-HDO6.
[Figure 9] Figure 9 shows changes in mMalat1 ncRNA expression levels caused by L021-HDO6, L003-HDO6, L005-HDO6, and L010-HDO6 observed by *in vitro* assays using the mPth1R-stably expressing cell line.
[Figure 10] Figure 10 shows changes in mMalat1 ncRNA expression levels caused by L021-HDO6 and L011-HDO6 observed by *in vitro* assays using the mPth1R-stably expressing cell line.
[Figure 11] Figure 11 shows changes in mMalat1 ncRNA expression levels caused by L031-HDO6 and L040-HDO6 observed by *in vitro* assays using the mPth1R-stably expressing cell line.
[Figure 12] Figure 12 shows changes in mMalat1 ncRNA expression levels caused by L089-HDO6 and L098-HDO6 observed by *in vitro* assays using the mPth1R-stably expressing cell line.
[Figure 13] Figure 13 shows changes in mMalat1 ncRNA expression levels caused by L165-HDO6 and L168-HDO6 observed by *in vitro* assays using the mPth1R-stably expressing cell line.
[Figure 14] Figure 14 shows changes in mMalat1 ncRNA expression levels caused by L001-ASO observed by *in vitro* assays using the mPth1R-stably expressing cell line.
[Figure 15] Figure 15 shows changes in Gapdh mRNA expression levels caused by L010-siRNA observed by *in vitro* assays using the mPth1R-stably expressing cell line.

### Description of Embodiments

The present invention relates to a ligand-binding nucleic acid complex in which a ligand binds to a nucleic acid complex. The ligand binds to the nucleic acid complex indirectly with a linker or directly without a linker.

### 1. Nucleic acid complex

In the present invention, a "nucleic acid complex" is a nucleic acid molecule that regulates expression or editing of a target gene or a transcription or translation product thereof. An example of the nucleic acid molecule is a nucleic acid molecule comprising a nucleic acid sequence complementary to that of a target gene or a transcription product thereof and having antisense activity. Specific examples of the nucleic acid molecules include a single-stranded antisense oligonucleotide (ASO), miRNA, anti-miR, RNA interference (RNAi), short interference RNA (siRNA), short hairpin RNA (shRNA), antisense double-stranded DNA oligonucleotide (ADO), and a hetero-duplex oligonucleotide (HDO).

Another example of the nucleic acid molecule is an aptamer having high specificity and high binding affinity to a target molecule, such as a protein. Another example of the nucleic acid molecule is a decoy. A decoy acts as follows. When a transcription factor binds to a binding site of a particular transcription regulator, such as a promoter of a gene, the gene is activated and gene functions are turned on or off by the transcription regulator under ordinary circumstances. However, a decoy hybridizes to the transcription factor and inhibits inherent functions of the transcription factor. A further example of the nucleic acid molecule is a nucleic acid molecule that binds specifically to a particular target molecule in a cell, which is a bait that modifies functions of the target molecule.

A "target gene" is a gene to which the antisense strand of the nucleic acid complex of the present invention can bind.

A target gene is not particularly limited, provided that it is expressed *in vivo.* An example of a target gene is an organism-derived gene into which the nucleic acid complex of the present invention is to be introduced, such as a gene, the expression level of which is elevated in the case of various diseases. Examples thereof include the Indian Hedgehog gene, the interferon gene, the apolipoprotein B gene, the Huntington's gene, the dystrophin gene, and the dystrophia myotonica-protein kinase (DMPK) gene.

The Indian Hedgehog (IHH) is a secretory protein of the hedgehog family. It is known to be located downstream of the transcription factor TAZ and worsen NASH fibrosis.

The cytokine interleukin 1 (IL-1) gene is known to cause diseases of chronic inflammation. A treatment strategy in which skipping of exon 9 encoding the transmembrane domain of IL-1RAcP, which is pre-messenger RNA (mRNA), aimed at substantial inhibition of IL-1 signal transmission has been reported (Mol. Ther. Nucleic Acids, 2013, Jan, 22 (1): e66.doi:10.1038/mtna.2012.58.).

The apolipoprotein B gene, ApoB-100, is known to cause an inherited metabolic disorder; i.e., familial hypercholesterolemia, and nucleic acid medicine (mipomersen) was commercialized in 2013 in U.S.A.

Huntington's disease is inherited in an autosomal dominant manner, and it is a chronic progressive neurodegenerative disease with presenting symptoms characterized by involuntary choreic movement, mental symptoms, and dementia. The short arm of chromosome 4 (4p16.3) of the Huntington gene is known to be a causal gene of Huntington's disease.

Duchenne muscular dystrophy is muscular dystrophy caused by mutations in the dystrophin gene, and it is known to be inherited in an X-linked recessive manner and predominantly found in male children. Exon skipping comprising skipping over exons with abnormalities in the dystrophin gene is effective for treatment of Duchenne muscular dystrophy.

The DMPK gene encodes myotonin protein kinase, and it is known as a causal gene of myotonic dystrophy that is developed most frequently in adults among various types of muscular dystrophies.

The COL4A3, COL4A4, or COL4A5 gene encodes the α3, α4, or α5 strand of type IV collagen constituting the basement membrane. When any of the COL4A3, COL4A4, or COL4A5 gene is mutated, the Alport syndrome occurs. The Alport syndrome involves, in addition to renal disorders, auditory disorders and eye complications, and it is known to often lead to end-stage renal failure.

The "target transcription product" is any RNA that serves as a direct target of the nucleic acid complex of the present invention and is synthesized by RNA polymerase. The "transcription product of the target gene" is equivalent to the "target transcription product." Specific examples include mRNA transcribed from the target gene (including mature mRNA, an mRNA precursor, and RNA with unmodified bases), non-coding RNA (ncRNA) such as miRNA, long non-coding RNA (lncRNA), and natural antisense RNA. Examples of target transcription products include pre-mRNA, which is a transcription product of the IL-1RAcP gene, mRNA, which is a transcription product of the ApoB-100 gene, mRNA, which is a transcription product of the IHH gene, pre-mRNA, which is a transcription product of the dystrophin gene, DMPK mRNA, which is a transcription product of the DMPK gene, and metastasis associated lung adenocarcinoma transcript 1 (Malat1) non-coding RNA (ncRNA).

Malat1 is a long-chain non-coding RNA (lncRNA) expressed at high levels in the case of malignant tumors including lung cancer, and it is known to remain in the nuclei of muscle cells.

The "target translation product" is a protein that is synthesized by catalyzing a reaction in which, among mRNAs as transcription products, mRNA other than non-coding RNA is used as a template, and ribosomes ligate amino acids delivered by transfer RNA in accordance with codons to form a peptide chain. The "translation product of the target gene" and the "translation product of the target transcription product" are equivalent to the "target translation product."

The "aptamer" is a nucleic acid molecule that binds specifically to a target translation product, such as a particular target molecule in a cell, on a cell membrane, or outside a cell, such as on a cell membrane or outside a cell. The aptamer is either the DNA or RNA aptamer, and it can be prepared by a method known in the art, such as *in vitro* selection performed with the use of the systematic evolution of ligands by exponential enrichment (SELEX) procedure. The nucleic acid base length of an aptamer is not particularly limited, and it is in the range of 10 to 70 bases and preferably in the range of 20 to 50 bases.

The "decoy" is a nucleic acid having the sequence of the binding site of a transcription factor (e.g., NF-kB) or a sequence similar thereto. When a "decoy" is introduced into a cell, it inhibits the action of the transcription factor (e.g., a decoy inhibits transcription when a transcription factor is a transcription activator and it promotes transcription when a transcription factor is a transcription inhibitor). A decoy nucleic acid can be easily designed based on information on a binding sequence of a target transcription factor. The base length of the decoy nucleic acid is not particularly limited, and it is in the range of 8 to 30 bases and preferably in the range of 10 to 25 bases.

The "nucleic acid" or "nucleic acid molecule" is a nucleoside or nucleotide in the case of a monomer, an oligonucleotide in the case of an oligomer, and a polynucleotide in the case of a polymer. The term "nucleic acid strand" is used to indicate an "oligonucleotide" herein. A nucleic acid strand may be prepared entirely or partially by a method of chemical synthesis performed with the use of an autosynthesizer, or it may be prepared by enzymatic treatment with the use of a polymerase, a ligase, or a restriction enzyme, although a method of preparation is not limited thereto.

The "nucleoside" is a molecule composed of bases and sugars, in general. A sugar portion of a nucleoside is not limited. In general, it is composed of pentofuranosyl sugars. Specific examples thereof include ribose and deoxyribose. In general, a base portion (nucleic acid bases) of a nucleoside is a heterocyclic base portion. Examples thereof include, but are not limited to, adenine, cytosine, guanine, thymine, uracil, and other modified nucleic acid bases (modified bases).

The "nucleotide" is a molecule composed of a sugar portion of the nucleoside comprising a phosphoric acid group covalently bound thereto. In the case of a nucleotide comprising pentofuranosyl sugar, in general, a phosphoric acid group is ligated to a hydroxyl group at position 2', 3', or 5' of the sugar.

The "oligonucleotide" is a linear oligomer formed by covalent binding of several to dozens of hydroxyl groups and phosphoric acid groups in sugar portions between adjacent nucleotides. The "polynucleotide" is a polymer comprising the number of nucleotides that is larger than the number of nucleotides constituting an oligonucleotide, and it is formed by covalent binding of dozens or more, and preferably hundreds or more nucleotides. In general, phosphoric acid groups seem to form the internucleoside bond in the oligonucleotide or polynucleotide structure.

The "antisense technique" regulates the amount, activity, and/or functions of a target nucleic acid with the use of a nucleic acid molecule having an antisense strand. The "antisense technique" that utilizes an antisense strand is characterized in that a nucleic acid molecule having an antisense strand hybridizes to a target nucleic acid and regulates the amount, activity, and/or functions of the target nucleic acid. In some embodiments, for example, a nucleic acid molecule having an antisense strand changes transcription or translation of the target. Such expression can be regulated by, for example, target mRNA degradation or occupancy-based inhibition. An example of regulation of RNA target functions by degradation is degradation of target RNA by ribonuclease (RNase) at the time of hybridization to a nucleic acid molecule having a DNA-like antisense strand. An antisense strand (ASO) consisting of DNA constituted to be hybridize to target RNA hybridizes to target RNA to form a double strand, and it is then degraded by RNase. By repeating such procedure, target RNA is decreased in a cell, target RNA expression is inhibited, and target RNA activity is inhibited. Antisense effects thus attained are referred to as "RNase-dependent antisense effects." In some embodiments, a nucleic acid molecule having an antisense strand exerts, as RNase-independent antisense effects, effects of converting splicing functions, such as inhibition of transcription or translation or exon skipping of target RNA. In such a case, ASO hybridizes to target RNA to inhibit or promote expression of the target gene. Another example of RNase-independent antisense effects is RNA interference (RNAi). RNAi is antisense-mediated gene silencing that uses an RNA-induced silencing complex (RISC). Examples of RNAi include siRNA, shRNA, and miRNA. Such antisense effects are referred to as "RNAi-dependent antisense effects."

The "antisense effects" are effects of regulating expression or editing of a target gene or a transcription product thereof attained when an antisense strand of a nucleic acid molecule hybridizes to the target gene or a transcription product thereof (an RNA sense strand).

A nucleic acid molecule "that regulates expression or editing of a target gene or a transcription or translation product thereof" inhibits, lowers, or enhances expression of the target gene or the expression level of the target transcription product ("the expression level of the target transcription product" is often referred to as "the target transcription product level" herein), inhibits translation, inhibits function of the translation product, controls RNA splicing (e.g., splicing switch, exon inclusion, and exon skipping), or inhibits degradation of the transcription product or binding of the target gene to a protein.

In some embodiments, the term "antisense effects" refers to effects of converting splicing functions, such as inhibition of translation or exon skipping that can occur by covering the transcription product by hybridization, and/or the inhibition that can occur upon degradation of the transcription product when the hybridized region is recognized. Upon exon inclusion into a target gene or a transcription product, some exons are excluded from mRNA due to gene abnormalities, unlike the case of exon skipping, such exons are included into mRNA by the antisense activity, such inclusion leads to enhanced expression of normal mRNA, and such enhanced expression is referred to as "antisense effects" in other embodiments.

In the case of post-transcriptional inhibition of the target gene, for example, an RNA oligonucleotide is introduced into a cell as ASO, and ASO then forms a partial double strand by annealing to mRNA, which is a transcription product of a target gene. This partial double strand serves as a cover to block translation by a ribosome, and expression of the target protein encoded by the target gene is inhibited at the translation level (steric blocking). When an oligonucleotide comprising DNA is introduced into a cell as ASO, a partial DNA-RNA hetero-duplex is formed. When the hetero-duplex structure is recognized by RNase, mRNA of the target gene is degraded, and expression of the protein encoded by the target gene is inhibited at the expression level. The antisense effects can also be attained by targeting the intron in the mRNA precursor. The antisense effects can also be attained by targeting miRNA. In such a case, inhibition of miRNA functions would lead to enhanced expression of a gene that is generally regulated to express by the miRNA. In an embodiment, regulation of expression of the target transcription product may be reduction in the amount of the target transcription product.

When ADO is used as a nucleic acid molecule having an antisense strand, for example, a DNA double strand is cleaved by DNA nuclease (DNase) in a cell, the DNA antisense strand hybridizes to target RNA to form a double strand, and the target RNA is then degraded by RNase. By repeating such procedure, expression of the target RNA is inhibited, and action of the target RNA is inhibited. In another embodiment, a DNA double strand of ADO is cleaved by DNA nuclease (DNase), the DNA antisense strand hybridizes to target RNA, and expression of the target gene is inhibited or enhanced by regulation of RNA splicing, such as inhibition of transcription or translation or exon skipping of the target RNA.

When HDO is used as a nucleic acid molecule having an antisense strand, for example, a complementary strand consisting of RNA of HDO is cleaved by RNase in a cell, the DNA antisense strand hybridizes to target RNA to form a double strand, and target RNA is then degraded by RNase. By repeating such procedure, expression of the target RNA is inhibited, and action of the target RNA is inhibited. Alternatively, an RNA complementary strand of HDO is cleaved by RNase in a cell, the DNA antisense strand hybridizes to target RNA, and expression of the target gene is inhibited or enhanced by regulation of RNA splicing, such as inhibition of transcription or translation or exon skipping of the target RNA.

When RNA interference (RNAi) is used as a nucleic acid molecule having an antisense strand, for example, RNAi is antisense-mediated gene silencing based on the mechanism involving the use of an RNA-induced silencing complex (RISC). Examples of RNAi include siRNA and shRNA. In another embodiment, functions of target RNA are regulated by the occupancy-based mechanism, such as a mechanism that is naturally used by microRNA. microRNA is small non-coding RNA that regulates expression of RNA that encodes a protein. When a nucleic acid molecule having an antisense strand binds to microRNA, binding of the microRNA to the messenger RNA target is inhibited, and functions of microRNA are thus interfered. A microRNA mimic can enhance inherent microRNA functions. A nucleic acid molecule having a particular antisense strand changes pre-mRNA splicing. Regardless of a particular mechanism, sequence specificity enables the use of a nucleic acid molecule having an antisense strand as a tool for target examination and gene functionalization and as a therapeutic agent that selectively regulates expression of a gene related to a cause of the disease.

The length of an antisense strand is not particularly limited, and an antisense strand comprises at least 8 bases, such as 8 to 40 bases, preferably 12 o 30 bases, and more preferably 12 to 25 or 13 to 20 bases. In some embodiments, a chain length is selected in accordance with other factors, such as the level of antisense effects of the nucleic acid strand on the target, the cost, and the synthetic yield, in general. In the case of a double-stranded nucleic acid, a chain length may be selected to adjust Tm of the double strand preferably to 50°C or higher, and more preferably to 60°C or higher.

In the case of a double-stranded nucleic acid, the length of a complementary strand may be the same as that of the antisense strand. In such a case, the complementary strand comprises at least 8 bases, such as 8 to 40 bases, preferably 12 o 30 bases, and more preferably 12 to 25 or 13 to 20 bases. The complementary strand may be longer or shorter than the antisense nucleic acid strand by several to dozen nucleotides.

The term "complementary" or "complementarity" used herein refers to a correlation such that a so-called Watson-click base pairing (natural base pairing) or non-Watson-click base pairing (Hoogsteen base pairing or the like) can be formed via hydrogen bond. When a sufficient number of nucleic acid bases in an antisense strand can form hydrogen bonds with corresponding nucleic acid bases in the target gene or target transcription product, desirable effects are achieved because the antisense strand is complementary to the target gene or target transcription product. Nucleic acid bases that are noncomplementary between the antisense strand and the target gene or target transcription product are acceptable, provided that the antisense strand can specifically hybridize to the target nucleic acid. In addition, an antisense strand can hybridize to one or more segments of the target gene or target transcription product. Thus, intervening or adjacent segments are not involved in hybridization events (e.g., a loop, mismatch, or hairpin structure). The antisense strand is complementary to the sequence of the target gene or target transcription product. At a sufficient extent of complementarity, an antisense strand can bind to the target gene or target transcription product. For example, an extent of complementarity may be 80% or higher, 90% or higher, 95% or higher, 96% or higher, 97% or higher, 98% or higher, or 99% or higher. An extent of complementarity may be 100%. There may be approximately 0 to 4 mismatches.

In a particular embodiment, "the nucleic acid complex" of the present invention may be a single-stranded oligonucleotide when it is prepared, and the nucleic acid complex may comprise an antisense strand consisting of a DNA nucleotide or DNA nucleotide analog, a linker sequence consisting of 3 to 10 nucleotides, and a sense strand consisting of an RNA nucleotide or RNA nucleotide analog complementary to the antisense strand. The nucleic acid complex as described above is referred to as a single-stranded hetero-duplex oligonucleotide (ss-HDO), which is an oligonucleotide consisting of a X-L-Y structure (Patent Literature 4). The X-L-Y structure comprises a nucleotide X serving as an antisense strand, a nucleotide Y serving as a strand complementary to the antisense strand, and a nucleotide L serving as a linker. When such single-stranded oligonucleotide is used as a pharmaceutical composition, single-molecule annealing takes place between an antisense strand and a strand complementary to the antisense strand with a linker in physiological saline, a solvent used for an aqueous injection preparation, a non-aqueous injection preparation, a suspended injection preparation, or a solid injection preparation, the blood, or the plasma to form a double-stranded structure. When such nucleic acid complex acts as a pharmaceutical composition, it undergoes single-molecule annealing to form a double-stranded structure. Thus, it is a duplex nucleic acid complex.

In the present invention, the nucleic acid complex as described above may be referred to as a "nucleic acid molecule."

In several embodiments described above, preferable representative examples of single-stranded ASOs and duplex nucleic acid complexes were described. It should be noted that single-stranded ASOs and duplex nucleic acid complexes in several embodiments are not limited to the representative examples above.

In an embodiment, an antisense strand comprises nucleotides, modified nucleotides, and/or nucleotide analogs. The antisense strand may comprise DNA nucleotides and RNA nucleotides, and the nucleic acid strand may further optionally comprise modified nucleotides and nucleotide analogs.

In an embodiment, a complementary strand comprises nucleotides, modified nucleotides, and/or nucleotide analogs.

This indicates that the complementary strand may comprise DNA nucleotides and RNA nucleotides, and the nucleic acid strand may further optionally comprise modified nucleotides and nucleotide analogs.

In an embodiment, a complementary strand comprises a center region comprising nucleotides and/or modified nucleotides and a wing region (or wing regions) comprising one or a plurality of nucleotide analogs and/or modified nucleotides provided on the 5' terminal side and/or 3' terminal side thereof.

The term "DNA nucleotide" used herein refers to a DNA nucleotide that exists in nature or a DNA nucleotide with a modified base, sugar, or phosphate binding subunit.

In the same manner, the term "RNA nucleotide" used herein refers to an RNA nucleotide that exists in nature or an RNA nucleotide with a modified base, sugar, or phosphate binding subunit.

In a "modified nucleotide," a substituent is added to a base, sugar, or phosphate binding subunit of the nucleotide, or one substitution is implemented in a subunit, and the entire subunit is not substituted with different chemical groups. From the viewpoint of high resistance to a DNA-degrading enzyme or the like, DNAs in the entire region comprising nucleotides or a part thereof may be modified nucleotides. Examples of modification include: 5-methylation, 5-fluorination, 5-bromation, 5-iodization, and N4-methylation of cytosine; 5-demethylation, 5-fluorination, 5-bromation, and 5-iodization of thymidine; N6-methylation and 8-bromation of adenine; N2-methylation and 8-bromation of guanine; phosphorothioation, boranophosphatation, methylphosphonation, methylthiophosphonation, chiral-methylphosphonation, phosphorodithioation, phosphoroamidation, 2'-O-methylation, 2'-methoxyethylation (MOE), 2'-aminopropylation (AP), and 2'-fluorination. From the viewpoint of excellent dynamics, phosphorothioation is preferable. Such modification may be provided in combinations of two or more on the same DNA. As described below, an RNA nucleotide may be modified to exert similar effects.

In some embodiments, the number or position of modified nucleotide(s) may affect the antisense effects exerted by the duplex oligonucleotide disclosed herein. While such embodiments vary depending on target gene sequences or other factors, a person skilled in the art may be able to determine the number or position of modified nucleotide(s) with reference to the literatures concerning the antisense method described below. When the assayed antisense effects of the modified duplex nucleic acid complex are not significantly lowered compared with those of the duplex nucleic acid complex before modification (e.g., the assayed value for the modified duplex nucleic acid complex is 30% or more of the assayed value for the duplex nucleic acid complex before modification), such modification can be evaluated effective. The antisense effects can be adequately assayed in the manner described below. For example, a test nucleic acid compound is introduced into a cell, and the target gene expression level (e.g., the mRNA level, the cDNA level, or the protein level) in the cell that is suppressed by the antisense effects exerted by the test nucleic acid compound is then assayed via a conventional technique as described in the examples below, such as Northern blotting, quantitative PCR, or Western blotting.

The "nucleotide analog" is a nucleotide that does not exist in nature. In a base, sugar, or phosphate binding subunit of a nucleotide, two or more substituents are added, two or more substituents in the subunit are substituted, or the entire subunit is substituted with different chemical groups. An example of an analog involving substitution of two or more substituents is a bridged nucleic acid. A bridged nucleic acid is a nucleotide analog comprising a crosslinking unit added thereto on the basis of substitution at 2 sites in a sugar ring. A typical example is a nucleotide analog in which carbon at position 2' is bound to carbon at position 4'. In an embodiment, the first nucleic acid strand further comprises a nucleotide analog to enhance the affinity to a partial sequence of a target gene transcription product and/or resistance to a nucleic acid-degrading enzyme. A "nucleotide analog" may be any nucleotide, provided that its affinity to a partial sequence of a target gene transcription product and/or resistance to a nucleic acid-degrading enzyme are enhanced via modification (e.g., crosslinking or substitution). Examples thereof that are preferably used in the antisense method are disclosed in JP H10-304889 A, WO 2005/021570, JP H10-195098 A, JP 2002-521310 A, WO 2007/143315, WO 2008/043753, WO 2008/029619, and WO 2008/049085 (hereafter, such literatures are also referred to as "the literatures concerning the antisense method"). Specific examples include the nucleic acids disclosed in the literatures mentioned above: hexitol nucleic acid (HNA), cyclohexene nucleic acid (CeNA), peptide nucleic acid (PNA), glycol nucleic acid (GNA), threose nucleic acid (TNA), morpholinonucleic acid, tricyclo-DNA (tcDNA), 2'-O-methylated nucleic acid (2'-OMe), 2'-O-methoxyethylated nucleic acid (2'-MOE), 2'-O-ethylated nucleic acid (cEt), 2'-O-aminopropylated nucleic acid (2'-AP), 2'-fluorinated nucleic acid, 2'F-arabinonucleic acid (2'-F-ANA), and bridged nucleic acid (BNA).

In an embodiment, BNA may be a ribonucleotide or deoxyribonucleotide in which carbon at position 2' is bridged with carbon at position 4' with the aid of two or more atoms. Examples of bridged nucleic acids are known in the art. An example of a BNA subgroup is BNA in which carbon at position 2' is bridged with carbon at position 4' with the aid of 4'-(CH₂)ₚ-O-2', 4'-(CH₂)ₚ-S-2', 4'-(CH₂)ₚ-OCO-2', and 4'-(CH₂)ₙ-N(R₃)-O-(CH₂)ₘ-2' (wherein p, m, and n are each an integer of 1 to 4, 0 to 2, and 1 to 3; and R₃ represents a hydrogen atom, an alkyl group, an alkenyl group, a cycloalkyl group, an aryl group, an aralkyl group, an acyl group, a sulfonyl group, and a unit substituent (e.g., a fluorescence or chemiluminescence label molecule, a functional group having nucleic acid cleavage activity, or intracellular or nuclear transfer signal peptide)). In an embodiment, BNA comprises a substituent of carbon at position 3' (OR₂) and a substituent of carbon at position 5' (OR₁), wherein OR₁ and OR₂ are typically hydrogen atoms, OR₁ and OR₂ may be the same with or different from each other, and OR₁ and OR₂ may be each a hydroxyl protective group in nucleic acid synthesis, an alkyl group, an alkenyl group, a cycloalkyl group, an aryl group, an aralkyl group, an acyl group, a sulfonyl group, a silyl group, a phosphoric acid group, a phosphoric acid group protected with a protective group of nucleic acid synthesis, or -P(R₄)R₅ (wherein R₄ and R₅ may be the same with or different from each other and R₄ and R₅ each represent a hydroxyl group, a hydroxyl group protected with a protective group of nucleic acid synthesis, a mercapto group, a mercapto group protected with a protective group of nucleic acid synthesis, an amino group, an alkoxy group having 1 to 5 carbon atoms, an alkylthio group having 1 to 5 carbon atoms, a cyanoalkoxy group having 1 to 6 carbon atoms, or an amino group substituted with an alkyl group having 1 to 5 carbon atoms). Examples of BNAs include α-L-methyleneoxy(4'-CH₂-O-2')BNA or β-D-methyleneoxy(4'-CH₂-O-2')BNA also referred to as LNA^{®} (a locked nucleic acid, 2',4'-BNA), ethyleneoxy(4'-(CH₂)₂-O-2')BNA, β-D-thio(4'-CH₂-S-2')BNA, and aminoxy(4'-CH₂-O-N(R₃)-2')BNA also referred to as ENA, oxyamino(4'-CH₂-N(R₃)-O-2')BNA, 2',4'-BNACOC, 3' amino-2',4'-BNA, and 5'-methyl BNA also referred to as 2',4'-BNANC, (4'-CH(CH₃)-O-2')BNA also referred to as cEt-BNA, (4'-CH(CH₂OCH₃)-O-2')BNA also referred to as cMOE-BNA, amide BNA(4'-C(O)-N(R)-2')BNA (R = H, Me) also referred to as AmNA, guanidine bridged nucleic acid (GuNA), 4'-C-spirocyclopropylene bridged nucleic acid (scpBNA), and other BNAs known in the art.

A modified nucleic acid according to an embodiment may be modified at its base site. Examples of modification at its base site include: 5-methylation, 5-fluorination, 5-bromation, 5-iodization, and N4-methylation of cytosine; 5-demethylation, 5-fluorination, 5-bromation, and 5-iodization of thymidine; N6-methylation and 8-bromation of adenine; and N2-methylation and 8-bromation of guanine. A modified nucleic acid according to another embodiment may be modified at its phosphodiester binding site. Examples of modification at the phosphodiester binding site include phosphorothioation, boranophosphatation, methylphosphonation, methylthiophosphonation, chiral-methylphosphonation, phosphorodithioation, and phosphoroamidation. From the viewpoint of excellent dynamics, phosphorothioation is adopted. Such modification at a base site or that at a phosphodiester binding site may be provided in combinations of two or more on the same nucleic acid.

In general, modified nucleotides and nucleotide analogs are not limited to those exemplified herein. Many modified nucleotides and nucleotide analogs are known in the art. For example, the description of US Patent No. 8299039 of Tachas et al., in particular, the description in the sections 17 to 22, can be adopted as the embodiments of the present application.

A person skilled in the art can adequately select nucleotide analogs from among such modified nucleic acids in view of antisense effects, affinity to a partial sequence of the target gene transcription product, resistance to a nucleic acid degrading enzyme, and other conditions as nucleic acids constituting the nucleic acid complex. In an embodiment, a nucleotide analog is LNA.

In an embodiment, an antisense strand is composed of a region comprising a plurality of DNA nucleotides (hereafter, may be referred to as a "DNA gap region") and a wing region (or wing regions) comprising one or a plurality of nucleotide analogs provided on the 5' terminal side and/or 3' terminal side thereof. This antisense strand is also referred to as a "Gapmer." The Gapmer comprises at least 8 bases, such as 8 to 40 bases, preferably 12 to 30 bases, and more preferably 12 to 25 or 13 to 20 bases.

In an embodiment, a plurality of DNA nucleotides may be modified nucleotides.

In another embodiment, a plurality of DNA nucleotides may be nucleotide analogs.

A region comprising a nucleotide analog provided at the 5' terminus of the DNA gap region (hereafter, it may be referred to as a "5' wing region") and a region comprising a nucleotide analog provided at the 3' terminus of the DNA gap region (hereafter, it may be referred to as a "3' wing region") are independently of each other, it would be sufficient if such region comprises at least one nucleotide analog mentioned in the literature concerning the antisense method, and such region may further comprise a naturally-occurring nucleic acid (DNA or RNA) or modified nucleotide other than the nucleotide analog. The 5' wing region and the 3' wing region each generally comprise 1 to 10 bases, 1 to 7 bases, or 2 to 5 bases.

In an embodiment, an antisense strand is not a Gapmer, but it is composed of a plurality of DNA nucleotides, modified nucleotides, or nucleotide analogs or two or more thereof in combination. This antisense strand is also referred to as a "Non-Gapmer." The Non-Gapmer comprises at least 8 bases, such as 8 to 40 bases, preferably 12 to 30 bases, and more preferably 12 to 25 or 13 to 20 bases.

In an embodiment, a complementary strand of a duplex nucleic acid complex may be a Gapmer composed of a region comprising a plurality of DNA nucleotides or RNA nucleotides and a wing region (or wing regions) comprising one or a plurality of modified nucleotides and/or nucleotide analogs provided on the 5' terminal side and/or 3' terminal side thereof. The 5' or 3' wing region composed of modified nucleotides and/or nucleotide analogs can more strongly bind to an antisense strand.

In an embodiment, a complementary strand of a duplex nucleic acid complex may comprise a plurality of DNA nucleotides and/or modified nucleotides or nucleotide analogs, or it may comprise RNA nucleotides and/or modified nucleotides or nucleotide analogs. Further, a complementary strand of a duplex nucleic acid complex may comprise a center region comprising nucleotides and/or modified nucleotides and a wing region (or wing regions) comprising one or a plurality of nucleotide analogs and/or modified nucleotides provided on the 5' terminal side and/or 3' terminal side thereof.

In the present invention, examples of a nucleic acid complex that regulates expression or editing of a target gene or a transcription or translation product thereof include a nucleic acid molecule selected from the group consisting of a nucleic acid molecule comprising an antisense strand consisting of an oligonucleotide having a nucleotide sequence complementary to a target gene or a transcription product thereof, an aptamer comprising a nucleotide sequence binding specifically to a target protein, and a decoy consisting of an oligonucleotide having a nucleic acid sequence complementary to a target transcription factor.

In the present invention, other examples of a nucleic acid molecule that regulates expression or editing of a target gene or a transcription or translation product thereof is a nucleic acid molecule selected from the group consisting of ADO, ASO, HDO, and RNAi.

### 2. Ligand

A "ligand" is a substance that forms a complex with a biomolecule to serve biological purpose. In an embodiment, a ligand has functions of delivery to a target. An example of a preferable ligand is a lipid since it can efficiently deliver a given nucleic acid complex to the liver or other organ with high specificity. Examples of lipids include lipids, such as cholesterol and fatty acid (e.g., vitamin E (tocopherols and tocotrienols), vitamin A, and vitamin D), fat-soluble vitamin such as vitamin K (e.g., acylcarnitine), an intermediate metabolite such as acyl-CoA, a glycolipid, a glyceride, and a derivative of any thereof. In an embodiment, use of cholesterol and vitamin E (tocopherols and tocotrienols) is particularly preferable because of higher safety. Another example of a preferable ligand is a sugar (e.g., glucose or sucrose) since it can efficiently deliver nucleic acid molecules to the brain with high specificity. A ligand binds to various proteins on the cell surfaces of organs to efficiently deliver a nucleic acid complex to the organ with high specificity. In this respect, an example of a preferable ligand is a peptide or protein of a receptor ligand, an antibody, and/or a fragment thereof.

An "PTH1 ligand" is a ligand that can bind to a PTH1 receptor. Representative examples of a ligand that can bind to a PTH1 receptor include human parathyroid hormone (hPTH), human parathyroid hormone-related protein (hPTHrP), and human tuberoinfundibular peptide (hTIP).

In an embodiment, the PTH1 ligand is a peptide consisting of the amino acid sequence represented by the formula below.

[Chemical Formula 2] A₁-A₂-A₃-A₄-A₅-A₆-A₇-A₈-A₉-A₁₀-A₁₁-A₁₂-A₁₃-A₁₄-A₁₅-A₁₆-A₁₇-A₁₈-A₁₉-A₂₀-A₂₁-A₂₂-A₂₃-A₂₄-A₂₅-A₂₆-A₂₇-A₂₈-A₂₉-A₃₀-A₃₁-A₃₂-A₃₃-A₃₄
wherein the amino acid sequence is arranged in order from the N terminus toward the C terminus;
A1 represents Ala, Ser, or Dap or is deleted;
A2 represents Val or is deleted;
A3 represents Ser, Thr, or Aib or is deleted;
A4 represents Glu or is deleted;
A5 represents Leu, His, Nle, Ile, Cha, β-Nal, Trp, Pal, Acc, or Phep-X-Phe, wherein X represents OH, halogen, or CH₃, or is deleted;
A6 represents Gin;
A7 represents Leu, Nle, Ile, Cha, β-Nal, Trp, Pal, Acc, or Phep-X-Phe, wherein X represents OH, halogen, or CH₃, or is deleted;
A8 represents Met, Nva, Leu, Val, Ile, Cha, Acc, or Nle or is deleted;
A9 represents His or is deleted;
A10 represents Asp or Asn;
A11 represents Leu, Lys, Nle, Ile, Cha, β-Nal, Trp, Pal, Acc, Phe, or p-X-Phe, wherein X represents OH, halogen, or CH₃;
A12 represents Gly, Acc, or Aib;
A13 represents Lys;
A14 represents Ser or His;
A15 represents Leu, Nle, Ile, Cha, β-Nal, Trp, Pal, Acc, Phe, or P-X-Phe, wherein X represents OH, halogen, or CH₃;
A16 represents Ser, Gin, Asn, Ala, or Aib;
A17 represents Ser, Asp, Thr, or Aib;
A18 represents Met, Nva, Leu, Val, Ile, Nle, Acc, Cha, or Aib;
A19 represents Arg, Glu, or Aib;
A20 represents Arg;
A21 represents Arg, Val, Acc, Cha, or Met;
A22 represents Phe, Glu, Aib, Acc, or Cha;
A23 represents Phe, Trp, Leu, Lys, Acc, or Cha;
A24 represents Leu, Lys, Acc, or Cha;
A25 represents His, Arg, Lys, Aib, Acc, or Glu;
A26 represents His, Aib, Acc, or Lys;
A27 represents Lys, Aib, Leu, hArg, Gin, Acc, or Cha;
A28 represents Ile, Leu, Lys, Acc, or Cha;
A29 represents Ala, Glu, Acc, or Aib;
A30 represents Glu, Asp, Leu, Nle, Cha, Aib, Acc, or Lys;
A31 represents Ile, Val, Leu, Nle, Cha, Lys, or Acc;
A32 represents His;
A33 represents Thr, Asn, Lys, or Cys; and
A34 represents Phe, Ala, Tyr, Amp, or Aib.

When amino acids and the like are abbreviated herein, such abbreviations are based on the abbreviations provided by the IUPAC-IUB Commission on Biochemical Nomenclature or common abbreviations in the art. Examples thereof are provided blow. When the enantiomers of amino acids can be present, such amino acids are in the L-form, unless otherwise specified.
Gly or G: glycine
Ala or A: alanine
Val or V: valine
Leu or L: leucine
Ile or I: isoleucine
Ser or S: serine
Thr or T: threonine
Cys or C: cysteine
Met or M: methionine
Glu or E: glutamic acid
Asp or D: aspartic acid
Lys or K: lysine
Arg or R: arginine
His or H: histidine
Phe or F: phenylalanine
Tyr or Y: tyrosine
Trp or W: tryptophan
Pro or P: proline
Asn or N: asparagine
Gln or Q: glutamine
Aib: aminoisobutyric acid
Nle: norleucine
β-Ala:β-alanine
hPTH: human PTH
Boc: t-butoxycarbonyl
Fmoc: 9-fluorenylmethyoxycarbonyl
Nva: norvaline
Abu: α-aminobutyric acid
Ahc: 1-aminocyclohexylcarboxilic acid
hArg: homoarginine
Cha: 2-amino-3-cyclohexylpropionic acid
Npa: 3-(2-naphthyl)-alanine
Dap: 2,3-aminopropionic acid
D-Ser: (D)-Ser
D-Leu: (D)-Leu
D-Trp: (D)-Trp

The peptides represented by the formulae above include hPTH (1-34), a hPTH (1-34) derivative derived from hPTH (1-34) by substitution of an amino acid in a particular site with another amino acid, hPTHrP (1-34), a hPTHrP(1-34) derivative, and a peptide in which the "n" number (n = an integer of 1 to 9) of amino acids are deleted from the N-terminus of the aforementioned peptides.

In some embodiments, a PTH1 ligand may be hTIP(1-39), hTIP(1-39) derivative, or a peptide in which the "n" number (n = an integer of 1 to 9) of amino acids are deleted from the N-terminus of the aforementioned peptides.

In some embodiments, a PTH1 ligand may be a hPTH (1-34) peptide, a hPTHrP (1-34) peptide, or a hTIP (1-39) peptide selected from the peptides represented by SEQ ID NOs: 1 to 168 included in Table 2 below, a derivative of any thereof, a partial peptide of any thereof, or a peptide in which the "n" number (n = an integer of 1 to 9) of amino acids are deleted from the N-terminus of the aforementioned peptides.

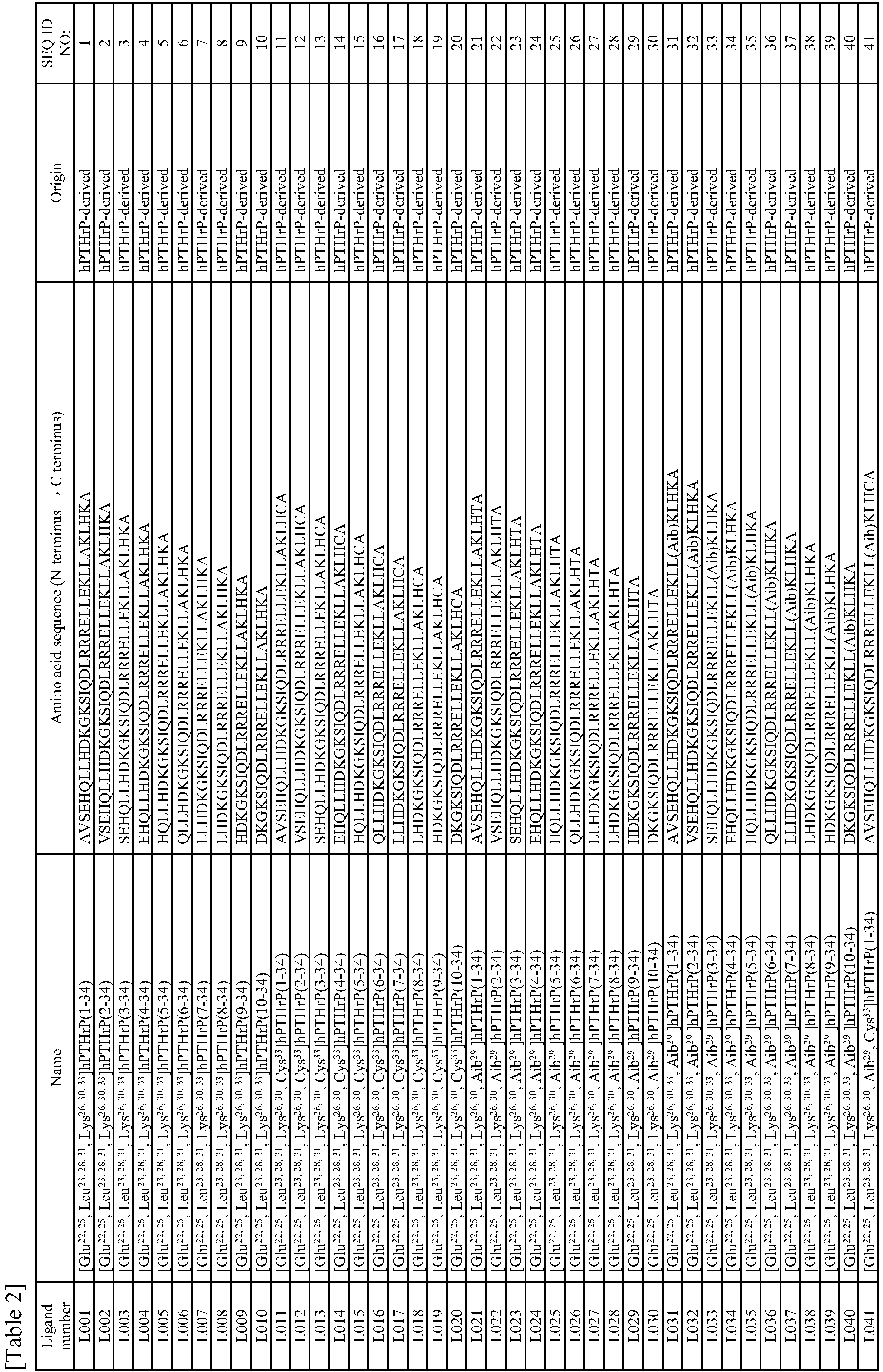

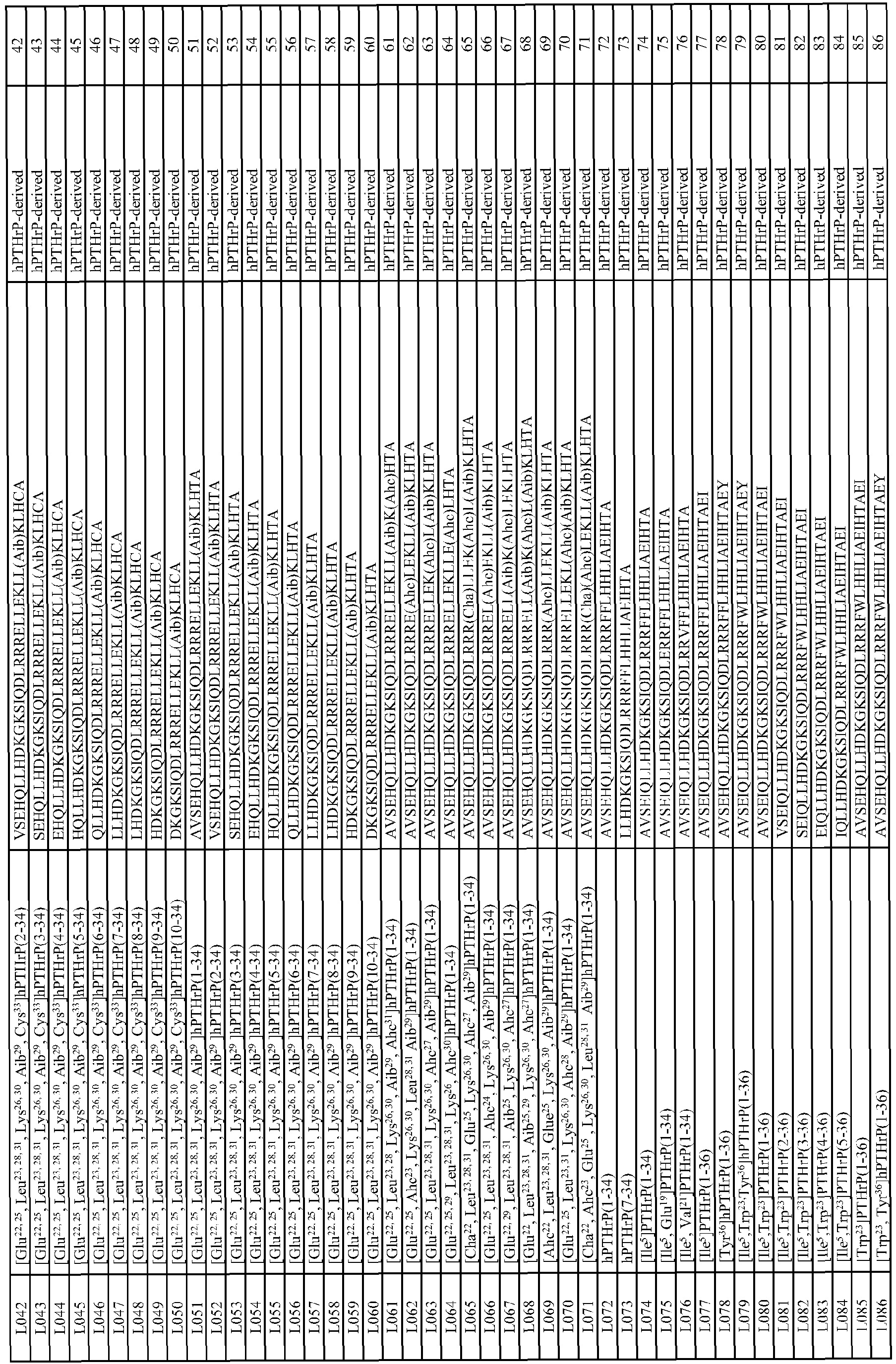

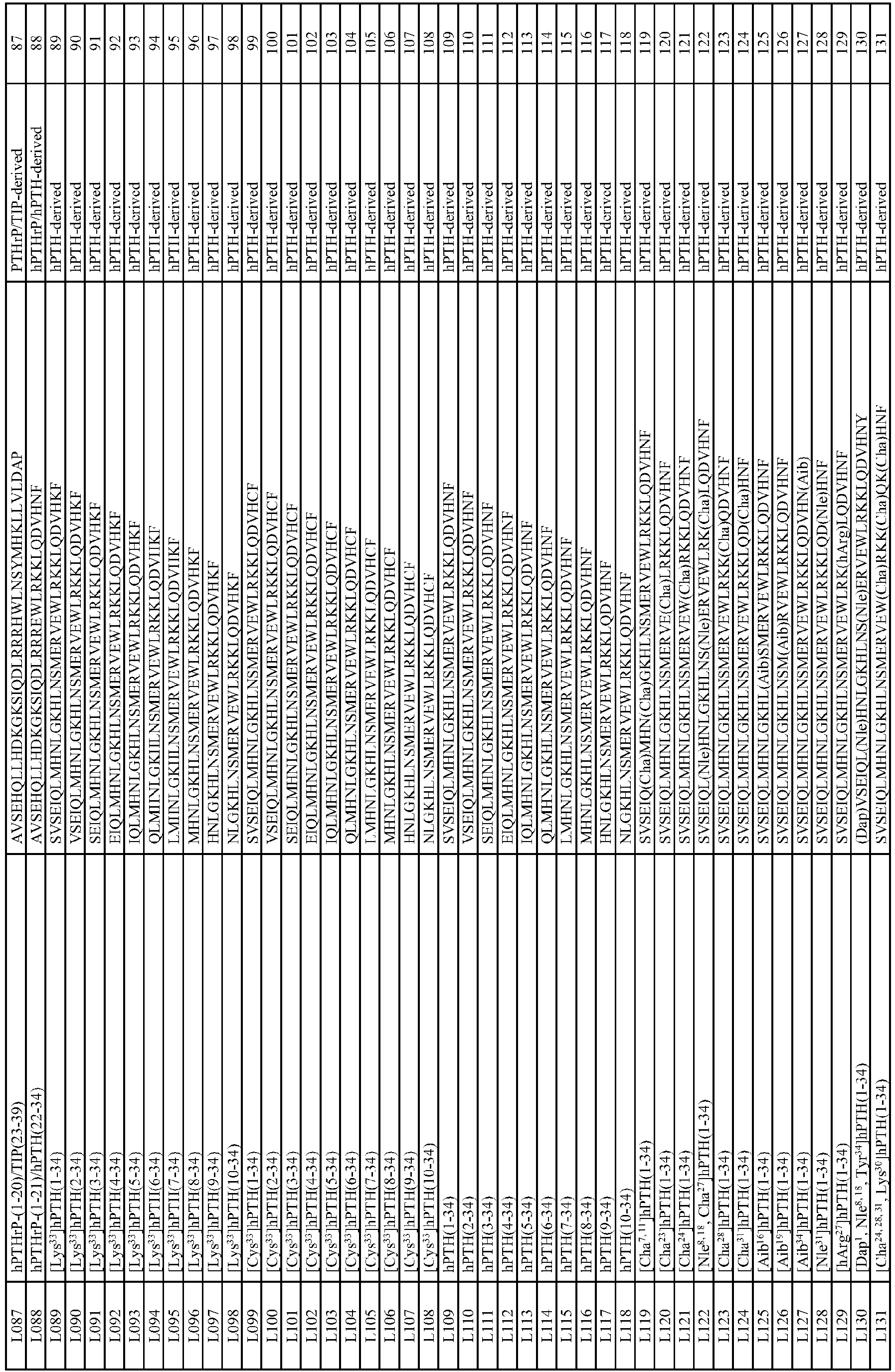

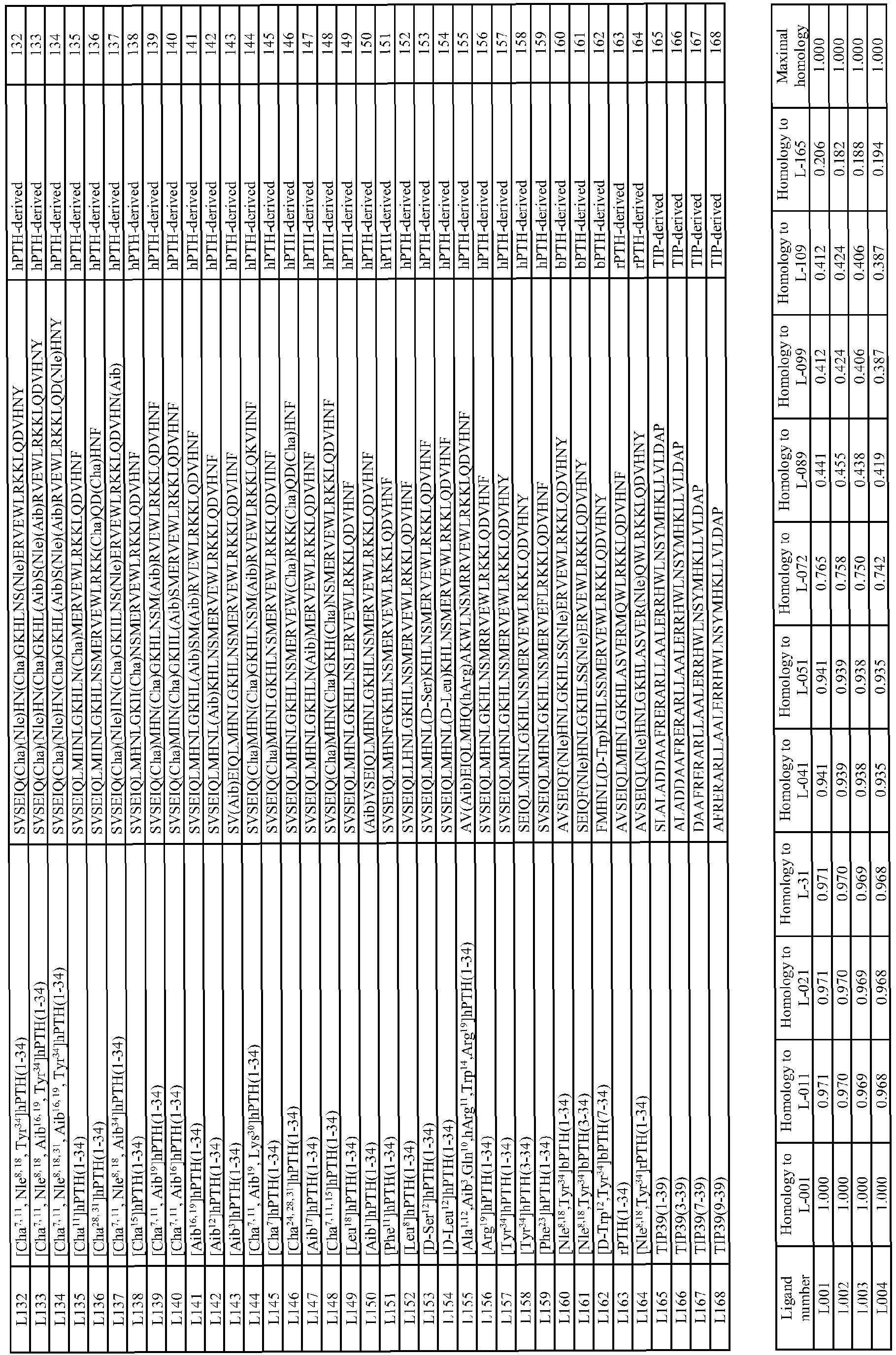

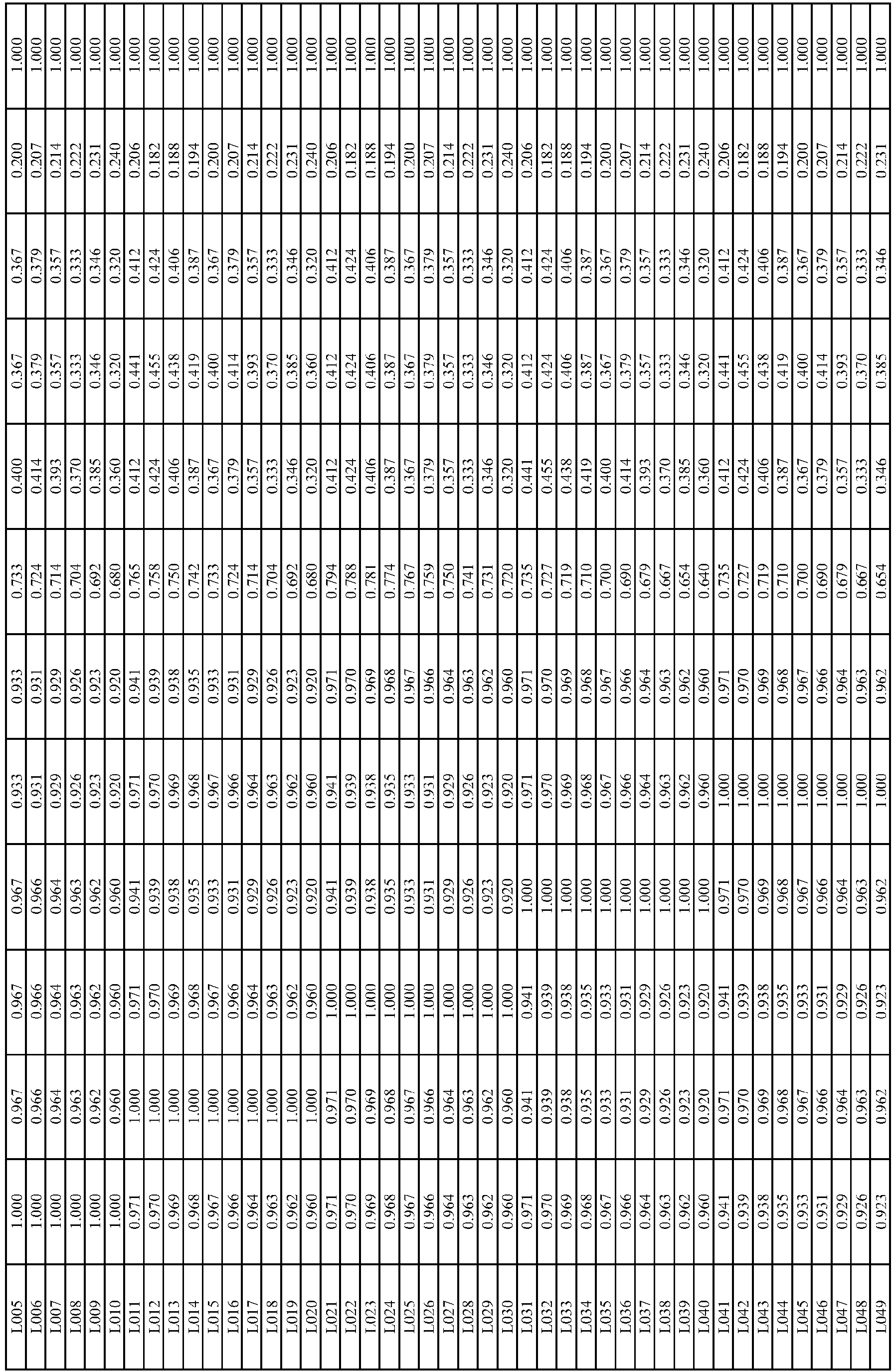

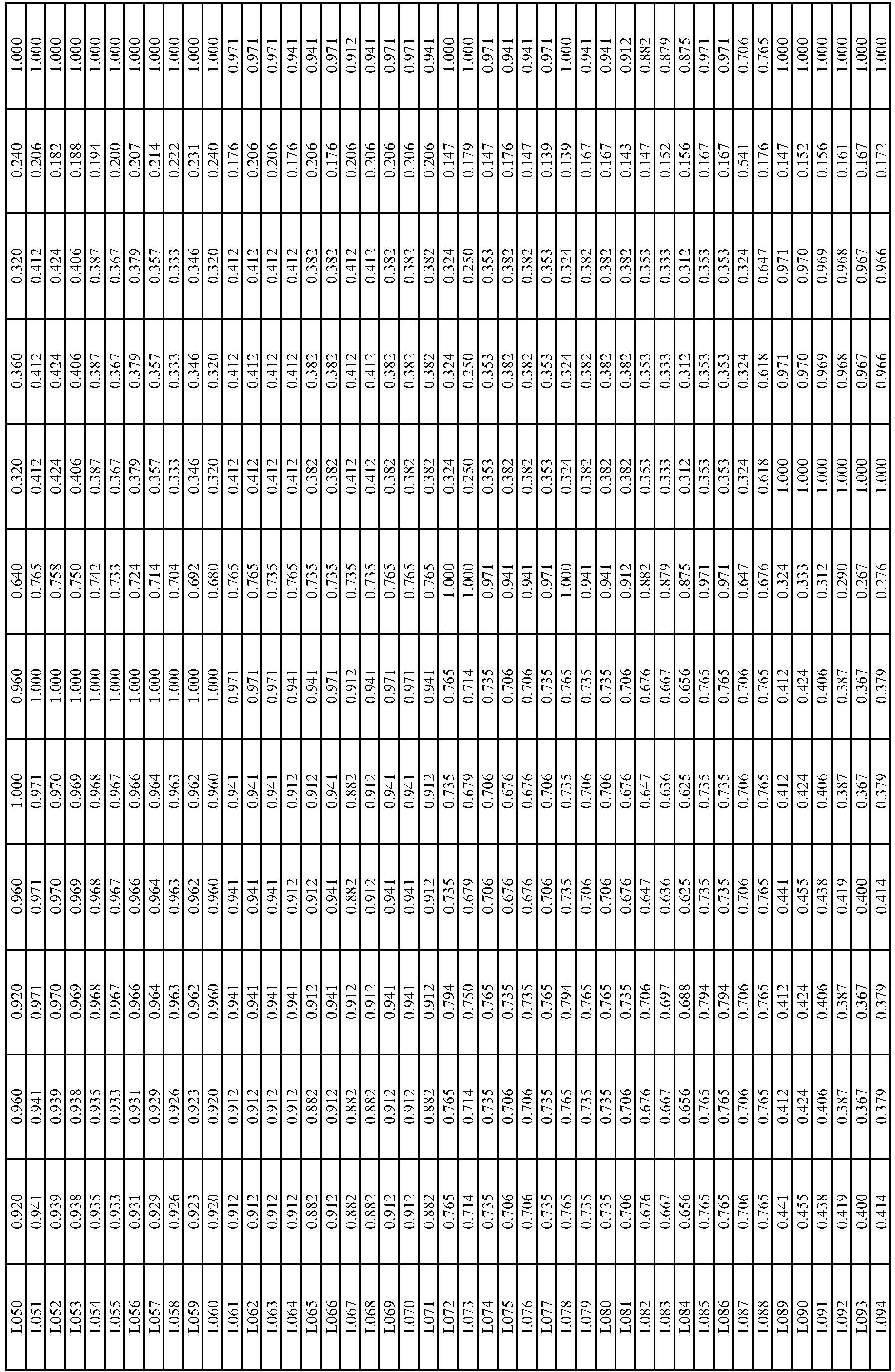

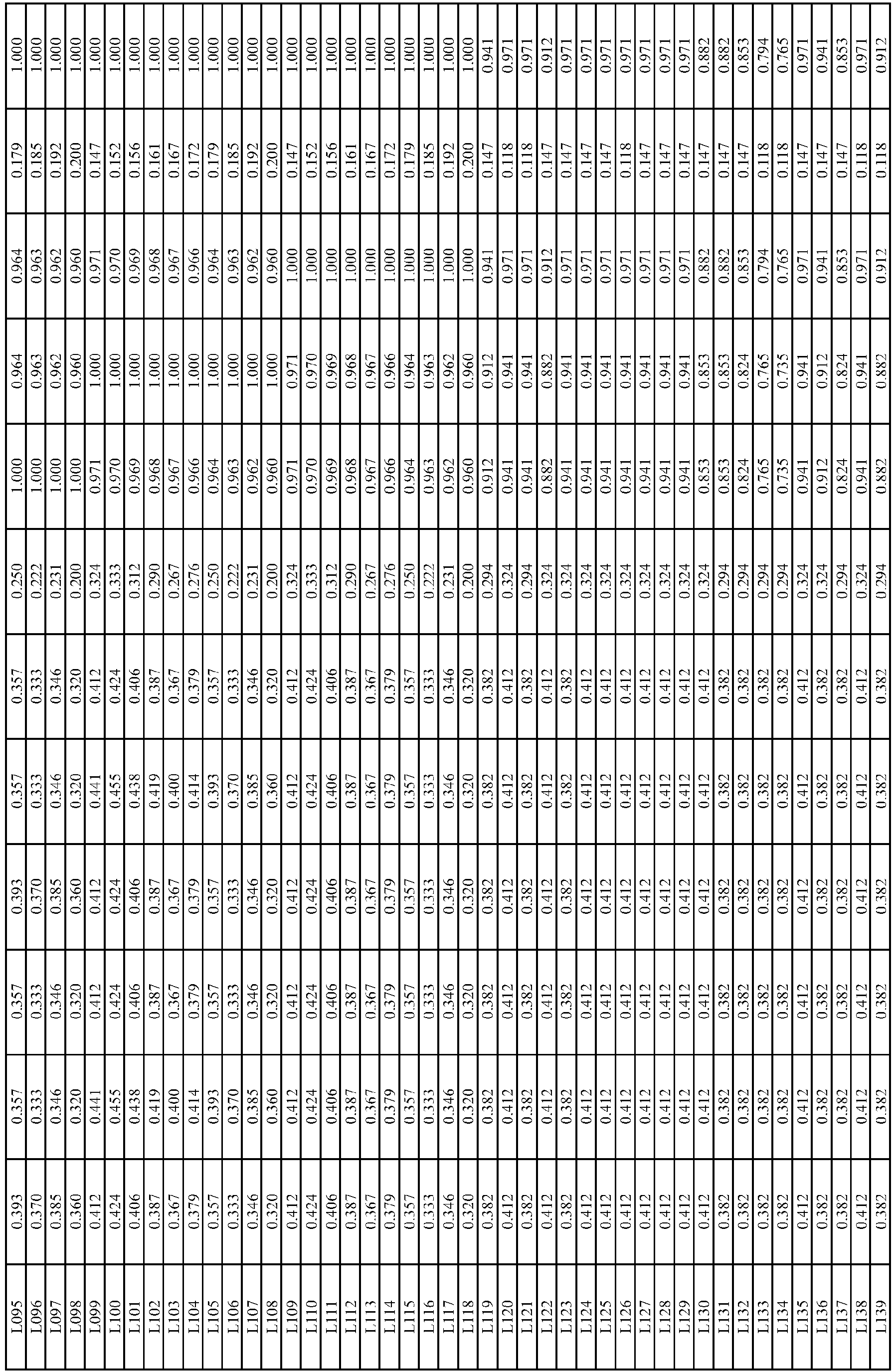

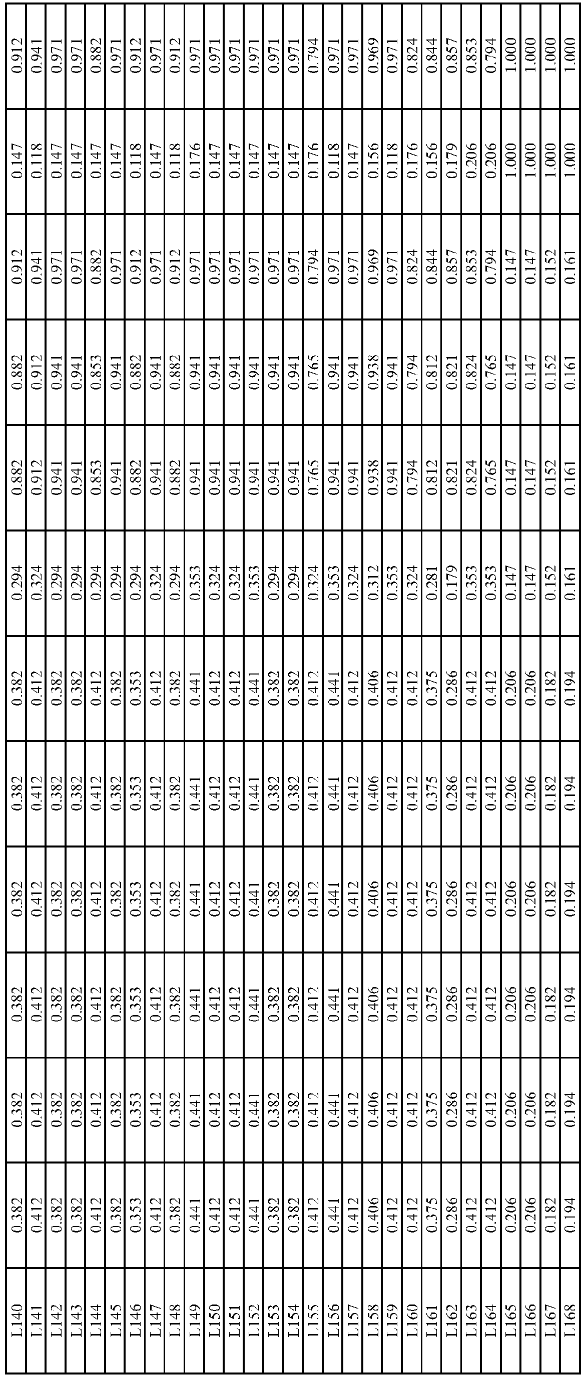

In Table 2, a homology score indicates homology between the amino acid sequence of the peptide with the ligand number indicated in the first column and the amino acid sequence of a comparable peptide. When the peptide in the first column is L011 and the comparable peptide is L001, specifically, the second amino acid K from the C terminus in the former is inconsistent with the second amino acid C from the C terminus in the latter among the 34 amino acids, and the other 33 amino acids are identical between these sequences. Thus, the homology score is calculated as 33/34 = 0.971 (97.1%). When a peptide comprises an amino acid sequence in which the "n" number (n = an integer of 1 to 9) of amino acids are deleted from the N-terminus of the full-length peptide derived from hPTHrP (1-34), hPTH (1-34), or hTIP (1-39) and the peptide in the first column is L012 (the number of deletion: n = 1) and the comparable peptide is L001, the second amino acid K from the C terminus in L012 is inconsistent with the second amino acid C from the C terminus in a region consisting of 33 amino acids of L001, which is equivalent to L012, and the other 32 amino acids are identical between these regions. Thus, the homology score is calculated to be 32/33 = 0.970 (97.0%).

All the PTH1 ligands shown in Table 2 are human PTH1 ligands.

In some embodiments, a PTH1 ligand is a peptide selected from the group of hPTHrP (1-34) peptides represented by SEQ ID NOs: 1 to 88 shown in Table 1 and partial peptides thereof.

In some embodiments, a PTH1 ligand is a peptide selected from the group of hPTH (1-34) peptides represented by SEQ ID NOs: 89 to 164 shown in Table 1 and partial peptides thereof.

In some embodiments, a PTH1 ligand is a peptide selected from the group of hTIP (1-39) peptides represented by SEQ ID NOs: 165 to 168 shown in Table 1 and partial peptides thereof.

In some embodiments, a PTH1 ligand may be a peptide comprising an amino acid sequence exhibiting at least 75%, 80%, 85%, 90%, 95%, or higher identity or homology to L001, L011, L021, L031, L041, L051, and L072 derived from hPTHrP (1-34).

In some embodiments, a PTH1 ligand may be a peptide comprising an amino acid sequence exhibiting at least 75%, 80%, 85%, 90%, 95%, or higher identity or homology to L089, L099, and L109 derived from hPTH (1-34).

In some embodiments, a PTH1 ligand may be a peptide comprising an amino acid sequence exhibiting at least 75%, 80%, 85%, 90%, 95%, or higher identity or homology to L165 derived from hTIP (1-39).

In the nucleic acid complex of the present invention, a ligand binds to an antisense strand and/or a complementary strand of a nucleic acid molecule. In some embodiments, a ligand binds to the 3' or 5' terminus of the nucleic acid strand. In some embodiments, a ligand binds to a site other than the terminus of the nucleic acid strand. A method of binding a ligand to position 2 of a pentose of a nucleotide is known. For example, such method can be performed in accordance with the method disclosed in WO 2018/003739.

A "PTH1 ligand" may be a salt of the peptide. Examples of salts of the peptide include sodium salt, potassium salt, calcium salt, hydrochloric acid salt, sulfuric acid salt, nitric acid salt, acetic acid salt, methanesulfonic acid salt, toluenesulfonic acid salt, citric acid salt, fumaric acid salt, maleic acid salt, and hydrobromic acid salt.

The peptide can be prepared in accordance with a conventional technique, such as solid-phase synthesis.

### 3. Binding of ligand to nucleic acid molecule

A ligand can bind to a nucleic acid by, for example, binding a bindable group of a ligand to a bindable group of a nucleic acid molecule covalently or non-covalently by means of hydrogen binding, electrostatic interaction, or hydrophobic interaction. Examples of bindable groups of a ligand include, but are not limited to, amino, hydroxy, carboxylic acid, thiol, disulfide, and azide groups of a peptide as a PTH1 ligand. Examples of bindable groups of a nucleic acid molecule include carbon at position 2 and a hydroxy group at position 3 or 5 of a nucleoside sugar, a phosphoric acid group at position 5 of a nucleotide, and a base portion of a nucleoside. Another example of a bindable group of a nucleic acid molecule is a phosphoric acid group in an oligonucleotide.

A nucleic acid molecule binds to a ligand with or without a linker.

### 4. Linker

In some embodiments, a ligand binds to a nucleic acid molecule with a linker. When a nucleic acid molecule is HDO, a ligand may bind to an antisense strand and/or a complementary strand of HDO with a linker. A cleavable or uncleavable linker can be used.

A "cleavable linker" is a linking group that is cleaved in, for example, a cell or animal body (e.g., human body) under physiological conditions. In a particular embodiment, a cleavable linker is selectively cleaved by an endogenous enzyme, such as a nuclease. Examples of cleavable linkers include an amide bond, an ester bond, one or both ester bonds of phosphodiester bonds, an ester phosphate bond, a carbamate bond, a disulfide bond, and a natural DNA linker. An "uncleavable linker" is a linker that is not cleaved in, for example, a cell or animal body (e.g., human body) under physiological conditions. Examples of uncleavable linkers include, but are not limited to, a phosphorothioate bond, a modified or unmodified deoxyribonucleoside linked via a phosphorothioate bond, and a linker consisting of a modified or unmodified ribonucleoside. When a linker is a nucleic acid or an oligonucleotide, such as DNA, a chain length is not particularly limited, and, in general, a linker may comprise 2 to 20, and preferably 3 to 10 bases.

In some embodiments, a linker used in the present invention may be a chain structure, such as a hydrocarbyl chain, or an oligomer, which is a repeating unit such as an ethylene glycol, nucleoside, or amino acid unit.

In some embodiments, a linker comprises at least one group selected from among alkyl, amino, oxo, amide, disulfide, polyethylene glycol, ether, thioether, and hydroxylamino groups.

In some embodiments, a linker comprises a group selected from among alkyl, amino, oxo, amide, and ether groups.

In some embodiments, a linker comprises a group selected from among alkyl and amide groups.

In some embodiments, a linker comprises a group selected from among alkyl and ether groups.

In some embodiments, a linker comprises at least one phosphorus portion.

In some embodiments, a linker comprises at least one phosphate group.

In some embodiments, a linker comprises at least one neutral linking group.

In some embodiments, a length of a linker is 1 to 1000 Å. A length of a particular linker is 3 to 500 Å. A length of a linker is preferably 3 to 200 Å. (The length was estimated based on the crystalline structure comprising the PTH11 receptor bound to a PTH analog (PDB 6F3J).)

In some embodiments, a bond between a linker and an oligonucleotide can be a bifunctional bond. In general, a bifunctional bond is formed of at least 2 functional groups. A functional group of a linker binding to a particular site of an oligonucleotide is selected, and another functional group of a linker binding to a ligand portion is selected. Examples of functional groups used for a bifunctional bond of a linker include, but are not limited to, an electrophile to react with a nucleophilic group and a nucleophilic agent to react with an electrophilic group. In a particular embodiment, a bifunctional bond can be formed with the use of one or more groups selected from among amino, hydroxyl, carboxylic acid, thiol, alkyl, alkenyl, and alkynyl groups.

Examples of linkers include, but are not limited to, 6-aminohexanoic acid (AHA or AHEM), (2,5-dioxypyrrolidin-1-yl)4-(2-azatricyclo[10.0.04,9]hexadeca-1(16),4,6,8,12,14-hexe-10-n-2-yl-4-oxobutanoate (DBCO-NHS), 3-mercaptopropionic acid, and succinimidyl 4-(N-meleimidemethyl)cyclohexane-1-carboxylate. Examples of other linkers include, but are not limited to, substituted or unsubstituted C¹ to C¹⁰ alkyl, substituted or unsubstituted C² to C¹⁰ alkenyl, and substituted or unsubstituted C² to C¹⁰ alkynyl. Examples of preferable substituents include, but are not limited to, hydroxyl, amino, alkoxy, carboxy, benzyl, phenyl, nitro, thiol, thioalkoxy, halogen, alkyl, aryl, alkenyl, and alkynyl groups.

In a particular embodiment, a linker can be selected from among compounds having the structures shown in Table 3 and derivatives thereof.

**[Table 3]**

| Linker type | Structural formula |
|---|---|
| Having a disulfide bond | 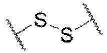 |
| Having an amide bond (n=0-9) | 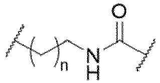 |
| Having a polyethylene glycol group (n=1-200) | 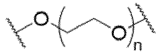 |
| Having a phosphate group | 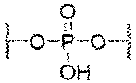 |
| Having a pyrrolidinyl group | 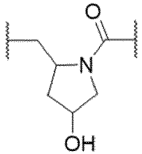 |
| Having a BCN group | 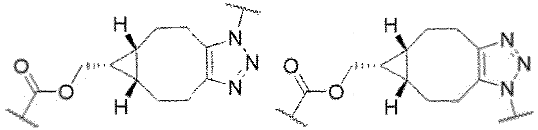 |
| Having a DBCO group | 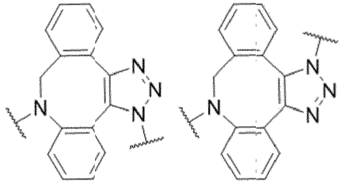 |
| Having a maleimide thioether group | 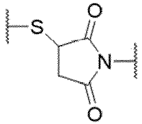 |

In Table 3, the number "n" of the polyethylene glycol groups is 1 to 200, preferably 1 to 100, and more preferably 1 to 50.

In a particular embodiment, a linker may be composed of 2 to 20 linker-nucleoside bonds. In a particular embodiment, a linker-nucleoside bond is composed of continuous modified nucleosides. In a particular embodiment, such linker-nucleoside bond may comprise a modified sugar portion. In a particular embodiment, a linker-nucleoside bond is not modified. In a particular embodiment, a linker-nucleoside bond comprises a protected heterocyclic base selected from among purine, substituted purine, pyrimidine, and substituted pyrimidine, according to need.

In a particular embodiment, a linker can be selected from among compounds having the structures shown below and derivatives thereof. 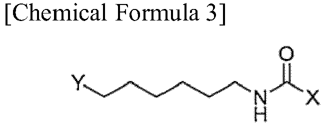 In the formula, X represents a ligand-binding site, and Y represents a nucleic acid molecule-binding site.

In a particular embodiment, a compound having active ester represented by Chemical Formula 4: 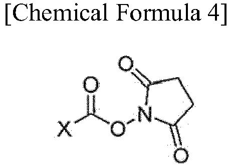
(wherein X represents a ligand-binding site) may be allowed to react with an oligonucleotide having terminal amine comprising a compound represented by Chemical Formula 5: 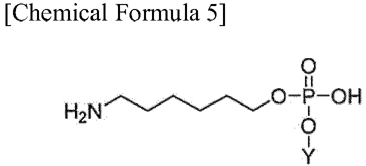
(wherein Y represents a nucleic acid molecule-binding site) to obtain a linker represented by Chemical Formula 6 below: 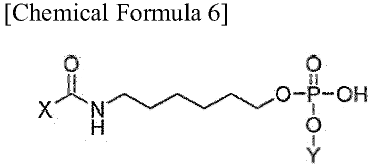
(wherein X represents a ligand-binding site, Y represents a nucleic acid molecule-binding site, and a nucleic acid molecule is selected from among a natural nucleotide, a modified nucleotide, and a nucleotide analogs or an oligonucleotide comprising the same).

In a particular embodiment, a linker comprising phosphate/triethylene glycol can be used.

In a particular embodiment, a linker represented by Chemical Formula 7 can be used.

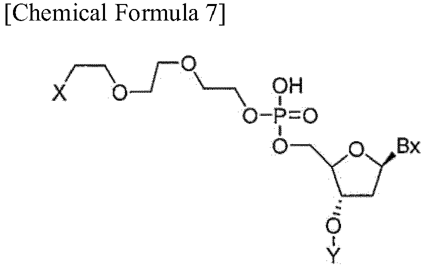

In the formula, X represents a ligand-binding site, Y represents a nucleic acid molecule-binding site, and Bx represents a modified or unmodified nucleic acid base.
In a particular embodiment, a compound having amidite represented by Chemical Formula 7 above and a compound represented by Chemical Formula 8: 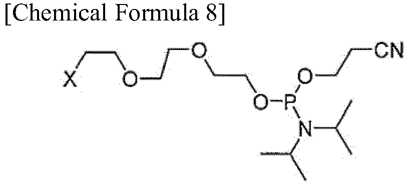
(wherein X represents a ligand) are allowed to react with an oligonucleotide site Y on a solid-phase support and cleaved from the solid-phase support to obtain a linker represented by Chemical Formula 9: 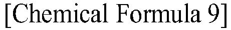 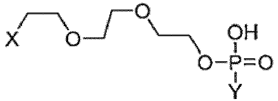
(wherein X represents a ligand, and Y represents a nucleic acid molecule).

In a particular embodiment, a linker has a structure represented by Chemical Formula 10 below: 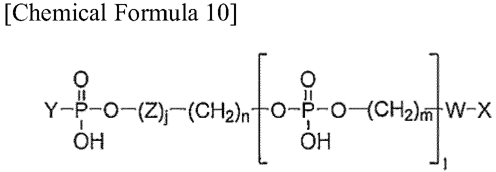 (wherein X represents a ligand-binding site, Y represents a nucleic acid molecule-binding site, W represents a phosphodiester or amino group, Z represents a pyrrolidinyl group represented by Chemical Formula 11 below, j is 0 or 1; n is about 1 to about 10, m is about 1 to about 10; 1 is 0 or 1 to 4, and, when X represents an amino group, 1 is 1).

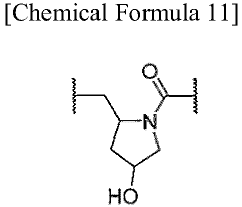

In a particular embodiment, a linker is prepared by click chemistry. Linkers that are preferably used in several embodiments can be prepared by click chemistry described in "Click Chemistry for Biotechnology and Materials Science," Ed. Joerg Laham, Wiley, 2009 (which is incorporated herein by reference in their entirety).

In a particular embodiment, a compound represented by Chemical Formula 12: 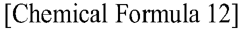 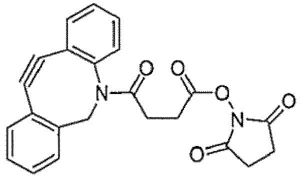
and a compound represented by Chemical Formula 13: 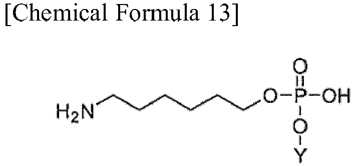
(wherein Y represents a nucleic acid molecule-binding site) were allowed to react with an oligonucleotide having terminal amine to obtain a compound represented by Chemical Formula 14.

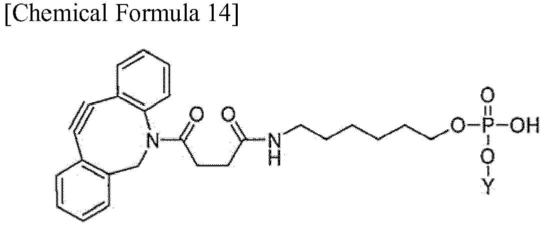

The resulting compound represented by Chemical Formula 14 was allowed to react with a ligand having azide to obtain a linker represented by Chemical Formula 15: 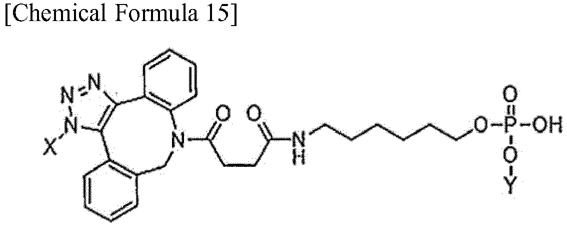 (wherein X represents a ligand-binding site, and Y represents a nucleic acid molecule-binding site).

In a particular embodiment, a maleimide linker can be used. The maleimide linker has a structure represented by Chemical Formula 16.

The linker comprises: 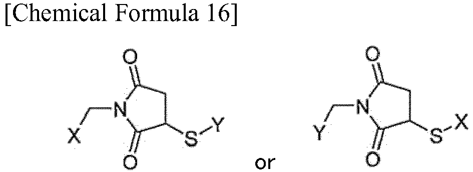 wherein X represents a ligand-binding site, and Y represents a nucleic acid molecule-binding site.

In a particular embodiment, a compound represented by Chemical Formula 17: 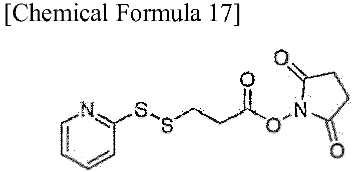
and a compound represented by Chemical Formula 18: 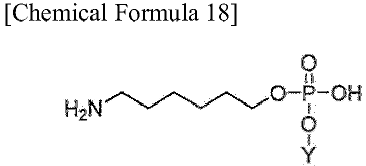
(wherein Y represents a nucleic acid molecule-binding site) were allowed to react with an oligonucleotide having terminal amine to obtain a compound represented by Chemical Formula 19.

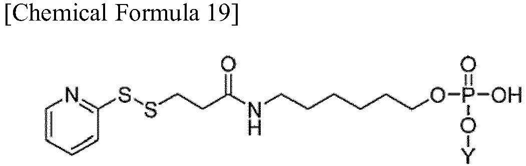

The compound represented by Chemical Formula 19 was allowed to react with a ligand conjugate portion having maleimide to obtain a linker represented by Chemical Formula 20: 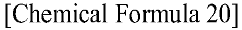 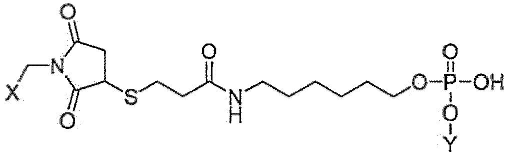 (wherein X represents a ligand-binding site, and Y represents a nucleic acid molecule-binding site).

In a particular embodiment, a compound represented by Chemical Formula 21: 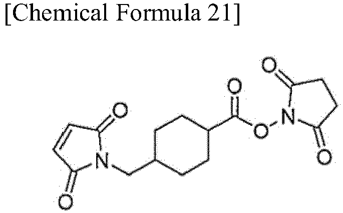
and a compound represented by Chemical Formula 22: 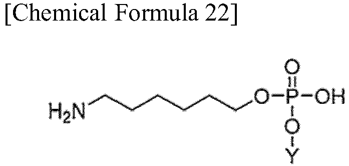
(wherein Y represents a nucleic acid molecule-binding site) were allowed to react with an oligonucleotide having terminal amine to obtain a compound represented by Chemical Formula 23.

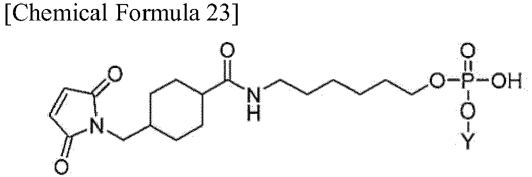

The compound represented by Chemical Formula 23 was allowed to react with a ligand conjugate portion having thiol to obtain a linker represented by Chemical Formula 24: 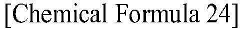 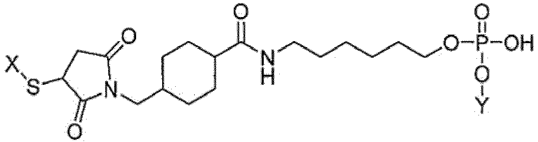 (wherein X represents a ligand-binding site, and Y represents a nucleic acid molecule-binding site).

### Specific examples and method of attaching disulfide linker

In a particular embodiment, a linker comprising a disulfide bond can be used. In a particular embodiment, a linker comprises activated disulfide that forms a disulfide bond with a ligand-binding portion.

In a particular embodiment, a compound represented by Chemical Formula 25: 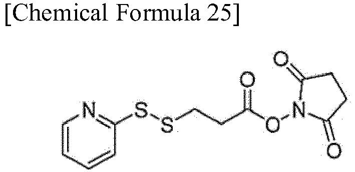
and a compound represented by Chemical Formula 26: 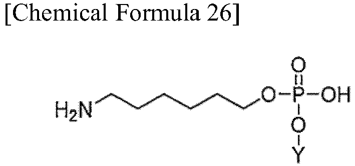
(wherein Y represents a nucleic acid molecule binding site) were allowed to react with an oligonucleotide having terminal amine to obtain a compound represented by Chemical Formula 27.

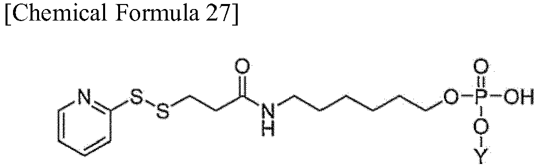

The disulfide bond thereof was cleaved to obtain a compound represented by Chemical Formula 28.

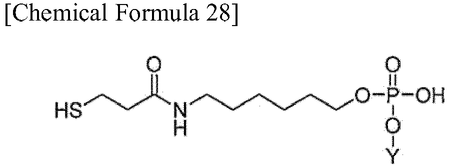

The compound represented by Chemical Formula 28 was allowed to react with a ligand conjugate portion having thiol to obtain a linker represented by Chemical Formula 29: 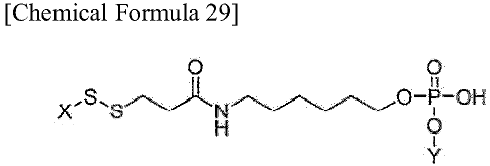 (wherein X represents a ligand-binding site, and Y represents a nucleic acid molecule-binding site).

In a particular embodiment, a linker chemically binds to the PTH1 ligand. The PTH1 ligand binds to a functional group with a linker so as to be attached to an oligonucleotide.

In a particular embodiment, an amino group (but is not limited thereto) in the ligand-binding portion (X-NH₂; in which X represents a ligand conjugate portion excluding the amino group) was allowed to react with a compound represented by Chemical Formula 30: 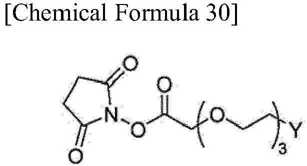
(wherein Y represents a functional group to be directly or indirectly attached to an oligo, such as azide or maleimide) to obtain a linker comprising a linker introduced into the ligand-binding portion represented by Chemical Formula 31: 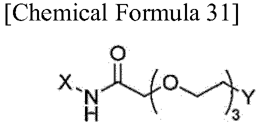
(wherein X represents a ligand-binding site, and Y represents a nucleic acid molecule-binding site).

### 5. Pharmaceutical composition

Diseases targeted by the ligand-binding nucleic acid complex of the present invention may be any diseases, provided that such diseases are associated with the target organs and tissue and cells in the target organs. Examples of such diseases include, but are not limited to, diabetes, metabolic syndrome, cardiac disease, muscular dystrophy, myotonic dystrophy, Becker muscular dystrophy, congenital muscular dystrophy, Duchenne muscular dystrophy, distal muscular dystrophy, Emery-Dreifuss muscular dystrophy, facioscapulohumeral muscular dystrophy, limb girdle muscular dystrophy, oculopharyngeal muscular dystrophy, chronic kidney disease (CKD), renal fibrosis, diabetic nephropathy, chronic glomerulonephritis, IgA nephropathy, lupus nephritis, primary glomerular disease, chronic obstructive pulmonary disease (COPD), lung emphysema, interstitial pneumonia, lung fibrosis, heart disease, and muscular disease.

A composition comprising the ligand-binding nucleic acid complex according to any of some embodiments can be prepared in the form of a pharmaceutical product in accordance with a conventional pharmaceutical method. Such composition can be used in the form of, for example, a capsule, a tablet, a pill, a liquid, a powder, granules, fine grains, a film-coated agent, a pellette, a troche, a sublingual formulation, a masticatory formulation, a buccal tablet, a paste, a syrup, a suspension, an elixir, an emulsion, an endermic liniment, an ointment, a plaster, a poultices, a transdermal formulation, a lotion, an inhalant, an aerosol, an injection preparation, or a suppository for enteral (oral) or nonenteral administration.

When manufacturing a pharmaceutical preparation, such composition can be used in adequate combination with a carrier that is pharmacologically acceptable or acceptable for a food or beverage product. Specific examples include sterilized water, physiological saline, vegetable oil, a solvent, a base, an emulsifier, a suspending agent, a surfactant, a pH modifier, a stabilizer, a flavoring agent, an aromatic agent, an excipient, a vehicle, a preservative, a binder, a diluent, an isotonizing agent, a soothing agent, a filler, a disintegrator, a buffer, a coating agent, a lubricant, a coloring agent, a sweetening agent, a thickener, a corrigent, a solubilizer, and other additives.

When manufacturing a pharmaceutical preparation, the ligand-binding nucleic acid complex according to any of the embodiments concerning enteral administration may form a composite with a substance having activity of increasing permeability of the large intestinal epithelium (e.g., medium chain fatty acid, long chain unsaturated fatty acid, or a derivative thereof (salt, ester, or ether) and a surfactant (a nonionic surfactant or anionic surfactant) (i.e., mixed micelle or emulsion)) in order to enhance efficiency for enteral administration.

Preferable administration routes of the composition comprising the ligand-binding nucleic acid complex according to some embodiments are not particularly limited, and enteral (oral) or nonenteral routes may be adopted. Specific examples include intravenous administration, intraarterial administration, intraperitoneal administration, subcutaneous administration, intracutaneous administration, intraspinal administration, tracheobronchial administration, rectal administration, intramuscular administration, and transfusion.

The composition comprising the ligand-binding nucleic acid complex according to some embodiments can be used for animal targets including humans. Animals other than humans are not particularly limited, and targets can be various livestock animals, fowls, pet animals, and experimental animals.

When the compositions comprising the ligand-binding nucleic acid complex according to some embodiments are administered or ingested, the amount of administration or ingestion may be adequately determined in accordance with, for example, age, body weight, symptoms, and health conditions of a target, and a composition type (e.g., a pharmaceutical product or food or beverage product). In an embodiment, an effective amount of the composition to be ingested is preferably 0.001 mg/kg/day to 50 mg/kg/day in terms of nucleotides.

### [Examples]

### [Example 1] Synthesis and purification of PTH1 ligand

Polypeptides shown in Table 1, which are the PTH1 ligands of the present invention, are introduced into oligonucleic acids in accordance with the patterns of synthesis shown in Table 4.

**[Table 4]**

| Ligand number | Modification pattern | Ligand linker number | Polypeptide sequence | Ligand linker number after oligo introduction | Polypeptide sequence upon introduction of oligonucleic acid | SEQ ID NO: |
|---|---|---|---|---|---|---|
| L001 | A | LG001 | AVSEHQLLHDKGKSIQDLRRRELLEKLLAKLH(K-PEG3-Azide)A | LGO001 | AVSEHQLLHDKGKSIQDLRRRELLEKLLAKLH(K-PEG3-Unit)A | 1 |
| L002 | A | LG002 | VSEHQLLHDKGKSIQDLRRRELLEKLLAKLH(K-PEG3-Azide)A | LGO002 | VSEHQLLHDKGKSIQDLRRRELLEKLLAKLH(K-PEG3-Unit)A | 2 |
| L003 | A | LG003 | SEHQLLHDKGKSIQDLRRRELLEKLLAKLH(K-PEG3-Azide)A | LGO003 | SEHQLLHDKGKSIQDLRRRELLEKLLAKLH(K-PEG3-Unit)A | 3 |
| L004 | A | LG004 | EHQLLHDKGKSIQDLRRRELLEKLLAKLH(K-PEG3-Azide)A | LGO004 | EHQLLHDKGKSIQDLRRRELLEKLLAKLH(K-PEG3-Unit)A | 4 |
| L005 | A | LG005 | HQLLHDKGKSIQDLRRRELLEKLLAKLH(K-PEG3-Azide)A | LGO005 | HQLLHDKGKSIQDLRRRELLEKLLAKLH(K-PFG3-Unit)A | 5 |
| L006 | A | LG006 | QLLHDKGKSIQDLRRRELLEKLLAKLH(K-PEG3-Azide)A | LGO006 | QLLHDKGKSIQDLRRRELLEKLLAKLH(K-PEG3-Unit)A | 6 |
| L007 | A | LG007 | LLHDKGKSIQDLRRRELLEKLLAKLH(K-PEG3-Azide)A | LGO007 | LLHDKGKSIQDLRRRELLEKLLAKLH(K-PEG3-Unit)A | 7 |
| L008 | A | LG008 | LHDKGKSIQDLRRRELLEKLLAKLH(K-PEG3-Azide)A | LGO008 | LHDKGKSIQDLRRPFITFRTTAKTH(K-PEG3-Unit)A | 8 |
| L009 | A | LG009 | HDKGKSIQDLRRRELLEKLLAKLH(K-PEG3-Azide)A | LGO009 | HDKGKSIQDLRRPFTLFRTTAKLH(K-PEG3-Unit)A | 9 |
| L010 | A | LG010 | DKGKSIQDLRRRELLEKLLAKLH(K-PEG3-Azide)A | LGO010 | DKGKSIQDLRRPFITFKTTAKLH(K-PEG3-Unit)A | 10 |
| L011 | B | LG011 | AVSEHQLLHDKGKSIQDLRRPFITFRTLAKTHCA | LGO011 | AVSEHQLLHDKGKSIQDLRRPFTTFRTTAKLH(C-S-Unit)A | 11 |
| L012 | B | LG012 | VSEHQLLHDKGKSIQDLRRPFIIFRTLAKTHCA | LGO012 | VSEHQLLHDKGKSIQDLRRPFTTFRTTAKLH(C-S-Unit)A | 12 |
| L013 | B | LG013 | SEHQLLHDKGKSIQDLRRPFITFKT LAKLHCA | LGO013 | SEHQLLHDKGKSIQDLRRPFITFRTTAKLH(C-S-Unit)A | 13 |
| L014 | B | LG014 | EHQLLHDKGKSIQDLRRPFTTFRTLAKT HCA | LGO014 | EHQLLHDKGKSIQDLRRRELIFKTLAKTH(C-S-Unit)A | 14 |
| L015 | B | LG015 | HQLLHDKGKSIQDLRRPFTTFKTLAKLHCA | LGO015 | HQLLHDKGKSIQDLRRPFTLFKTTAKLH(C-S-Unit)A | 15 |
| L016 | B | LG016 | QLLHDKGKSIQDLRRPFITFRTLAKLHCA | LGO016 | QLLHDKGKSIQDLRRPFITFRTTAKLH(C-S-Unit)A | 16 |
| L017 | B | LG017 | LLHDKGKSIQDLRRPFTTFRTLAKLHCA | LGO017 | LLHDKGKSIQDLRRPFTTFRTTAKTH(C-S-Unit)A | 17 |
| L018 | B | LG018 | LHDKGKSIQDLRRRELTFRTLAKTHCA | LGO018 | LHDKGKSIQDLRRPFITFRTTAKLH(C-S-Unit)A | 18 |
| L019 | B | LG019 | HDKGKSIQDLRRPFTIFKTLAKLHCA | LGO019 | HDKGKSIQDLRRPFTIFKTIAKLH(C-S-Unit)A | 19 |
| L020 | B | LG020 | DKGKSIQDLRRPFTTEKLAKLHCA | LGO020 | DKGKSIQDLRRPFTTFRTLAKLH(C-S-Unit)A | 20 |
| L021 | C | LG021 | AVSEHQLLHDKGKSIQDLRRRELLEKLLAKLHKA(K-PEG3-Azide) | LGO021 | AVSEHQLLHDKGKSIQDLRRRELLEKLLAKLHKA(K-PEG3-Unit) | 21 |
| L022 | C | LG022 | VSEHQLLHDKGKSIQDLRRRELLEKLLAKLHKA(K-PEG3-Azide) | LGO022 | VSEHQLLHDKGKSIQDLRRRELLEKLLAKLHKA (K-PEG3-Unit) | 22 |
| L023 | C | LG023 | SEHQLLHDKGKSIQDLRRRELLEKLLAKLHKA(K-PEG3-Azide) | LGO023 | SEHQLLHDKGKSIQDLRRRELLEKLLAKLHKA (K-PEG3-Unit) | 23 |
| L024 | C | LG024 | EHQLLHDKGKSIQDLRRRELLEKLLAKLHKA(K-PEG3-Azide) | LGO024 | EHQLLHDKGKSIQDLRRRELLEKLLAKLHKA (K-PEG3-Unit) | 24 |
| L025 | C | LG025 | HQLLHDKGKSIQDLRRRELLEKLLAKLHKA(K-PEG3-Azide) | LGO025 | HQLLHDKGKSIQDLRRRELLEKLLAKLHKA (K-PEG3-Unit) | 25 |
| L026 | C | LG026 | QLLHDKGKSIQDLRRRELLEKLLAKLHKA(K-PEG3-Azide) | LGO026 | QLLHDKGKSIQDLRRRELLEKLLAKLHKA (K-PEG3-Unit) | 26 |
| L027 | C | LG027 | LLHDKGKSIQDLRRRELLEKLLAKLHKA(K-PEG3-Azide) | LGO027 | LLHDKGKSIQDLRRRELLEKLLAKLHKA (K-PEG3-Unit) | 27 |
| L028 | C | LG028 | LHDKGKSIQDLRRRELLEKLLAKLHKA(K-PEG3-Azide) | LGO028 | LHDKGKSIQDLRRRELLEKLLAKLHKA(K-PEG3-Unit) | 28 |
| L029 | C | LG029 | HDKGKSIQDLRRPFTIFKTIAKLHKA(K-PFG3-Azide) | LGO029 | HDKGKSIQDLRRPETTFKTLAKLHKA(K-PEG3-Unit) | 29 |
| L030 | C | LG030 | DKGKSIQDLRRPETTFKTTAKLHKA(K-PEG3-Azide) | LGO030 | DKGKSIQDLRRPETIFKTIAKLHKA(K-PEG3-Unit) | 30 |
| L031 | A | LG031 | AVSEHQLLHDKGKSIQDLRRRELLEKLL(Aib)KLH (K-PEG3-Azide)A | LGO031 | AVSEHQLLHDKGKSIQDLRRRELLEKLL(Aib)KLH(K-PEG3-Unit)A | 31 |
| L032 | A | LG032 | VSEHQLLHDKGKSIQDLRRRELLEKLL(Aib)KLH(K-PEG3-Azide)A | LGO032 | VSEHQLLHDKGKSIQDLRRRELLEKLL(Aib)KLH(K-PEG3-Unit)A | 32 |
| L033 | A | LG033 | SEHQLLHDKGKSIQDLRRRELLEKLL(Aib)KLH (K-PEG3-Azide)A | LGO033 | SEHQLLHDKGKSIQDLRRRELLEKLL(Aib)KLH (K-PEG3-Unit)A | 33 |
| L034 | A | LG034 | EHQLLHDKGKSIQDLRRRELLEKLL(Aib)KLH (K-PEG3-Azide)A | LGO034 | EHQLLHDKGKSIQDLRRRELLEKLL(Aib)KLH (K-PEG3-Unit)A | 34 |
| L035 | A | LG035 | HQLLHDKGKSIQDLRRRELLEKLL(Aib)KLH (K-PEG3-Azide)A | LGO035 | HQLLHDKGKSIQDLRRRELLEKLL(Aib)KLH (K-PEG3-Unit)A | 35 |
| L036 | A | LG036 | QLLHDKGKSIQDLRRRELLEKLL(Aib)KLH (K-PEG3-Azide)A | LGO036 | QLLHDKGKSIQDLRRRELLEKLL(Aib)KLH (K-PEG3-Unit)A | 36 |
| L037 | A | LG037 | LLHDKGKSIQDLRRRELLEKLL(Aib)KLH (K-PEG3-Azide)A | LGO037 | LLHDKGKSIQDLRRRELLEKLL(Aib)KLH (K-PEG3-Unit)A | 37 |
| L038 | A | LG038 | LHDKGKSIQDLRRRELLEKLL(Aib)KLH (K-PEG3-Azide)A | LGO038 | LHDKGKSIQDLRRRELLEKLL(Aib)KLH (K-PEG3-Unit)A | 38 |
| L039 | A | LG039 | HDKGKSIQDLRRRELLEKLL(Aib)KLH (K-PEG3-Azide)A | LGO039 | HDKGKSIQDLRRRELLEKLL(Aib)KLH (K-PEG3-Unit)A | 39 |
| L040 | A | LG040 | DKGKSIQDLRRRELLEKLL(Aib)KLH (K-PEG3-Azide)A | LGO040 | DKGKSIQDLRRRELLEKLL(Aib)KLH (K-PEG3-Unit)A | 40 |
| L041 | B | LG041 | AVSEHQLLHDKGKSIQDLRRRELLEKLL(Aib)KLHCA | LGO041 | AVSEHQLLHDKGKSIQDLRRRELLEKLL(Aib)KLH (C-S-Unit)A | 41 |
| L042 | B | LG042 | VSEHQLLHDKGKSIQDLRRRELLEKLL(Aib)KLHCA | LGO042 | VSEHQLLHDKGKSIQDLRRRELLEKLL(Aib)KLH (C-S-Unit)A | 42 |
| L043 | B | LG043 | SEHQLLHDKGKSIQDLRRRELLEKLL(Aib)KLHCA | LGO043 | SEHQLLHDKGKSIQDLRRRELLEKLL(Aib)KLH (C-S-Unit)A | 43 |
| L044 | B | LG044 | EHQLLHDKGKSIQDLRRRELLEKLL(Aib)KLHCA | LGO044 | EHQLLHDKGKSIQDLRRRELLEKLL(Aib)KLH (C-S-Unit)A | 44 |
| L045 | B | LG045 | HQLLHDKGKSIQDLRRRELLEKLL(Aib)KLHCA | LGO045 | HQLLHDKGKSIQDLRRRELLEKLL(Aib)KLH (C-S-Unit)A | 45 |
| L046 | B | LG046 | QLLHDKGKSIQDLRRRELIFKTL(Aib)KLHCA | LGO046 | QLLHDKGKSIQDLRRRELIFKTI(Aib)KLH(C-S-Unit)A | 46 |
| L047 | B | LG047 | LLHDKGKSIQDLRRRELIFKTI(Aib)KLHCA | LGO047 | LLHDKGKSIQDLRRRELIFKTI(Aib)KLH(C-S-Unit)A | 47 |
| L048 | B | LG048 | LHDKGKSIQDLRRRELIFKTI(Aib)KLHCA | LGO048 | LHDKGKSIQDLRRRELIFKTI(Aib)KLH(C-S-Unit)A | 48 |
| L049 | B | LG049 | HDKGKSIQDLRRRELIFKTI(Aib)KLHCA | LGO049 | HDKGKSIQDLRRRELIFKTI(Aib)KLH(C-S-Unit)A | 49 |
| L050 | B | LG050 | DKGKSIQDLRRRELIFKTI(Aib)KLHCA | LGO050 | DKGKSIQDLRRRELIFKTI(Aib)KLH(C-S-Unit)A | 50 |
| L051 | C | LG051 | AVSEHQLLHDKGKSIQDLRRRELLEKLL(Aib) KLHTA(K-PEG3-Azide) | LGO051 | AVSEHQLLHDKGKSIQDLRRRELLEKLL(Aib)KLHTA (K-PEG3-Unit) | 51 |
| L052 | C | LG052 | VSEHQLLHDKGKSIQDLRRRELLEKLL(Aib) KLHTA(K-PEG3-Azide) | LGO052 | VSEHQLLHDKGKSIQDLRRRELLEKLL(Aib)KLHTA (K-PEG3-Unit) | 52 |
| L053 | C | LG053 | SEHQLLHDKGKSIQDLRRRELLEKLL(Aib)KLHTA (K-PEG3-Azide) | LGO053 | SEHQLLHDKGKSIQDLRRRELLEKLL(Aib)KLHTA (K-PEG3-Unit) | 53 |
| L054 | C | LG054 | EHQLLHDKGKSIQDLRRRELLEKLL(Aib)KLHTA (K-PEG3-Azide) | LGO054 | EHQLLHDKGKSIQDLRRRELLEKLL(Aib)KLHTA (K-PEG3-Unit) | 54 |
| L055 | C | LG055 | HQLLHDKGKSIQDLRRRELLEKLL(Aib)KLHTA (K-PEG3-Azide) | LGO055 | HQLLHDKGKSIQDLRRRELLEKLL(Aib)KLHTA (K-PEG3-Unit) | 55 |
| L056 | C | LG056 | QLLHDKGKSIQDLRRRELLEKLL(Aib)KLHTA (K-PEG3-Azide) | LGO056 | QLLHDKGKSIQDLRRRELLEKLL(Aib)KLHTA (K-PEG3-Unit) | 56 |
| L057 | C | LG057 | LLHDKGKSIQDLRRRELLEKLL(Aib)KLHTA (K-PEG3-Azide) | LGO057 | LLHDKGKSIQDLRRRELLEKLL(Aib)KLHTA (K-PEG3-Unit) | 57 |
| L058 | C | LG058 | LHDKGKSIQDLRRRELLEKLL(Aib)KLHTA (K-PEG3-Azide) | LGO058 | LHDKGKSIQDLRRRELLEKLL(Aib)KLHTA (K-PEG3-Unit) | 58 |
| L059 | C | LG059 | HDKGKSIQDLRRRELLEKLL(Aib)KLHTA (K-PEG3-Azide) | LGO059 | HDKGKSIQDLRRRELLEKLL(Aib)KLHTA (K-PEG3-Unit) | 59 |
| L060 | C | LG060 | DKGKSIQDLRRRELLEKLL(Aib)KLHTA (K-PEG3-Azide) | LGO060 | DKGKSIQDLRRRELLEKLL(Aib)KLHTA (K-PEG3-Unit) | 60 |
| L061 | C | LG061 | AVSEHQLLHDKGKSIQDLRRRELLEKLL(Aib)K (Ahc)HTA(K-PEG3-Azide) | LGO061 | AVSEHQLLHDKGKSIQDLRRRELLEKLL(Aib)K (Ahc)HTA(K-PEG3-Unit) | 61 |
| L062 | C | LG062 | AVSEHQLLHDKGKSIQDLRRRE(Ahc)LEKLL(Aib) KLHTA(K-PEG3-Azide) | LGO062 | AVSEHQLLHDKGKSIQDLRRRE(Ahc)LEKLL(Aib) KLHTA(K-PEG3 -Unit) | 62 |
| L063 | C | LG063 | AVSEHQLLHDKGKSIQDLRRRELLEK(Ahc)L(Aib)KLH TA(K-PEG3-Azide) | LGO063 | AVSEHQLLHDKGKSIQDLRRRELLEK(Ahc)L(Aib) KLHTA(K-PEG3 -Unit) | 63 |
| L064 | C | LG064 | AVSEHQLLHDKGKSIQDLRRRELLEKLLE(Ahc)LHTA (K-PEG3-Azide) | LGO064 | AVSEHQLLHDKGKSIQDLRRRELLEKLLE(Ahc)LHTA (K-PEG3-Unit) | 64 |
| L065 | C | LG065 | AVSEHQLLHDKGKSIQDLRRR(Cha)LLEK(Ahc)L(Aib) KLHTA(K-PEG3-Azide) | LGO065 | AVSEHQLLHDKGKSIQDLRRR(Cha)LLEK(Ahc)L(Aib) KLHTA(K-PEG3-Unit) | 65 |
| L066 | C | LG066 | AVSEHQLLHDKGKSIQDLRRREL(Ahc)EKLL(Aib)KLH TA(K-PEG3-Azide) | LGO066 | AVSEHQLLHDKGKSIQDLRRREL(Ahc)EKLL(Aib) KLHTA(K-PEG3-Unit) | 66 |
| L067 | C | LG067 | AVSEHQLLHDKGKSIQDLRRRELL(Aib)K(Ahc)LEKLH TA(K-PEG3-Azide) | LGO067 | AVSEHQLLHDKGKSIQDLRRRELL(Aib)K(Ahc)LEKLHTA (K-PEG3-Unit) | 67 |
| L068 | C | LG068 | AVSEHQLLHDKGKSIQDLRRRELL(Aib)K(Ahc)L(Aib) KLHTA(K-PEG3-Azide) | LGO068 | AVSEHQLLHDKGKSIQDLRRRELL(Aib)K(Ahc)L(Aib)KLH TA(K-PEG3-Unit) | 68 |
| L069 | C | LG069 | AVSEHQLLHDKGKSIQDLRRR(Ahc)LLFKTT(Aib)KLH TA(K-PEG3-Azide) | LGO069 | AVSEHQLLHDKGKSIQDLRRR(Ahc)LLFKT T (Aib) KLHTA(K-PEG3 -Unit) | 69 |
| L070 | C | LG070 | AVSEHQLLHDKGKSIQDLRRRELLEKL(Ahc)(Aib)KLH TA(K-PEG3-Azide) | LGO070 | AVSEHQLLHDKGKSIQDLRRRELLEKL(Ahc)(Aib) KLHTA(K-PEG3 -Unit) | 70 |
| L071 | C | LG071 | AVSEHQLLHDKGKSIQDLRRR(Cha)(Ahc)LEKLL(Aib) KLHTA(K-PEG3-Azide) | LGO071 | AVSEHQLLHDKGKSIQDLRRR(Cha)(Ahc)LEKLL(Aib) KLHTA(K-PEG3-Unit) | 71 |
| L072 | C | LG072 | AVSEHQLLHDKGKSIQDLRRRFFLHHLIAEIHTA (K-PEG3-Azide) | LGO072 | AVSEHQLLHDKGKSIQDLRRRFFLHHLIAEIHTA (K-PEG3-Unit) | 72 |
| L073 | C | LG073 | LLHDKGKSIQDLRRRFFLHHLIAEIHTA (K-PEG3-Azide) | LGO073 | LLHDKGKSIQDLRRRFFLHHLIAEIHTA (K-PEG3-Unit) | 73 |
| L074 | C | LG074 | AVSEIQLLHDKGKSIQDLRRRFFLHHLIAEIHTA (K-PEG3-Azide) | LGO074 | AVSEIQLLHDKGKSIQDLRRRFFLHHLIAEIHTA (K-PEG3-Unit) | 74 |
| L075 | C | LG075 | AVSEIQLLHDKGKSIQDLERRFFLHHLIAEIHTA (K-PEG3-Azide) | LGO075 | AVSEIQLLHDKGKSIQDLERRFFLHHLIAEIHTA (K-PEG3-Unit) | 75 |
| L076 | C | LG076 | AVSEIQLLHDKGKSIQDLRRVFFLHHLIAEIHTA (K-PEG3-Azide) | LGO076 | AVSEIQLLHDKGKSIQDLRRVFFLHHLIAEIHTA (K-PEG3-Unit) | 76 |
| L077 | C | LG077 | AVSEIQLLHDKGKSIQDLRRRFFLHHLIAEIHTAEI (K-PEG3-Azide) | LGO077 | AVSEIQLLHDKGKSIQDLRRRFFLHHLIAEIHTAEI (K-PEG3-Unit) | 77 |
| L078 | C | LG078 | AVSEHQLLHDKGKSIQDLRRRFFLHHLIAEIHTAEY (K-PEG3-Azide) | LGO078 | AVSEHQLLHDKGKSIQDLRRRFFLHHLIAEIHTAEY (K-PEG3-Unit) | 78 |
| L079 | C | LG079 | AVSEIQLLHDKGKSIQDLRRRFWLHHLIAEIHTAEY (K-PEG3-Azide) | LGO079 | AVSEIQLLHDKGKSIQDLRRRFWLHHLIAEIHTAEY (K-PEG3-Unit) | 79 |
| L080 | C | LG080 | AVSEIQLLHDKGKSIQDLRRRFWLHHLIAEIHTAEI (K-PEG3-Azide) | LGO080 | AVSEIQLLHDKGKSIQDLRRRFWLHHLIAEIHTAEI (K-PEG3-Unit) | 80 |
| L081 | C | LG081 | VSEIQLLHDKGKSIQDLRRRFWLHHLIAEIHTAEI (K-PEG3-Azide) | LGO081 | VSEIQLLHDKGKSIQDLRRRFWLHHLIAEIHTAEI (K-PEG3-Unit) | 81 |
| L082 | C | LG082 | SEIQLLHDKGKSIQDLRRRFWLHHLIAEIHTAEI (K-PEG3-Azide) | LGO082 | SEIQLLHDKGKSIQDLRRRFWLHHLIAEIHTAEI (K-PEG3-Unit) | 82 |
| L083 | C | LG083 | EIQLLHDKGKSIQDLRRRFWLHHLIAEIHTAEI (K-PEG3-Azide) | LGO083 | EIQLLHDKGKSIQDLRRRFWLHHLIAEIHTAEI (K-PEG3-Unit) | 83 |
| L084 | C | LG084 | IQLLHDKGKSIQDLRRRFWLHHLIAEIHTAEI (K-PEG3-Azide) | LGO084 | IQLLHDKGKSIQDLRRRFWLHHLIAEIHTAEI (K-PEG3-Unit) | 84 |
| L085 | C | LG085 | AVSEHQLLHDKGKSIQDLRRRFWLHHLIAEIHTAEI (K-PEG3-Azide) | LGO085 | AVSEHQLLHDKGKSIQDLRRRFWLHHLIAEIHTAEI (K-PEG3-Unit) | 85 |
| L086 | C | LG086 | AVSEHQLLHDKGKSIQDLRRRFWLHHLIAEIHTAEY (K-PEG3-Azide) | LGO086 | AVSEHQLLHDKGKSIQDLRRRFWLHHLIAEIHTAEY (K-PEG3-Unit) | 86 |
| L087 | C | LG087 | AVSEHQLLHDKGKSIQDLRRRHWLNSYMHKLLVLD AP(K-PEG3-Azide) | LGO087 | AVSEHQLLHDKGKSIQDLRRRHWLNSYMHKLLVLDAP (K-PEG3-Unit) | 87 |
| L088 | C | LG088 | AVSEHQLLHDKGKSIQDLRRREWLRKKLQDVHNF (K-PEG3-Azide) | LGO088 | AVSEHQLLHDKGKSIQDLRRREWLRKKLQDVHNF (K-PEG3-Unit) | 88 |
| L089 | A | LG089 | SVSEIQLMHNLGKHLNSMERVEWLRKKLQDVH (K-PEG3-Azide)F | LGO089 | SVSEIQLMHNLGKHLNSMERVEWLRKKLQDVH (K-PEG3-Unit)F | 89 |
| L090 | A | LG090 | VSEIQLMHNLGKHLNSMERVEWLRKKLQDVH(K-PEG3-Azide)F | LGO090 | VSEIQLMHNLGKHLNSMERVEWLRKKLQDVH (K-PEG3-Unit)F | 90 |
| L091 | A | LG091 | SEIQLMHNLGKHLNSMERVEWLRKKLQDVH(K-PEG3-Azide)F | LGO091 | SEIQLMHNLGKHLNSMERVEWLRKKLQDVH (K-PEG3-Unit)F | 91 |
| L092 | A | LG092 | EIQLMHNLGKHLNSMERVEWLRKKLQDVH(K-PEG3-Azide)F | LGO092 | EIQLMHNLGKHLNSMERVEWLRKKLQDVH (K-PEG3-Unit)F | 92 |
| L093 | A | LG093 | IQLMHNLGKHLNSMERVEWLRKKLQDVH (K-PEG3-Azide)F | LGO093 | IQLMHNLGKHLNSMERVEWLRKKLQDVH (K-PEG3-Unit)F | 93 |
| L094 | A | LG094 | QLMHNLGKHLNSMERVEWLRKKLQDVH (K-PEG3-Azide)F | LGO094 | QLMHNLGKHLNSMERVEWLRKKLQDVH (K-PEG3-Unit)F | 94 |
| L095 | A | LG095 | LMHNLGKHLNSMERVEWLRKKLQDVH (K-PEG3-Azide)F | LGO095 | LMHNLGKHLNSMERVEWLRKKLQDVH (K-PEG3-Unit)F | 95 |
| L096 | A | LG096 | MHNLGKHLNSMERVEWLRKKLQDVH (K-PEG3-Azide)F | LGO096 | MHNLGKHLNSMERVEWLRKKLQDVH (K-PEG3-Unit)F | 96 |
| L097 | A | LG097 | HNLGKHLNSMERVEWLRKKLQDVH (K-PEG3-Azide)F | LGO097 | HNLGKHLNSMERVEWLRKKLQDVH (K-PEG3-Unit)F | 97 |
| L098 | A | LG098 | NLGKHLNSMERVEWLRKKLQDVH (K-PEG3-Azide)F | LGO098 | NLGKHLNSMERVEWLRKKLQDVH (K-PEG3-Unit)F | 98 |
| L099 | B | LG099 | SVSEIQLMHNLGKHLNSMERVEWLRKKLQDVHCF | LGO099 | SVSEIQLMHNLGKHLNSMERVEWLRKKLQDVH (C-S-Unit)F | 99 |
| L100 | B | LG100 | VSEIQLMHNLGKHLNSMERVEWLRKKLQDVHCF | LGO100 | VSEIQLMHNLGKHLNSMERVEWLRKKLQDVH (C-S-Unit)F | 100 |
| L101 | B | LG101 | SEIQLMHNLGKHLNSMERVEWLRKKLQDVHCF | LGO101 | SEIQLMHNLGKHLNSMERVEWLRKKLQDVH (C-S-Unit)F | 101 |
| L102 | B | LG102 | EIQLMHNLGKHLNSMERVEWLRKKLQDVHCF | LGO102 | EIQLMHNLGKHLNSMERVEWLRKKLQDVH (C-S-Unit)F | 102 |
| L103 | B | LG103 | IQLMHNLGKHLNSMERVEWLRKKLQDVHCF | LGO103 | IQLMHNLGKHLNSMERVEWLRKKLQDVH (C-S-Unit)F | 103 |
| L104 | B | LG104 | QLMHNLGKHLNSMERVEWLRKKLQDVHCF | LGO104 | QLMHNLGKHLNSMERVEWLRKKLQDVH (C-S-Unit)F | 104 |
| L105 | B | LG105 | LMHNLGKHLNSMERVEWLRKKLQDVHCF | LGO105 | LMHNLGKHLNSMERVEWLRKKLQDVH (C-S-Unit)F | 105 |
| L106 | B | LG106 | MHNLGKHLNSMERVEWLRKKLQDVHCF | LGO106 | MHNLGKHLNSMERVEWLRKKLQDVH (C-S-Unit)F | 106 |
| L107 | B | LG107 | HNLGKHLNSMERVEWLRKKLQDVHCF | LGO107 | HNLGKHLNSMERVEWLRKKLQDVH (C-S-Unit)F | 107 |
| L108 | B | LG108 | NLGKHLNSMERVEWLRKKLQDVHCF | LGO108 | NLGKHLNSMERVEWLRKKLQDVH (C-S-Unit)F | 108 |
| L109 | C | LG109 | SVSEIQLMHNLGKHLNSMERVEWLRKKLQDVHNF (K-PEG3-Azide) | LGO109 | SVSEIQLMHNLGKHLNSMERVEWLRKKLQDVHNF (K-PEG3-Unit) | 109 |
| L110 | C | LG110 | VSEIQLMHNLGKHLNSMERVEWLRKKLQDVHNF (K-PEG3-Azide) | LGO110 | VSEIQLMHNLGKHLNSMERVEWLRKKLQDVHNF (K-PEG3-Unit) | 110 |
| L111 | C | LG111 | SEIQLMHNLGKHLNSMERVEWLRKKLQDVHNF(K-PEG3-Azide) | LGO111 | SEIQLMHNLGKHLNSMERVEWLRKKLQDVHNF (K-PEG3-Unit) | 111 |
| L112 | C | LG112 | EIQLMHNLGKHLNSMERVEWLRKKLQDVHNF(K-PEG3-Azide) | LGO112 | EIQLMHNLGKHLNSMERVEWLRKKLQDVHNF (K-PEG3-Unit) | 112 |
| L113 | C | LG113 | IQLMHNLGKHLNSMERVEWLRKKLQDVHNF(K-PEG3-Azide) | LGO113 | IQLMHNLGKHLNSMERVEWLRKKLQDVHNF (K-PEG3-Unit) | 113 |
| L114 | C | LG114 | QLMHNLGKHLNSMERVEWLRKKLQDVHNF(K-PEG3-Azide) | LGO114 | QLMHNLGKHLNSMERVEWLRKKLQDVHNF (K-PEG3-Unit) | 114 |
| L115 | C | LG115 | LMHNLGKHLNSMERVEWLRKKLQDVHNF(K-PEG3-Azide) | LGO115 | LMHNLGKHLNSMERVEWLRKKLQDVHNF (K-PEG3-Unit) | 115 |
| L116 | C | LG116 | MHNLGKHLNSMERVEWLRKKLQDVHNF(K-PEG3-Azide) | LGO116 | MHNLGKHLNSMERVEWLRKKLQDVHNF (K-PEG3-Unit) | 116 |
| L117 | C | LG117 | HNLGKHLNSMERVEWLRKKLQDVHNF(K-PEG3-Azide) | LGO117 | HNLGKHLNSMERVEWLRKKLQDVHNF (K-PEG3-Unit) | 117 |
| L118 | C | LG118 | NLGKHLNSMERVEWLRKKLQDVHNF(K-PEG3-Azide) | LGO118 | NLGKHLNSMERVEWLRKKLQDVHNF (K-PEG3-Unit) | 118 |
| L119 | C | LG119 | SVSEIQ(Cha)MHN(Cha)GKHLNSMERVEWLRKKLQD VHNF(K-PEG3-Azide) | LGO119 | SVSEIQ(Cha)MHN(Cha)GKHLNSMERVEWLRKKLQDVHN F(K-PEG3-Unit) | 119 |
| L120 | C | LG120 | SVSEIQLMHNLGKHLNSMERVE(Cha)LRKKLQDVHN F(K-PEG3-Azide) | LGO120 | SVSEIQLMHNLGKHLNSMERVE(Cha)LRKKLQDVHNF (K-PEG3-Unit) | 120 |
| L121 | C | LG121 | SVSEIQLMHNLGKHLNSMERVEW(Cha)RKKLQDVHN F(K-PEG3-Azide) | LGO121 | SVSEIQLMHNLGKHLNSMERVEW(Cha)RKKLQDVHNF (K-PEG3-Unit) | 121 |
| L122 | C | LG122 | SVSEIQL(Nle)HNLGKHLNS(Nle)ERVEWLRK(Cha)LQD VHNF(K-PEG3-Azide) | LGO122 | SVSEIQL(Nle)HNLGKHLNS(Nle)ERVEWLRK(Cha)LQDVH NF(K-PEG3-Unit) | 122 |
| L123 | C | LG123 | SVSEIQLMHNLGKHLNSMERVEWLRKK(Cha)QDVHN F(K-PEG3-Azide) | LGO123 | SVSEIQLMHNLGKHLNSMERVEWLRKK(Cha)QDVHNF (K-PEG3-Unit) | 123 |
| L124 | C | LG124 | SVSEIQLMHNLGKHLNSMERVEWLRKKLQD(Cha)HN F(K-PEG3-Azide) | LGO124 | SVSEIQLMHNLGKHLNSMERVEWLRKKLQD(Cha)HNF (K-PEG3-Unit) | 124 |
| L125 | C | LG125 | SVSEIQLMHNLGKHL(Aib)SMERVEWLRKKLQDVHN F(K-PEG3-Azide) | LGO125 | SVSEIQLMHNLGKHL(Aib)SMERVEWLRKKLQDVHNF (K-PEG3-Unit) | 125 |
| L126 | C | LG126 | SVSEIQLMHNLGKHLNSM(Aib) RVEWLRKKLQDVHNF(K-PEG3-Azide) | LGO126 | SVSEIQLMHNLGKHLNSM(Aib)RVEWLRKKLQDVHNF (K-PEG3-Unit) | 126 |
| L127 | C | LG127 | SVSEIQLMHNLGKHLNSMERVEWLRKKLQDVHN (Aib)(K-PEG3-Azide) | LGO127 | SVSEIQLMHNLGKHLNSMERVEWLRKKLQDVHN(Aib) (K-PEG3-Unit) | 127 |
| L128 | C | LG128 | SVSEIQLMHNLGKHLNSMERVEWLRKKLQD(Nle) HNF(K-PEG3-Azide) | LGO128 | SVSEIQLMHNLGKHLNSMERVEWLRKKLQD(Nle)HNF(K-PEG3-Unit) | 128 |
| L129 | C | LG129 | SVSEIQLMHNLGKHLNSMERVEWLRK(hArg) LQDVHNF(K-PEG3-Azide) | LGO129 | SVSEIQLMHNLGKHLNSMERVEWLRK(hArg)LQDVHNF(K -PEG3-Unit) | 129 |
| L130 | C | LG130 | (Dap)VSEIQL(Nle)HNLGKHLNS(Nle)ERVEWLRKKLQ DVHNY(K-PEG3-Azide) | LGO130 | (Dap)VSEIQL(Nle)HNLGKHLNS(Nle)ERVEWLRKKLQDVH NY(K-PEG3-Unit) | 130 |
| L131 | C | LG131 | SVSEIQLMHNLGKHLNSMERVEW(Cha)RKK(Cha)QK (Cha)HNF(K-PEG3-Azide) | LGO131 | SVSEIQLMHNLGKHLNSMERVEW(Cha)RKK(Cha)QK(Cha) HNF(K-PEG3-Unit) | 131 |
| L132 | C | LG132 | SVSEIQ(Cha)(Nle)HN(Cha)GKHLNS(Nle)ERVEWLRKK LQDVHNY (K-PEG3 -Azide) | LGO132 | SVSEIQ(Cha)(Nle)HN(Cha)GKHLNS(Nle)ERVEWLRKKLQD VHNY(K-PEG3-Unit) | 132 |
| L133 | C | LG133 | SVSEIQ(Cha)(Nle)HN(Cha)GKHL(Aib)S(Nle)(Aib)RVEW LRKKLQDVHNY(K-PEG3-Azide) | LGO133 | SVSEIQ(Cha)(Nle)HN(Cha)GKHL(Aib)S(Nle)(Aib)RVEWLR KKLQDVHNY(K-PEG3-Unit) | 133 |
| L134 | C | LG134 | SVSEIQ(Cha)(Nle)HN(Cha)GKHL(Aib)S(Nle)(Aib)RVEW LRKKLQD(Nle)HNY(K-PEG3-Azide) | LGO134 | SVSEIQ(Cha)(Nle)HN(Cha)GKHL(Aib)S(Nle)(Aib)RVEWLR KKLQD(Nle)HNY(K-PEG3-Unit) | 134 |
| L135 | C | LG135 | SVSEIQLMHNLGKHLN(Cha)MERVEWLRKKLQDVHN F(K-PEG3-Azide) | LGO135 | SVSEIQLMHNLGKHLN(Cha)MERVEWLRKKLQDVHNF (K-PEG3-Unit) | 135 |
| L136 | C | LG136 | SVSEIQLMHNLGKHLNSMERVEWLRKK(Cha)QD(Cha) HNF(K-PEG3-Azide) | LGO136 | SVSEIQLMHNLGKHLNSMERVEWLRKK(Cha)QD(Cha)HN F(K-PEG3-Unit) | 136 |
| L137 | C | LG137 | SVSEIQ(Cha)(Nle)HN(Cha)GKHLNS(Nle)ERVEWLRKK LQDVHN(Aib)(K-PEG3 -Azide) | LGO137 | SVSEIQ(Cha)(Nle)HN(Cha)GKHLNS(Nle)ERVEWLRKKLQD VHN(Aib)(K-PEG3-Unit) | 137 |
| L138 | C | LG138 | SVSEIQLMHNLGKH(Cha)NSMERVEWLRKKLQDVHN F(K-PEG3-Azide) | LGO138 | SVSEIQLMHNLGKH(Cha)NSMERVEWLRKKLQDVHNF (K-PEG3-Unit) | 138 |
| L139 | C | LG139 | SVSEIQ(Cha)MHN(Cha)GKHLNSM(Aib)RVEWLRKKL QDVHNF(K-PEG3-Azide) | LGO139 | SVSEIQ(Cha)MHN(Cha)GKHLNSM(Aib)RVEWLRKKLQDV HNF(K-PEG3-Unit) | 139 |
| L140 | C | LG140 | SVSEIQ(Cha)MHN(Cha)GKHL(Aib)SMERVEWLRKKL QDVHNF(K-PEG3-Azide) | LGO140 | SVSEIQ(Cha)MHN(Cha)GKHL(Aib)SMERVEWLRKKLQDV HNF(K-PEG3-Unit) | 140 |
| L141 | C | LG141 | SVSEIQLMHNLGKHL(Aib)SM(Aib)RVEWLRKKLQDV HNF(K-PEG3-Azide) | LGO141 | SVSEIQLMHNLGKHL(Aib)SM(Aib)RVEWLRKKLQDVHNF (K-PEG3-Unit) | 141 |
| L142 | C | LG142 | SVSEIQLMHNL(Aib)KHLNSMERVEWLRKKLQDVHN F(K-PEG3-Azide) | LGO142 | SVSEIQLMHNL(Aib)KHLNSMERVEWLRKKLQDVHNF (K-PEG3-Unit) | 142 |
| L143 | C | LG143 | SV(Aib)EIQLMHNLGKHLNSMERVEWLRKKLQDVHN F(K-PEG3-Azide) | LGO143 | SV(Aib)EIQLMHNLGKHLNSMERVEWLRKKLQDVHNF (K-PEG3-Unit) | 143 |
| L144 | C | LG144 | SVSEIQ(Cha)MHN(Cha)GKHLNSM(Aib)RVEWLRKKL QKVHNF(K-PEG3-Azide) | LGO144 | SVSEIQ(Cha)MHN(Cha)GKHLNSM(Aib)RVEWLRKKLQKV HNF(K-PEG3-Unit) | 144 |
| L145 | C | LG145 | SVSEIQ(Cha)MHNLGKHLNSMERVEWLRKKLQDVHN F(K-PEG3-Azide) | LGO145 | SVSEIQ(Cha)MHNLGKHLNSMERVEWLRKKLQDVHNF (K-PEG3-Unit) | 145 |
| L146 | C | LG146 | SVSEIQLMHNLGKHLNSMERVEW(Cha)RKK(Cha)QD (Cha)HNF(K-PEG3-Azide) | LGO146 | SVSEIQLMHNLGKHLNSMERVEW(Cha)RKK(Cha)QD(Cha) HNF(K-PEG3-Unit) | 146 |
| L147 | C | LG147 | SVSEIQLMHNLGKHLN(Aib)MERVEWLRKKLQDVHN F(K-PEG3-Azide) | LGO147 | SVSEIQLMHNLGKHLN(Aib)MERVEWLRKKLQDVHNF (K-PEG3-Unit) | 147 |
| L148 | C | LG148 | SVSEIQ(Cha)MHN(Cha)GKH(Cha)NSMERVEWLRKKL QDVHNF(K-PEG3-Azide) | LGO148 | SVSEIQ(Cha)MHN(Cha)GKH(Cha)NSMERVEWLRKKLQDV HNF(K-PEG3-Unit) | 148 |
| L149 | C | LG149 | SVSEIQLMHNLGKHLNSLERVEWLRKKLQDVHNF (K-PEG3-Azide) | LGO149 | SVSEIQLMHNLGKHLNSLERVEWLRKKLQDVHNF (K-PEG3-Unit) | 149 |
| L150 | C | LG150 | (Aib)VSEIQLMHNLGKHLNSMERVEWLRKKLQDVHN F(K-PEG3-Azide) | LGO150 | (Aib)VSEIQLMHNLGKHLNSMERVEWLRKKLQDVHNF (K-PEG3-Unit) | 150 |
| L151 | C | LG151 | SVSEIQLMHNFGKHLNSMERVEWLRKKLQDVHNF (K-PEG3-Azide) | LGO151 | SVSEIQLMHNFGKHLNSMERVEWLRKKLQDVHNF (K-PEG3-Unit) | 151 |
| L152 | C | LG152 | SVSEIQLLHNLGKHLNSMERVEWLRKKLQDVHNF (K-PEG3-Azide) | LGO152 | SVSEIQLLHNLGKHLNSMERVEWLRKKLQDVHNF (K-PEG3-Unit) | 152 |
| L153 | C | LG153 | SVSEIQLMHNL(D-Ser) KHLNSMERVEWLRKKLODVHNF(K-PEG3 -Azide) | LGO153 | SVSEIQLMHNL(D-Ser)KHLNSMERVEWLRKKLQDVHNF (K-PEG3-Unit) | 153 |
| L154 | C | LG154 | SVSEIQLMHNL(D-Leu) KHLNSMERVEWLRKKLQDVHNF(K-PEG3-Azide) | LGO154 | SVSEIQLMHNL(D-Leu)KHLNSMERVEWLRKKLQDVHNF (K-PEG3-Unit) | 154 |
| L155 | C | LG155 | AV(Aib)EIQLMHQ(hArg)AKWLNSMRRVEWLRKKLQ DVHNF(K-PEG3-Azide) | LGO155 | AV(Aib)EIQLMHQ(hArg)AKWLNSMRRVEWLRKKLQDVH NF(K-PEG3-Unit) | 155 |
| L156 | C | LG156 | SVSEIQLMHNLGKHLNSMRRVEWLRKKLQDVHNF (K-PEG3-Azide) | LGO156 | SVSEIQLMHNLGKHLNSMRRVEWLRKKLQDVHNF (K-PEG3-Unit) | 156 |
| L157 | C | LG157 | SVSEIQLMHNLGKHLNSMERVEWLRKKLQDVHNY (K-PEG3-Azide) | LGO157 | SVSEIQLMHNLGKHLNSMERVEWLRKKLQDVHNY (K-PEG3-Unit) | 157 |
| L158 | C | LG158 | SEIQLMHNLGKHLNSMERVEWLRKKLQDVHNY (K-PEG3-Azide) | LGO158 | SEIQLMHNLGKHLNSMERVEWLRKKLQDVHNY (K-PEG3-Unit) | 158 |
| L159 | C | LG159 | SVSEIQLMHNLGKHLNSMERVEFLRKKLQDVHNF (K-PEG3-Azide) | LGO159 | SVSEIQLMHNLGKHLNSMERVEFLRKKLQDVHNF (K-PEG3-Unit) | 159 |
| L160 | C | LG160 | AVSEIQF(Nle)HNLGKHLSS(Nle)ERVEWLRKKLQDVH NY(K-PEG3-Azide) | LGO160 | AVSEIQF(Nle)HNLGKHLSS(Nle)ERVEWLRKKLQDVHNY (K-PEG3-Unit) | 160 |
| L161 | C | LG161 | SEIQF(Nle)HNLGKHLSS(Nle)ERVEWLRKKLQDVHNY (K-PEG3-Azide) | LGO161 | SEIQF(Nle)HNLGKHLSS(Nle)ERVEWLRKKLQDVHNY (K-PEG3-Unit) | 161 |
| L162 | C | LG162 | FMHNL(D-Trp)KHLSSMERVEWLRKKLQDVHNY (K-PEG3-Azide) | LGO162 | FMHNL(D-Trp)KHLSSMERVEWLRKKLQDVHNY (K-PEG3-Unit) | 162 |
| L163 | C | LG163 | AVSEIQLMHNLGKHLASVERMQWLRKKLQDVHNF (K-PEG3-Azide) | LGO163 | AVSEIQLMHNLGKHLASVERMQWLRKKLQDVHNF (K-PEG3-Unit) | 163 |
| L164 | C | LG164 | AVSEIQL(Nle)HNLGKHLASVER(Nle)QWLRKKLQDV HNY(K-PEG3-Azide) | LGO164 | AVSEIQL(Nle)HNLGKHLASVER(Nle)QWLRKKLQDVHNY (K-PEG3-Unit) | 164 |
| L165 | C | LG165 | SLALADDAAFRERARLLAALERRHWLNSYMHKLLV LDAP(K-PEG3-Azide) | LGO165 | SLALADDAAFRERARLLAALERRHWLNSYMHKLLVLDA P(K-PEG3-Unit) | 165 |
| L166 | C | LG166 | ALADDAAFRERARLLAALERRHWLNSYMHKLLVLD AP(K-PEG3-Azide) | LGO166 | ALADDAAFRERARLLAALERRHWLNSYMHKLLVLDAP (K-PEG3-Unit) | 166 |
| L167 | C | LG167 | DAAFRERARLLAALERRHWLNSYMHKLLVLDAP (K-PEG3-Azide) | LGO167 | DAAFRERARLLAALERRHWLNSYMHKLLVLDAP (K-PEG3-Unit) | 167 |
| L168 | C | LG168 | AFRERARLLAALERRHWLNSYMHKLLVLDAP (K-PEG3-Azide) | LGO168 | AFRERARLLAALERRHWLNSYMHKLLVLDAP (K-PEG3-Unit) | 168 |

In Table 4, the modification pattern A is performed by, for example, substituting an amino group in the side chain of lysine, which is the second amino acid from the C terminus of a polypeptide, such as L001-L009, L031-L040, or L080-L098, with a PEG3-azide group to prepare "K-PEG3-azide," and binding an azide group of K-PEG3-azide to 5'-dibenzocyclooctyne-succinyl-hexylamino oligonucleic acid modified with a dibenzocyclooctyne-succinyl-hexylamino group at the 5' terminus of an oligonucleotide, so as to bind a polypeptide, such as L001-L009, L031-L040, or L080-L098, to the oligonucleotide.

In Table 4, the modification pattern C is performed by, for example, substituting an amino group in the side chain of lysine at the C terminus of a polypeptide, such as L021-L030, L051-L088, or L109-L168, with a PEG3-azide group to prepare "K-PEG3-azide," and binding an azide group of K-PEG3-azide to 5'-dibenzocyclooctyne-succinyl-hexylamino oligonucleic acid modified with a dibenzocyclooctyne-succinyl-hexylamino group at the 5' terminus of an oligonucleotide, so as to bind a polypeptide, such as L021-L030, L051-L088, or L109-L168, to the oligonucleotide.

### (K-PEG3-azide)

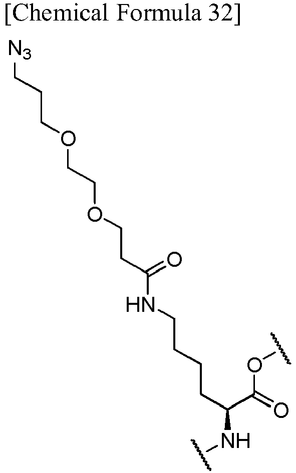

In this method, an example of a linker structure when L001 binds to an oligonucleotide is a K-PEG3-unit. The term "unit" used herein refers to a linker portion on the oligo side of the linker structure. In the structural formula represented by Chemical Formula 33 below, a region from the binding site (a structure in which azide reacts with a DBCO structure) to a phosphoric acid group at the oligo 5' terminus constitutes the "unit."

### (K-PEG3-unit)

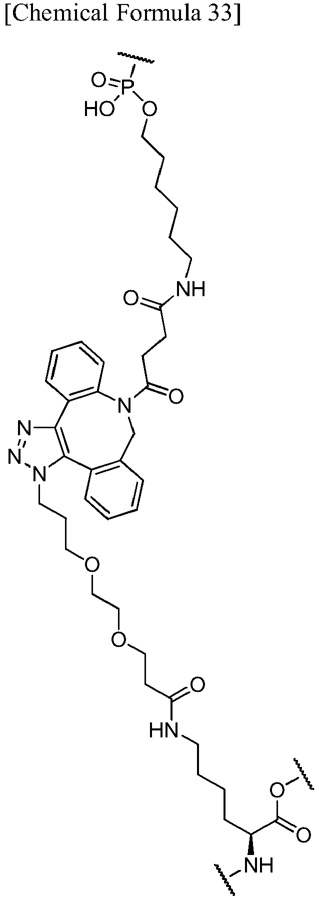

In Table 4, the modification pattern B is performed by, for example, binding thiol of cysteine, which is the second amino acid from the C terminus of a polypeptide, such as L011-L020, L041-L050, or L099-L108, to 5'-3pyridyldithiopropionyl-hexylamino oligonucleotide modified with a 3-pyridyldithiopropionyl group at the 5' terminus via an SS bond and introducing the resultant into an oligonucleotide.

In this method, an example of a linker structure when L099 binds to an oligonucleotide is a C-S-unit. The term "unit" used herein refers to a linker portion on the oligo side of the linker structure. In the structural formula represented by Chemical Formula 34 below, a region from the binding site (an SS bond) to a phosphoric acid group at the oligo 5' terminus constitutes the "unit."

### (C-S-unit)

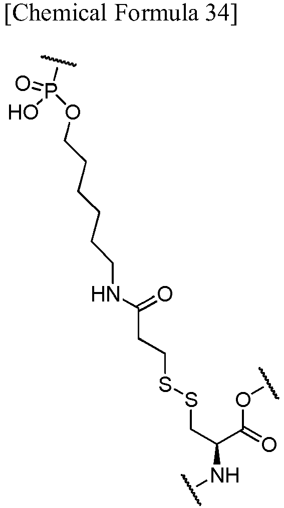

As a method for synthesizing a ligand linker LG001 used to introduce L001 into an oligonucleic acid, the method of solid-phase peptide synthesis developed by Merrifield et al. can be employed. A generally available automated peptide synthesizer may be used. For example, the polypeptide L001 can be synthesized with the use of the automated peptide synthesizer 430A (Applied Biosystems). The resulting peptide-containing resin is subjected to gel filtration to obtain a purified polypeptide.

Other peptides, which are the PTH1 ligands used in the present invention, can be prepared in the same manner as described above by a person skilled in the art.

The peptides shown in Table 1 can be synthesized in accordance with the method described below.

To an amino acid at the C terminus of the synthetic peptide immobilized on a solid-phase support with a cleavable spacer and comprising an adequate protective group, such as a Boc, Fmoc, benzyl, or t-butyl group, introduced into a main chain and a side chain in advance, a reagent that can selectively deprotect an amino protective group in the amino acid main chain is introduced, so as to selectively detach the amino group in the main chain. The deprotected amino group in the main chain is allowed to react with an amino acid to be elongated, which is protected with an adequate protective group having carboxylic acid activated with the addition of a condensation agent, such as HATU (1-[bis(dimethylamino)methylene]-1H-1,2,3-triazolo[4,5-b]pyridinium-3-oxidehexafluorophosphate), and a base, such as DIEA (diisopropylethylamine), to form an amide bond and elongate the main chain toward the N terminus. This process is repeated to synthesize a polypeptide having a target amino acid sequence. In the case of L-001, a protective group in the amine side chain is selectively deprotected, an activated carboxylic acid comprising an azide group, such as 2,5-dioxypyrrolidin-1-yl-2-(2-(2-(2-azidoethoxy)ethoxy)ethoxy)acetate, is then allowed to react with an amino group on the side chain of lysine 33 from the N terminus, so as to introduce an azide group. Thereafter, the remaining protective groups and spacers that bind the solid phase support to a peptide are cleaved. The resulting roughly purified peptides are purified using reversed-phase columns, and the solvent is removed by distillation by lyophilization or other means to obtain the target peptide chain.

In some embodiments, L001, which is a polypeptide represented by SEQ ID NO: 1 shown in Table 1, was used as a PTH1 ligand. The ligand linker LG001 used to introduce L001 (the polypeptide represented by SEQ ID NO: 1) shown in Table 1 was obtained from Peptide Institute, Inc. The purity was found to be approximately 99% by HPLC. The molecular weight determined by mass analysis (ESI-MS) was 4188.8 (it was consistent with the calculated molecular weight 4188.8). L002-L168 can be synthesized in the same manner.

### [Example 2]

The nucleic acid complex of the present invention is not particularly limited, provided that it has an antisense strand that can hybridize to a target gene or a target transcription product and has antisense effects. In the case of a duplex complex, a complementary strand that can hybridize to an antisense strand can further be used in combination. For example, a nucleic acid molecule can be selected from among ASO, HDO, and siRNA comprising the sequences shown below. When a different disease is targeted, the target transcription product is different. Accordingly, a nucleic acid molecule having an antisense strand comprising a relevant nucleic acid sequence in accordance with the target can be used.

In some embodiments, ASO having an antisense strand consisting of any of the nucleic acid sequences shown in Table 5, HDO having a strand complementary to the antisense strand, and/or a nucleic acid complex comprising siRNA and a PTH1 ligand bound thereto can be used.

**[Table 5]**

| Nucleic acid complex | Target gene | Antisense effects/action | Antisense strand! complementary strand | PTH1 ligand | Sequence (5'→3') | SEQ ID NO: |
|---|---|---|---|---|---|---|
| L001-ASO | IHH | RNaseH-dependent antisense effect | Antisense strand | L001 | A*A*T*g*c*a*c*g*t*g*g*G*C*C | 169 |
| L001-HDO | IHH | RNaseH-dependent antisense effect | Antisense strand | | A*A*T*g*c*a*c*g*t*g*g*G*C*C | 169 |
| | IHH | RNaseH-dependent antisense effect | Complementary strand | L001 | *G***G***C**CCACGUGC**A***U***U* | 170 |
| L001-AS02 | APOB | RNaseH-dependent antisense effect | Antisense strand | L001 | G*C*a*t*t*g*g*t*a*t*T*C*A | 171 |
| L001-HD02 | APOB | RNaseH-dependent antisense effect | Antisense strand | | G*C*a*t*t*g*g*t*a*t*T*C*A | 171 |
| | APOB | RNaseH-dependent antisense effect | Complementary strand | L001 | U*G*A*A*UACCAAU*G*C | 172 |
| L001-AS03 | DMPK | RNaseH-dependent antisense effect | Antisense strand | L001 | A*C*A*a*t*a*a*a*t*a*c*c*g*A*G*G | 173 |
| L001-HD03 | DMPK | RNaseH-dependent antisense effect | Antisense strand | | A*C*A*a*t*a*a*a*t*a*c*c*g*A*G*G | 173 |
| | DMPK | RNaseH-dependent antisense effect | Complementary strand | L001 | C*C*U*CGGUAUUUAU*U*G*U | 174 |
| L001-AS04 | mDystrophin | Regulation of pre-mRNA exon 23 splicing | Antisense strand | L001 | *ggccaaacctcggcttacctgaaat* | 175 |
| L001-HD04 | mDystrophin | Regulation of pre-mRNA exon 23 splicing | Antisense strand | | *ggccaaacctcggcttacctgaaat* | 175 |
| | mDystrophin | Regulation of pre-mRNA exon 23 splicing | Complementary strand | L001 | A *U*U*UCAGGUAAGCCGAGGUUUAG*G*C*C | 176 |
| L001-ASOS | IL-1 | Regulation of pre-mRNA exon 9 splicing | Antisense strand | L001 | G*C*A**c*a***c***u***u***c***c***a***a***u*a***c***u***u**A*C*C | 177 |
| L001-HDOS | IL-1 | Regulation of pre-mRNA exon 9 splicing | Antisense strand | | G*C*A**c***a***c***u***u***c***c***a***a***u***a***c***u***u**A*C*C | 177 |
| | IL-1 | Regulation of pre-mRNA exon 9 splicing | Complementary strand | L001 | *G*G*U*aaguauuggaagug*U*G*C* | 178 |
| L001-AS06 | Malat-1 | RNaseH-dependent antisense effect | Antisense strand | L001 | C*T*A*g*t*t*c*a*c*t*g*a*a*T*G*C | 179 |
| L001-HDO6 | Malat-1 | RNaseH-dependent antisense effect | Antisense strand | | C*T*A*g*t*t*c*a*c*t*g*a*a*T*G*C | 179 |
| | Malat-1 | RNaseH-dependent antisense effect | Complementary strand | L001 | *G***C***A**UUCAGUGAAC**U***A***G* | 180 |
| L010-siRNA | Gapdh | RNaseH-independent antisense effect (RNAi) | Antisense strand | | *AuUgUcAuUgAGAgCaAuGcC*a*G* | 181 |
| | Gapdh | RNaseH-independent antisense effect (RNAi) | Complementary strand | L010 | *gGcAuUgCucuCaAuGaCaAu* | 182 |

In Table 5, a lowercase letter represents DNA, an underlined uppercase letter represents LNA (C represents LNA methylcytosine), an uppercase letter represents RNA, an uppercase italic letter represents 2'-O-methyl sugar modification, a lowercase italic letter represents a morpholinonucleic acid, an underlined lowercase italic letter represents 2'-fluoro-sugar modification, and an asterisk represents a phosphorothioate bond.

In Example 2, L001-HDO6 targeting mouse Malat1 (mMalatl) ncRNA was prepared with the use of the antisense strand (SEQ ID NO: 179), the complementary strand (SEQ ID NO: 180), and the PTH1 ligand L001.

### [Chemical Formula 35]

SEQ ID NO: 179:
   5'-C*T*A*g*t*t*c*a*c*t*g*a*a*T*G*C-3'
SEQ ID NO: 180:
   5'-G*C*A*UUCAGUGAAC*U*A*G-3'

A lowercase letter represents DNA, an underlined uppercase letter represents LNA (C represents LNA methylcytosine), an uppercase letter represents RNA, a double-underlined uppercase letter represents 2'-O-methyl sugar modification, and an asterisk represents a phosphorothioate bond.

SEQ ID NO: 179 is an antisense strand consisting of 16 nucleic acids (16mer) targeting mMalatl ncRNA, SEQ ID NO: 179 targets mMalatl ncRNA, and SEQ ID NO: 179 comprises a sequence that can hybridize to the target site of mouse Malat1 ncRNA.

SEQ ID NO: 180 is a complementary strand consisting of 16 nucleic acids (16mer) having a sequence complementary to the antisense strand represented by SEQ ID NO: 179, and SEQ ID NO: 180 comprises a sequence that can hybridize to the antisense strand represented by SEQ ID NO: 179.

A conjugation reaction is performed in the manner described below, so that a ligand molecule can bind to the 5' terminus of the complementary strand represented by SEQ ID NO: 180.

To 5' dibenzocyclooctyne-succinyl-hexylamino oligonucleic acid (1.0 mM 3.0 ml) modified with a dibenzocyclooctyne-succinyl-hexylamino group at the 5' terminus of SEQ ID NO: 180:
[Chemical Formula 36]
SEQ ID NO: 180: 5'-X-G*C*A*UUCAGUGAAC*U*A*G-3' (wherein X represents a dibenzocyclooctyne-succinyl-hexylamino group, an asterisk represents a phosphorothioate bond, a double-underlined uppercase letter represents 2'-O-methyl sugar modification, and an uppercase letter represents RNA), the L001 solution obtained in Example 1 is added, the mixture is agitated, and the resultant is then allowed to stand. After an aqueous solution of sodium chloride is added and the mixture is agitated, alcohol such as 2-isopropanol or an acetonitrile solvent is added, the mixture is agitated, and the resultant is then allowed to stand at -20°C. After the mixture is centrifuged for a given period of time, the solid is precipitated, the supernatant was removed, and the resultant is purified by HPLC. The solvent is removed by distillation, the resultant is dissolved again in water, an aqueous solution of sodium chloride is added thereto, the mixture is agitated, and alcohol is added, followed by centrifugation. Thus, a precipitate; i.e., a L001-binding complementary strand, can be obtained. Subsequently, the antisense strand represented by SEQ ID NO: 179 and the L001-binding complementary strand represented by SEQ ID NO: 180 are added to a microtube at an equimolar concentration and in an equivalent amount, the resultant is agitated, and the microtube is then subjected to incubation at 95°C for 5 minutes. Thereafter, the microtube is allowed to cool to room temperature, so that L001-HDO6 can be prepared.

In the case of the PTH1 ligand introduced in the modification patterns A and C shown in Table 4, ASO, HDO, and siRNA comprising the ligands L002-L010, L021-L040, L051-L098, and L0109-L168 bound thereto can be prepared in the same manner as described above, except for the use of the ligands L002-L010, L021-L040, L051-L098, and L0109-L168 instead of L001.

In the case of the PTH1 ligands introduced in the modification pattern B, ASO, HDO, and siRNA comprising the ligands LG012-LG020, LG041-LG050, and LG099-LG108 bound thereto can be prepared in the same manner as described above, except for substitution of X in Chemical Formula 36 with the 3-pyridyldithiopropionyl group and the use of the ligands LG012-LG020, LG041-LG050, and LG099-LG108 instead of L001.

The HPLC purity, the theoretical molecular weight, and the detected value of the complementary strands into which L001, L003, L005, L010, L021, L040, L089, L098, L098, L165, or L168 had been introduced by the method described above are as shown in Table 6.

**[Table 6]**

| Ligand number | SEQ ID NO: of complementary strand | HPLC purity | Theoretical molecular weight | Detected value |
|---|---|---|---|---|
| L001 | 180 | 98% | 9941.0 | 9941.0 |
| L003 | 180 | 98% | 9770.7 | 9768.2 |
| L005 | 180 | 96% | 9554.6 | 9553.7 |
| L010 | 180 | 96% | 8925.8 | 8923.9 |
| L011 | 180 | 90% | 9499.5 | 9497.2 |
| L021 | 180 | 98% | 10042.1 | 10039.0 |
| L031 | 180 | 99% | 9955.0 | 9951.1 |
| L040 | 180 | 99% | 8939.9 | 8937.4 |
| L089 | 180 | 98% | 10098.1 | 10095.3 |
| L098 | 180 | 97% | 9072.9 | 9070.1 |
| L165 | 180 | 94% | 10598.7 | 10595.0 |
| L168 | 180 | 96% | 9770.8 | 9767.0 |

The antisense strand represented by SEQ ID NO: 179 (0.2 mM, 750 µl) was mixed with the complementary strand comprising L001 bound thereto represented by SEQ ID NO: 180 (0.20 mM, 750 µl) to obtain HDO comprising a ligand bound thereto; i.e., L001-HDO6 (0.1 mM, 1.5 ml).

L001-HDO6 was added to physiological saline (Otsuka Normal Saline (Otsuka Pharmaceutical Co., Ltd.)), the solution was heated at 95°C for 5 minutes, and the solution was slowly cooled to room temperature to form an annealed duplex complex. Thus, the duplex complex L001-HDO6 was prepared.

With the use of an oligonucleic acid comprising L002-L168 introduced thereinto instead of L001 or an oligonucleic acid comprising the sequence of HDO, HDO2, HDO3, HDO4, or HDO5, it is possible to prepare a PTH1 ligand-bound HDO by the same method.

Tm of HDO6 comprising L001, L003, L005, L010, L021, L040, L089, L098, L098, L165, or L168 introduced thereinto is as shown in Table 7.

**[Table 7]**

| Ligand number | Nucleic acid complex | Tm |
|---|---|---|
| L001 | L001-HDO6 | 64°C |
| L003 | L003-HDO6 | 64°C |
| L005 | L005-HDO6 | 64°C |
| L010 | L010-HDO6 | 64°C |
| L011 | L011-HDO6 | 63°C |
| L021 | L021-HDO6 | 62°C |
| L031 | L031-HDO6 | 63°C |
| L040 | L040-HDO6 | 62°C |
| L089 | L089-HDO6 | 60°C |
| L098 | L098-HDO6 | 64°C |
| L165 | L165-HDO6 | 64°C |
| L168 | L168-HDO6 | 64°C |

### [Example 3]

### Reagent

The nucleic acid complex used in Example 3 is L001-HDO6, which is a conjugate of 16-mer HDO targeting mouse Malat1 ncRNA prepared in Example 2 and the L001 ligand. The present inventors adjusted the oligo complex to 100 µM with physiological saline (Otsuka Pharmaceutical Co., Ltd.), and double-stranded HDO was subjected to an annealing procedure comprising denaturing at 90°C for 5 minutes and then naturally cooling to room temperature over a period of about 2 hours in a block bath (CDB-105, AS ONE Corporation).

### Animal

Species differences in the PTH1 receptor, PTH, and PTHrP are insignificant between humans and mice. Accordingly, c57BL/6J mice (25 mice, Charles River Laboratories Japan, Inc., female, 4-week-old when arrived) were used as the subjects. Five mice were raised in a plastic cage at room temperature (24 ± 2°C) at humidity (55 + 5%) under light conditions for 12 hours (8:00 to 20:00), and mice were conditioned for 1 week while given free access to drinking water and a solid feed (MF, Oriental Yeast Co., Ltd.).

### Method

Mice were divided into groups depending on body weights: the vehicle (saline, Otsuka Pharmaceutical Co., Ltd.) administration group; the L001 complementary strand (1 µmol/kg) administration group; and the L001-HDO6 (1 µmol/kg) administration group (each group consisting of 5 mice (n = 5)). On the day of reagent administration, mice were retained in a retainer in the awakened state, and a reagent was slowly administered intravenously through the caudal vein (10 ml/kg, Day 0). The L001 complementary strand (1 µmol/kg) administration group was used as a negative control. After administration, hemostasis at the site of administration was confirmed and the mice were returned to the cages. The mice were subjected to autopsy 3 days after administration (Day 3). On the day of autopsy, body weight of each mouse was measured, a blood sample was taken from the heart under anesthesia with isoflurane (introduced at 3% to 5%, maintained at 1% to 3%) (Terumo syringe SS-01P2525, containing the anticoagulant heparin), and mice were euthanized by exsanguination. The liver and the kidney were extracted by laparotomy. The extracted organs were immediately soaked in ISOGEN and subjected to mRNA extraction. A blood sample was introduced into a 1.5-ml Eppendorf tube and centrifuged at 10,000 rpm for 5 minutes, the supernatant was cryopreserved at -30°C, and the resultant was used for measurement of the levels of liver deviation enzymes; i.e., alanine aminotransferase (ALT) and aspartate aminotransferase (AST), in blood.

Tissue was fractured using a biomasher, GentleMACS Dissociators (Miltenyi Biotec) or FastPrep-24 5G (MP Biomedicals). Total RNA was extracted from the solution containing fractured cells using the ReliaPrep^{™} RNA Tissue Miniprep System (Z6112, Promega Corporation). cDNA was prepared from total RNA by reverse transcription using the PrimeScript^{™} RT Master Mix (RR036A, TaKaRa). mRNA expression of each gene was measured using the real-time PCR system (StepOnePlus, Applied Biosystems). The TaqMan probe set for mouse Malat1 (Mm 01227912-s1, Thermo Fisher Scientific) and 18S rRNA (Mm 03928990-gl, Thermo Fisher Scientific) mRNA was used.

ALT/AST activity in blood was measured using the Test Wako ALT/AST assay kit (431-3090, FUJIFILM Wako Pure Chemical Corporation) and an enzyme calibrator (416-57191, FUJIFILM Wako Pure Chemical Corporation).

All the measured values were indicated in terms of "mean ± standard deviation." For statistical analysis, the one-way analysis of variance (ANOVA) was performed using GraphPad Prism 7.04. For comparison between groups, the Dunnett's multiple comparison test was employed. A risk of lower than 5% is designated significant.

### Result

### 1. General conditions

No apparent changes were observed in coats, behaviors, and stools of mice between before and after administration. The reagent was administered intravenously through the caudal vein at 10 ml/kg, and no abnormality was observed in behavior immediately after administration.

### 2. Body weight

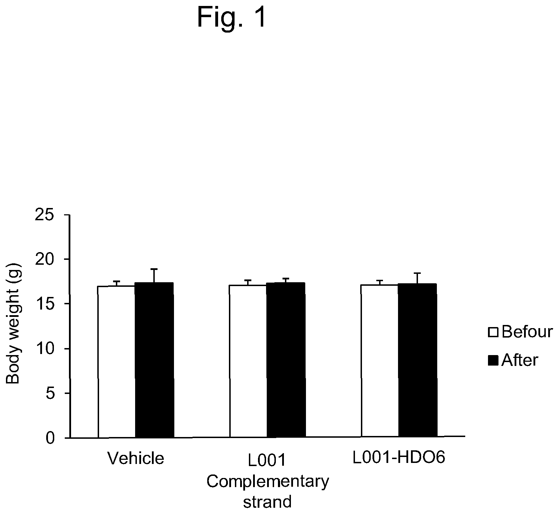

Before administration, the average body weight was 17 g in each animal group, and no significant change was observed between before and after administration. In comparison with the vehicle group, no significant change was observed in the average body weight of the oligo administration group (Figure 1).

### 3. ALT/AST activity in blood

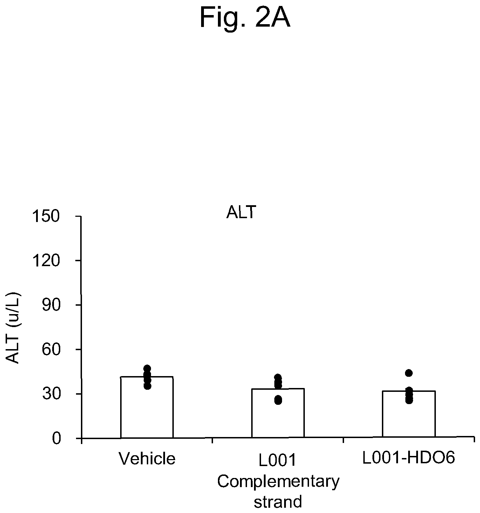

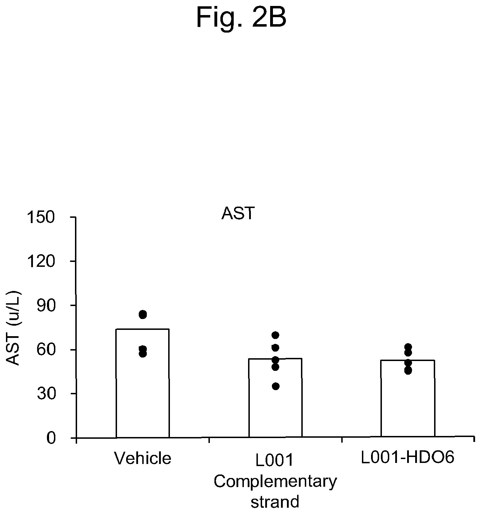

Figure 2A shows changes in ALT activity in blood 3 days after administration of mMalatl oligo to mice. Figure 2B shows changes in AST activity in blood 3 days after administration of mMalatl oligo to mice. No significant changes were observed in ALT activity and AST activity of the mMalatl oligo administration group, compared with those of the vehicle administration group.

### 4. Changes in mMalat1 ncRNA expression in tissue

### 4.1 Liver

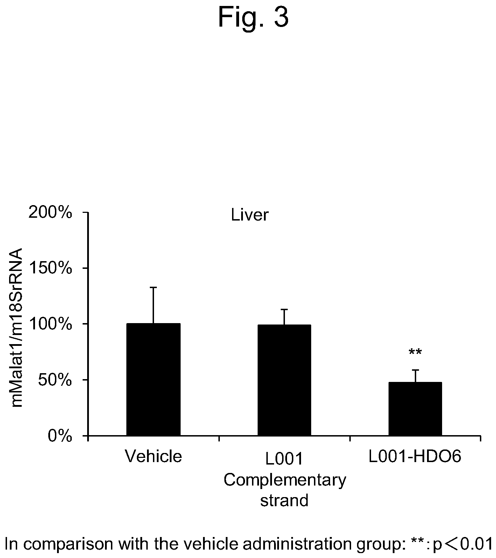

The mMalatl ncRNA expression level in the liver was lowered in the L001-HDO6 administration group to a significant extent, compared with that in the vehicle administration group and the L001 complementary strand administration group (Figure 3).

### 4.2 Kidney

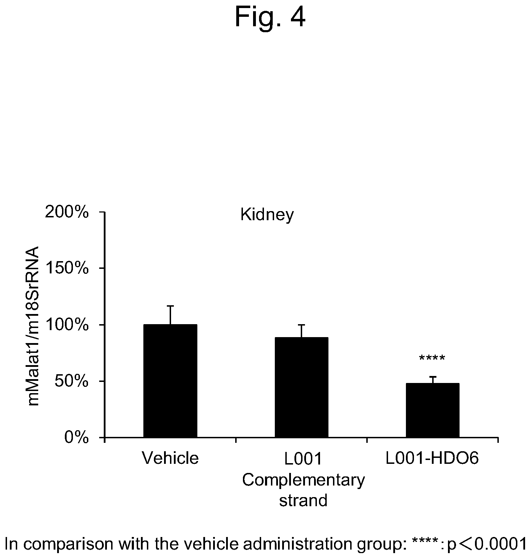

The mMalatl ncRNA expression level in the kidney was lowered in the L001-HDO6 administration group to a significant extent, compared with that in the vehicle administration group and the L001 complementary strand administration group (Figure 4).

In Example 3, L001-HDO6 was administered intravenously to the subjects. As a result, L001-HDO6 was delivered to organs expressing the PTH1 receptor, such as the liver, and expression of a target transcription product; i.e., mMalatl ncRNA, was inhibited.

### [Example 4]

### Histological test based on immunostained image of kidney after administration of L001-HDO6-Alexa488

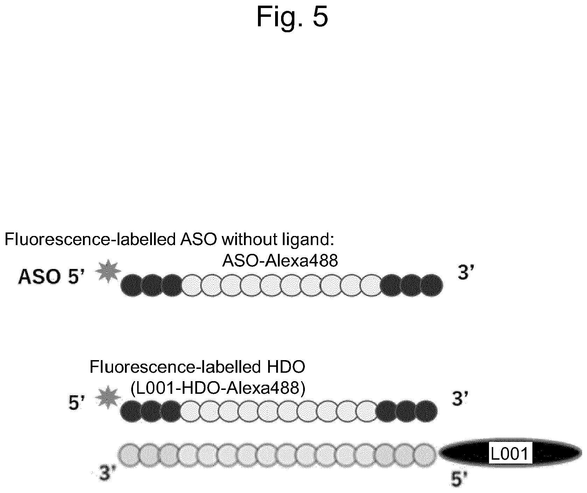

Figure 5 shows the fluorescence-labeled HDO used for the histological test based on immunostained images.

The fluorescence-labeled ASO comprising Alexa488 bound to the 5' terminus of the antisense strand (SEQ ID NO: 179) prepared in Example 2 was annealed to the L001 complementary strand (SEQ ID NO: 180) by the method described in Example 2 to form a duplex complex. Thus, the fluorescence-labeled HDO (L001-HDO6-Alexa488) was prepared.

The immunohistochemical test was performed with the use of c57BL/6J mice (12 mice). The fluorescence-labeled HDO (L001-HDO6-Alexa488) was intravenously administered to mice at 1 µmol/kg once, a blood sample was taken from the heart under anesthesia with isoflurane 10 minutes, 6 hours, 24 hours, and 72 hours later, the kidney was perfused by left ventricular cannulation with 10 ml of saline followed by 10 ml of 4% paraformaldehyde solution, and the kidney was extracted from the mice. The extracted kidney was soaked in 4% paraformaldehyde to prepare frozen sections. The frozen sections were stained with Nephrin (the primary antibody: mouse Nephrin affinity purified polyclonal antibody, Goat IgG (AF3459), R&D Systems) and Alexa Fluor 594 (the secondary antibody: chicken anti-goat IgG (H+L) (A-21468), Invitrogen) to observe the renal glomerulus, and the frozen sections were stained with a DAPI (4',6-diamidino-2-phenylindole) solution to observe the DNA-containing cell nuclei. The fluorescent images of the pathological sections were incorporated with the use of the Olympus VS120 Slide Scanning System.

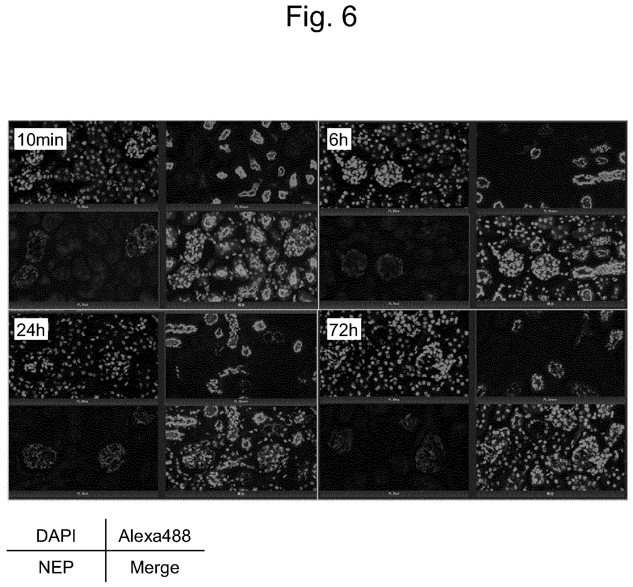

Figure 6 shows the immunostained images of the kidney obtained 10 minutes, 6 hours, 24 hours, and 72 hours after a single intravenous administration of the fluorescence-labeled HDO (L001-HDO6-Alexa488) at 1 µmol/kg to the c57BL/6J mice. The photographs demonstrating the images after given periods of time after administration are each composed of four photographs. The photograph in the upper left is an ultraviolet (UV) fluorescent image demonstrating the results of DAPI staining. The photograph in the lower left is a red fluorescent image (red-Alexa594) demonstrating the results of staining with the anti-Nephrin antibody. The photograph in the upper right is a green fluorescent image demonstrating the results of staining of L001-HD06-Alexa488 (green-Alexa488). The photograph in the lower right is an image obtained by superposing the aforementioned 3 photographs superposed on top of each other (merged).

### Results

### 1 Glomerulus

In the glomerulus, the fluorescence Alexa-488 was slightly observed 10 minutes after administration of the L001-HDO6 reagent, although no fluorescence was observed 6, 24, or 72 hours after administration.

### 2 Kidney tubule

In kidney tubular cells, the fluorescence Alexa-488 was observed 10 minutes after the administration of the L001-HDO6 reagent, the fluorescence was intensified 6 hours after the administration, the maximum fluorescence was observed 24 hours after the administration, and the fluorescence was still observed in many kidney tubules 72 hours after the administration.

In Example 4, L001-HDO6 was administered intravenously to the subjects. As a result, L001-HDO6 was delivered to organs expressing the PTH1 receptor, such as the liver, and accumulated in the kidney tubules in the liver for 72 hours.

### [Example 5]

The nucleic acid complexes used in Example 5 are each a conjugate of 16-mer HDO or ASO targeting mouse Malatl ncRNA prepared in the example above and the PTH1 ligand. The present inventors adjusted the oligo complex with physiological saline (Otsuka Pharmaceutical Co., Ltd.), the oligo complex for animal evaluation was denatured in a block bath (CDB-105, AS ONE Corporation), and the double-stranded HDO for cell evaluation was subjected to an annealing procedure comprising denaturing at 90°C for 10 minutes and then naturally cooling to room temperature over a period of about 2 hours in a T100 thermal cycler (Bio-Rad). The oligo complex for animal evaluation was adjusted to the final concentration of 100 nmol/ml with physiological saline, and the oligo complex for cell evaluation was adjusted to various concentrations with 0.1% FCS in DMEM (Invitrogen).

### Animal

c57BL/6J mice (Japan SLC, Inc., female, 4-week-old when arrived) were used. Five mice were raised in a plastic cage at room temperature (24 ± 2°C) at humidity (55 + 5%) under light conditions for 12 hours (7:00 to 19:00), and mice were conditioned for 1 week while given free access to drinking water and a solid feed (MF, Oriental Yeast Co., Ltd.).

### Method of in vivo evaluation

Mice were divided into groups depending on body weights: the vehicle (saline, Otsuka Pharmaceutical Co., Ltd.) administration group; and various PTH1 ligand-HDO (1 µmol/kg) administration groups (each group consisting of 5 mice (n = 5)). On the day of reagent administration, mice were retained in a retainer in the awakened state, and a reagent was slowly administered intravenously through the caudal vein or subcutaneously through the back of the neck (10 ml/kg, Day 0). After administration, hemostasis at the site of administration was confirmed and the mice were returned to the cages. The mice were subjected to autopsy 3 days after administration (Day 3). On the day of autopsy, mice were euthanized by exsanguination under anesthesia with isoflurane (introduced at 3% to 5%, maintained at 1% to 3%). The liver, the kidney, the lung, the heart, and the thigh muscle were extracted by laparotomy. The extracted organs were immediately soaked in ISOGEN and subjected to mRNA extraction.

For mRNA extraction, tissue was fractured by bead crushing using a TissueLyser II (QIAGEN). Total RNA was extracted from the solution containing fractured tissue using the ReliaPrep^{™} RNA Tissue Miniprep System (Promega Corporation). cDNA was prepared from total RNA by reverse transcription using the PrimeScript^{™} RT Master Mix (TaKaRa). mRNA expression of each gene was measured using the real-time PCR system (StepOnePlus, Applied Biosystems). The TaqMan probe set for mouse Malatl (Mm 01227912-s1, Applied Biosystems) and mouse Gapdh (Mm99999915_g1, Applied Biosystems) mRNA was used.

All the measured values were indicated in terms of "mean ± standard deviation." Statistical analysis was performed using GraphPad Prism 9.4.0. Comparison between groups was performed by the one-way analysis of variance (ANOVA) followed by the Dunnett's multiple comparison test. Comparison between two independent samples was performed by the Student's t-test. A risk of lower than 5% was designated statistically significant.

### Results

### 1. Examination ofPHTl ligand with modified amino acid sequence length of peptide

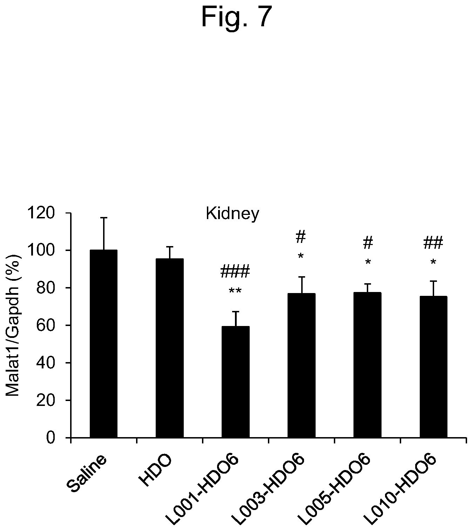

The Malatl ncRNA expression in the kidney was not inhibited to a statistically significant extent in the HDO intravenous administration group without the PTH1 ligand (HDO). In the HDO intravenous administration groups with the PTH1 ligand (L001-HDO6, L003-HDO6, L005-HDO6, and L010-HDO6), however, the Malatl ncRNA expression was lowered to a statistically significant extent, compared with the saline administration group. In comparison with HDO, the Malatl ncRNA expression in the kidney was lowered to a statistically significant extent in the HDO intravenous administration groups with the PTH1 ligand (L001-HDO6, L003-HDO6, L005-HDO6, and L010-HDO6). The results are shown in Figure 7.

L001 is a peptide consisting of 34 amino acids, L003 is a peptide consisting of 32 amino acids that lacks 2 amino acids from the N terminus of L001, L005 is a peptide consisting of 30 amino acids that lacks 4 amino acids from the N terminus of L001, and L010 is a peptide consisting of 25 amino acids that lacks 9 amino acids from the N terminus of L001.

HDO comprising a peptide consisting of 34 amino acids of L001 bound thereto was found to inhibit expression of the target gene. In addition, HDO comprising a peptide consisting of 32, 30, or 25 amino acids that lacks 2, 4, or 9 amino acids from the N terminus of a peptide consisting of 34 amino acids was found to inhibit expression of the target gene. Thus, a peptide in which at least 9 amino acids are deleted from the N terminus of a peptide was found to exert antisense effects.

In Figure 7 and other figures, the symbols * and *** indicate P < 0.05 and P < 0.001 (Dunnett) in comparison with the vehicle administration group (vs saline), and the symbols #, ##, and ### indicate P < 0.05, P < 0.01, and P < 0.001 (Dunnett) in comparison with the vehicle administration group (vs HDO).

### 2. Subcutaneous administration

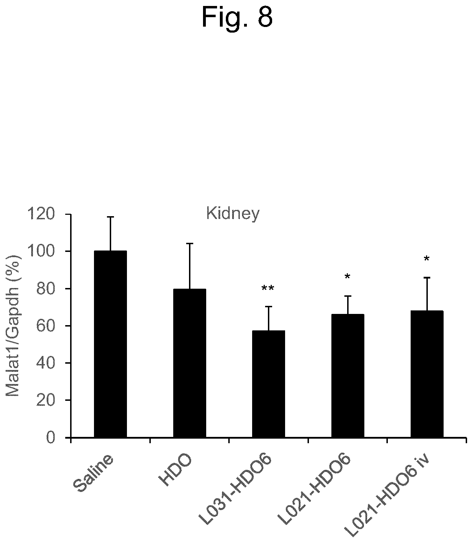

The Malatl ncRNA expression in the kidney was not inhibited to a statistically significant extent in the HDO subcutaneous administration group without the ligand (HDO). In the HDO subcutaneous administration groups with the ligand (L031-HDO6 and L021-HDO6), however, the Malatl ncRNA expression was lowered to a statistically significant extent, compared with the saline administration group (Figure 8). L021-HDO6 was found to have significant inhibitory activity on the target gene, and such inhibitory activity was equivalent between the subcutaneous administration group and the intravenous administration group.

L031 is a peptide consisting of 35 amino acids, and L021 is a peptide consisting of 34 amino acids. Differences in the amino acid sequences of the peptides are the presence or absence of C-terminal T and the position of K (Lys). Both peptides were found to inhibit expression of the target gene at a significant level.

### 3. In vitro assays

### Cells

The NIH/3T3 cell line stably expressing mouse PthlR (mPthlR) using the PiggyBac transposon method was used. The mPthlR expressing stable clones used for evaluation of PTH1 ligand actions are 33K cAMP-inducing cells having agonist activity with IC50 of approximately 100 pM.

### Method of in vitro evaluation

Cells of the mPthlR-stably expressing cell line were inoculated at 1.5 × 10⁴ cells/well on a 96-well plate. The medium was suction-removed from each well 24 hours after inoculation, and PTH1 ligand-HDO adjusted to various concentrations with DMEM containing 0.1% FBS was added at 100 µl/well. Total RNA was extracted from the cells using the RNA purification kit of the SV96 Total RNA Isolation System (Promega) in accordance with the instructions thereof. Specifically, the medium was suction-removed from each well 24 hours after the addition of PTH1 ligand-HDO, and the wells were then washed with PBS. PBS was suction-removed, and the RNA Lysis Buffer included in the RNA purification kit was added at 100 µl/well for cell lysis. The subsequent procedure was implemented in accordance with the SV96 Total RNA Isolation System (Promega) to obtain total RNA. Total RNA was reverse-transcribed into cDNA with the use of PrimeScript^{™} RT Master Mix (TaKaRa). mRNA expression of each gene was measured using the real-time PCR system (Step One Plus, Applied Biosystems). The target gene was analyzed using Malatl (Mm 01227912-s1, Applied Biosystems), and the TaqMan probe of Gapdh (Mm99999915_g1, Applied Biosystems) was used as the internal reference gene.

All the measured values were indicated in terms of "mean ± standard deviation" (N = 3). IC50 was calculated using GraphPad Prism 9.4.0.

### 3.1. L021-HDO6, L003-HDO6, L005-HDO6, and L010-HDO6

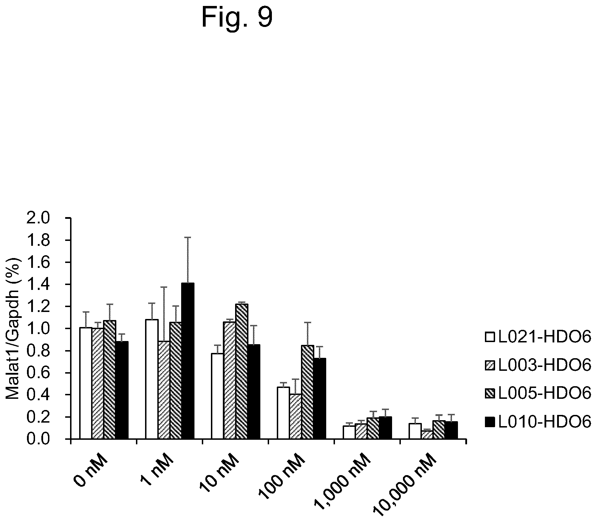

All of L021-HDO6, L003-HDO6, L005-HDO6, and L010-HDO6 lowered the Malatl ncRNA expression levels in a concentration-dependent manner, and IC50 values thereof were 46.8 nM, 88.9 nM, 121.3 nM, and 166.7 nM (Figure 9). HDO comprising the PTH1 ligand consisting of the peptide represented by SEQ ID NO: 21, SEQ ID NO: 3, SEQ ID NO: 5, SEQ ID NO: 6, or SEQ ID NO: 10 bound thereto was found to exert concentration-dependent antisense effects on the target gene.

### 3.2. L021-HDO6 and L011-HDO6

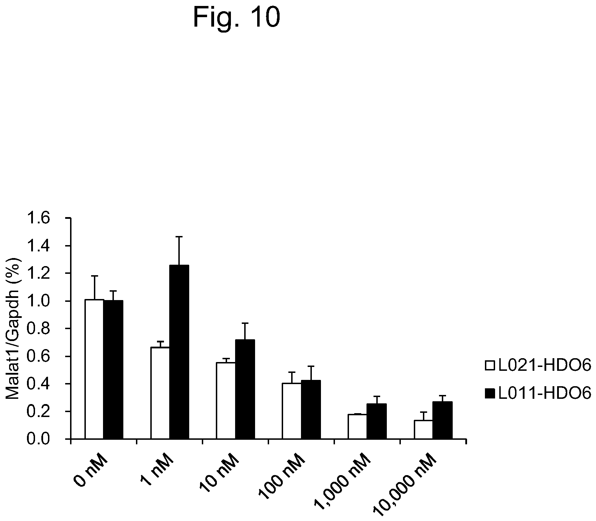

Both L021-HDO6 and L011-HDO6 lowered the Malat1 ncRNA expression levels in a concentration-dependent manner, and IC50 values thereof were 6.94 to 10.2 nM and 11.8 to 14.1 nM (Figure 10). HDO comprising the PTH1 ligand consisting of the peptide represented by SEQ ID NO: 21 or SEQ ID NO: 11 bound thereto was found to exert concentration-dependent antisense effects on the target gene.

### 3.3. L031-HDO6 and L040-HDO6

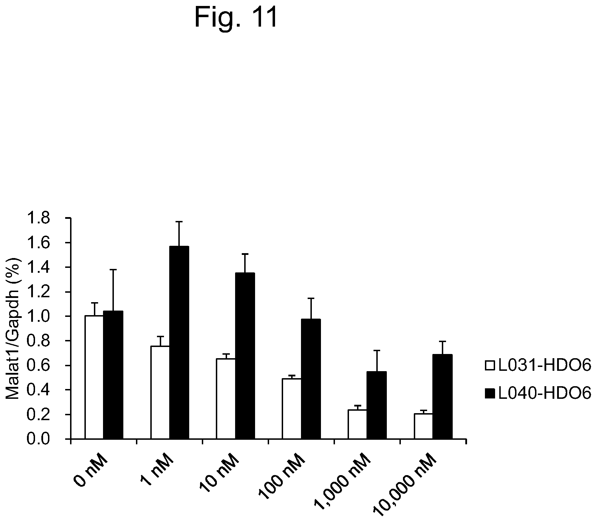

Both L031-HDO6 and L040-HDO6 lowered the Malat1 ncRNA expression levels in a concentration-dependent manner, and IC50 values thereof were 41.5 to 71 nM and 92.2 to 111 nM (Figure 11). HDO comprising the PTH1 ligand consisting of the peptide represented by SEQ ID NO: 31 or SEQ ID NO: 40 bound thereto was found to exert concentration-dependent antisense effects on the target gene.

### 3.4. L089-HDO6 and L098-HDO6

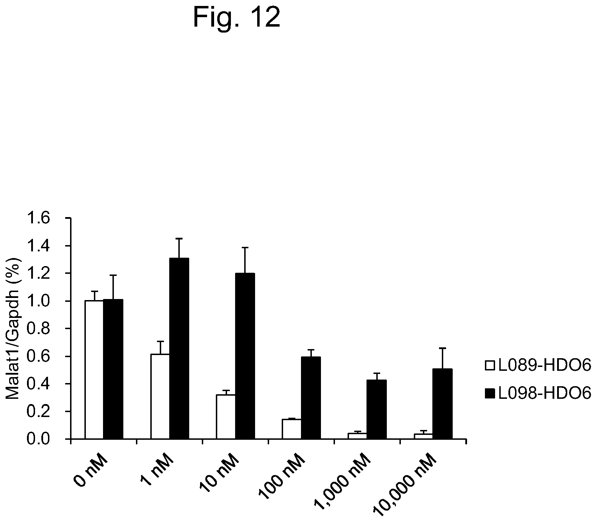

Both L089-HDO6 and L098-HDO6 lowered the Malatl ncRNA expression levels in a concentration-dependent manner, and IC50 values thereof were 1.14 to 1.61 nM and 47.7 to 82.7 nM (Figure 12). HDO comprising the PTH1 ligand consisting of the peptide represented by SEQ ID NO: 89 or SEQ ID NO: 98 bound thereto was found to exert concentration-dependent antisense effects on the target gene.

### 3.5. L165-HDO6 and L168-HDO6

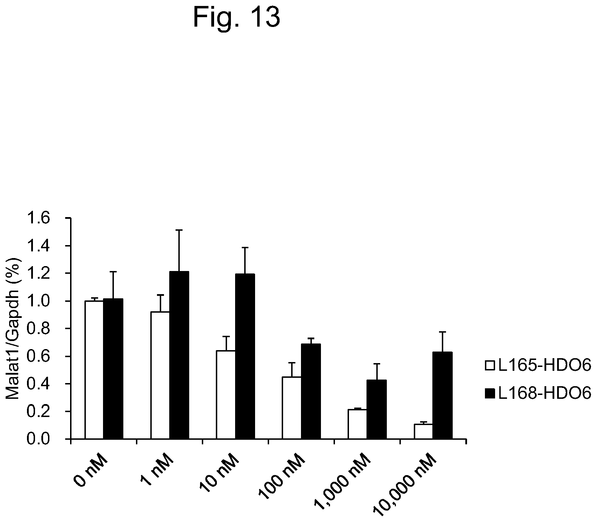

Both L165-HDO6 and L168-HDO6 lowered the Malatl ncRNA expression levels in a concentration-dependent manner, and IC50 values thereof were 27.8 to 185 nM and 11.4 to 96.5 nM (Figure 13). HDO comprising the PTH1 ligand consisting of the peptide represented by SEQ ID NO: 165 or SEQ ID NO: 168 bound thereto was found to exert concentration-dependent antisense effects on the target gene.

### 3.6. L001-ASO

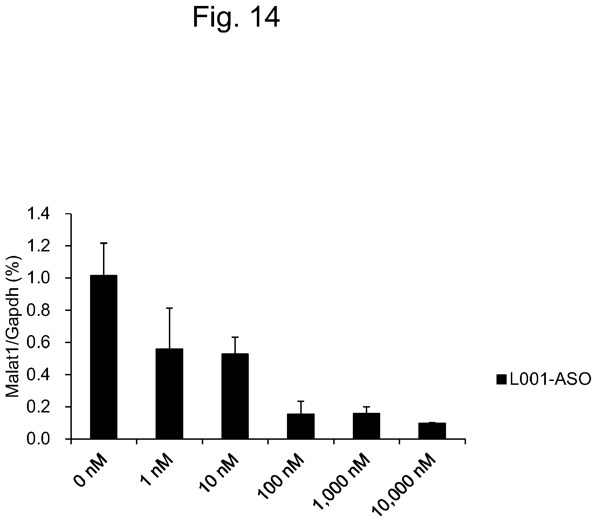

L001-ASO, which is ASO comprising the PTH1 ligand L001 bound thereto, lowered the Malatl ncRNA expression levels in a concentration-dependent manner, and IC50 thereof was 1.25 nM (Figure 14). ASO comprising the PTH1 ligand consisting of the peptide represented by SEQ ID NO: 1 bound thereto was found to exert concentration-dependent antisense effects on the target gene. The PTH1 ligand of the present invention was found to bind to a single-stranded nucleic acid molecule and exert concentration-dependent antisense effects on the target gene.

### [Example 6]

The nucleic acid complex of the present invention is not particularly limited, provided that it has an antisense strand that can hybridize to a target gene or a target transcription product and exerts antisense effects. In the case of a duplex nucleic acid complex, a combination of an antisense strand and a complementary strand that can hybridize thereto can be employed. For example, siRNA consisting of the sequence indicated below can be selected. When a different disease is a target, a target transcription product is different. Accordingly, a nucleic acid molecule having an antisense strand comprising a nucleic acid sequence in accordance with a relevant target can be used.

In some embodiments, a nucleic acid complex can comprise siRNA consisting of the antisense strand (SEQ ID NO: 181) and the guide strand (SEQ ID NO: 182) indicated below.

SEQ ID NO: 181:
   [Chemical Formula 37]
   5'-*AuUgUcAuUgAGAgCaAuGcC*a*G*-3'
SEQ ID NO: 182:
   [Chemical Formula 38]
   5'-*gGcAuUgCucuCaAuGaCaAu-*3'

In SEQ ID NOs: 181 and 182, an uppercase italic letter represents 2'-O-methyl sugar modification, an underlined lowercase italic letter represents 2'-fluoro-sugar modification, and an asterisk represents a phosphorothioate bond.

SEQ ID NO: 181 is an antisense strand consisting of 23 nucleic acids (23mer) targeting mGapdh mRNA, SEQ ID NO: 181 targets mGapdh mRNA, and SEQ ID NO: 181 comprises a sequence that can hybridize to the target site of mouse Gapdh mRNA.

SEQ ID NO: 182 is a complementary strand consisting of 23 nucleic acids (23mer) having a sequence complementary to the antisense strand represented by SEQ ID NO: 181, and SEQ ID NO: 182 comprises a sequence that can hybridize to the antisense strand represented by SEQ ID NO: 181.

A conjugation reaction is performed in the same manner as with the case of HDO, so that a ligand molecule can bind to the 5' terminus of the complementary strand represented by SEQ ID NO: 182.

The PTH1 ligand-binding nucleic acid complex used in the present example is L010-siRNA, which is a conjugate of the 23mer siRNA targeting mouse Gapdh mRNA and the PTH1 ligand L010 bound thereto in accordance with the method described in Example 2. The present inventors adjusted the oligo complex with physiological saline (Otsuka Pharmaceutical Co., Ltd.), and siRNA for cell evaluation was subjected to an annealing procedure comprising denaturing at 95°C for 10 minutes and then cooling to room temperature over a period of about 2 hours in a T100 thermal cycler (Bio-Rad). The oligo complex for animal evaluation was adjusted to the final concentration of 100 nmol/ml with physiological saline, and the oligo complex for cell evaluation was adjusted to various concentrations with Opti-MEM (Invitrogen).

### Cell

The NIH/3T3 cell line stably expressing mouse PthlR (mPthlR) using the PiggyBac transposon method was used.

### Method of in vitro evaluation

Cells of the mPthlR-stably expressing cell line were inoculated at 3.75 × 10³ cells/well on a 96-well plate. The medium was suction-removed from each well 4 hours after inoculation, and L010-siRNA adjusted to various concentrations with Opti-MEM was added at 100 µl/well. Total RNA was extracted from the cells using the RNA purification kit of the SV96 Total RNA Isolation System (Promega) in accordance with the instructions thereof. Specifically, the medium was suction-removed from each well 72 hours after the addition of L010-siRNA, and the wells were then washed with PBS. PBS was suction-removed, and the RNA Lysis Buffer included in the RNA purification kit was added at 100 µl/well for cell lysis. The subsequent procedure was implemented in accordance with the SV96 Total RNA Isolation System to obtain total RNA. Total RNA was reverse-transcribed into cDNA with the use of PrimeScript RT Mster Mix (TaKaRa). mRNA expression of each gene was measured using the real-time PCR system (Step One Plus, Applied Biosystems). The target gene was analyzed using Gapdh (Mm99999915_g1, Applied Biosystems), and the TaqMan probe of Actb (Mm01205647_g1, Applied Biosystems) was used as the internal reference gene. All the measured values were indicated in terms of "mean ± standard deviation" (n = 4).

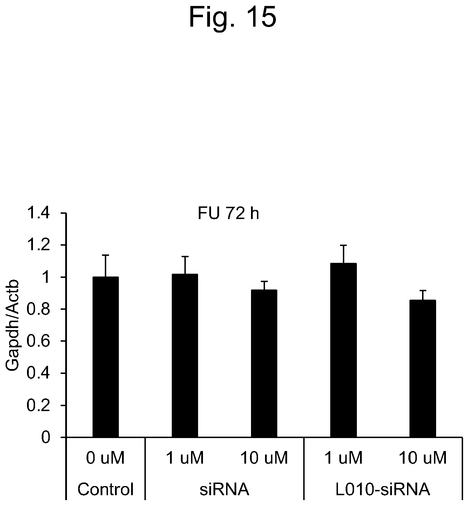

The test results are shown in Figure 15. siRNA (10 µM) comprising L010 as a PTH1 ligand bound thereto lowered the mGapdh mRNA expression level by 14%, compared with the control (0 µM) administration group. The PTH1 ligand of the present invention exerted antisense effects on the target gene when it bound to siRNA.

### Industrial Applicability

The ligand-binding nucleic acid complex of the present invention can be used for nucleic acid medicine that is delivered to an organ having the PTH1 receptor to regulate expression or editing of a target gene or a transcription product thereof.

### Sequence Listing Free Text

### SEQ ID NOs: 1 to 182: synthetic sequences

All publications, patents, and patent applications cited herein are incorporated herein by reference in their entirety.

## Claims

1. A ligand-binding nucleic acid complex in which a nucleic acid molecule that regulates expression or editing of a target gene or a transcription or translation product thereof binds to a PTH1 ligand with or without a linker.

2. The ligand-binding nucleic acid complex according to claim 1, wherein the PTH1 ligand is a peptide consisting of the amino acid sequence represented by the following formula:
[Chemical Formula 1] A₁-A₂-A₃-A₄-A₅-A₆-A₇-A₈-A₉-A₁₀-A₁₁-A₁₂-A₁₃-A₁₄-A₁₅-A₁₆-A₁₇-A₁₈-A₁₉-A₂₀-A₂₁-A₂₂-A₂₃-A₂₄-A₂₅-A₂₆-A₂₇-A₂₈-A₂₉-A₃₀-A₃₁-A₃₂-A₃₃-A₃₄
wherein the amino acid sequence is arranged in order from the N terminus toward the C terminus;
A1 represents Ala, Ser, or Dap or is deleted;
A2 represents Val or is deleted;
A3 represents Ser, Thr, or Aib or is deleted;
A4 represents Glu or is deleted;
A5 represents Leu, His, Nle, Ile, Cha, β-Nal, Trp, Pal, Acc, or Phep-X-Phe, wherein X represents OH, halogen, or CH₃, or is deleted;
A6 represents Gin;
A7 represents Leu, Nle, Ile, Cha, β-Nal, Trp, Pal, Acc, or Phep-X-Phe, wherein X represents OH, halogen, or CH₃, or is deleted;
A8 represents Met, Nva, Leu, Val, Ile, Cha, Acc, or Nle or is deleted;
A9 represents His or is deleted;
A10 represents Asp or Asn;
A11 represents Leu, Lys, Nle, Ile, Cha, β-Nal, Trp, Pal, Acc, Phe, or p-X-Phe, wherein X represents OH, halogen, or CH3;
A12 represents Gly, Acc, or Aib;
A13 represents Lys;
A14 represents Ser or His;
A15 represents Leu, Nle, Ile, Cha, β-Nal, Trp, Pal, Ace, Phe, or P-X-Phe, wherein X represents OH, halogen, or CH3;
A16 represents Ser, Gin, Asn, Ala, or Aib;
A17 represents Ser, Asp, Thr, or Aib;
A18 represents Met, Nva, Leu, Val, Ile, Nle, Ace, Cha, or Aib;
A19 represents Arg, Glu, or Aib;
A20 represents Arg;
A21 represents Arg, Val, Ace, Cha, or Met;
A22 represents Phe, Glu, Aib, Ace, or Cha;
A23 represents Phe, Trp, Leu, Lys, Ace, or Cha;
A24 represents Leu, Lys, Ace, or Cha;
A25 represents His, Arg, Lys, Aib, Ace, or Glu;
A26 represents His, Aib, Ace, or Lys;
A27 represents Lys, Aib, Leu, hArg, Gin, Ace, or Cha;
A28 represents Ile, Leu, Lys, Ace, or Cha;
A29 represents Ala, Glu, Ace, or Aib;
A30 represents Glu, Asp, Leu, Nle, Cha, Aib, Ace, or Lys;
A31 represents Ile, Val, Leu, Nle, Cha, Lys, or Acc;
A32 represents His;
A33 represents Thr, Asn, Lys, or Cys; and
A34 represents Phe, Ala, Tyr, Amp, or Aib.

3. The ligand-binding nucleic acid complex according to claim 1, wherein the PTH1 ligand is a peptide consisting of a sequence selected from the group consisting of SEQ ID NO: 1 to SEQ ID NO: 168.

4. The ligand-binding nucleic acid complex according to claim 2, wherein the PTH1 ligand is a peptide consisting of a sequence selected from the group consisting of SEQ ID NO: 1 to SEQ ID NO: 164.

5. The ligand-binding nucleic acid complex according to claim 4, wherein the nucleic acid molecule binds to the PTH1 ligand with a linker.

6. The ligand-binding nucleic acid complex according to claim 5, wherein the linker is a cleavable linker having a cleavable structure.

7. The ligand-binding nucleic acid complex according to claim 6, wherein the linker has the structure shown below.
**[Table 1]**
| Linker type | Structural formula |
|---|---|
| Having a disulfide bond | 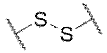 |
| Having an amide bond (n=0-9) | 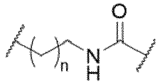 |
| Having a polyethylene glycol group (n=1-200) | 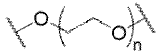 |
| Having a phosphate group | 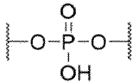 |
| Having a pyrrolidinyl group | 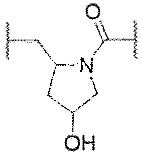 |
| Having a BCN group | 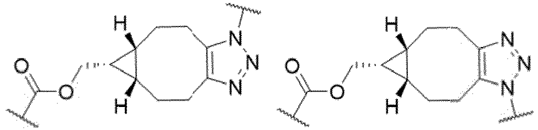 |
| Having a DBCO group | 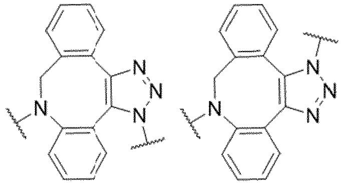 |
| Having a maleimide thioether group | 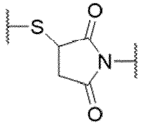 |

8. The ligand-binding nucleic acid complex according to claim 1, wherein the nucleic acid molecule is selected from the group consisting of a nucleic acid molecule comprising an antisense strand consisting of an oligonucleotide having a nucleotide sequence complementary to a target gene or a transcription product thereof, an aptamer comprising a nucleotide sequence binding specifically to a target protein, and a decoy consisting of an oligonucleotide having a nucleic acid sequence complementary to a target transcription factor.

9. The ligand-binding nucleic acid complex according to claim 1, wherein the nucleic acid molecule is selected from the group consisting of ADO, ASO, HDO, and RNAi.

10. The ligand-binding nucleic acid complex according to claim 9, wherein the antisense strand of the nucleic acid molecule consists of 12 to 30 continuous nucleotides.

11. The ligand-binding nucleic acid complex according to claim 8, wherein the nucleic acid molecule is a decoy comprising a nucleic acid sequence complementary to a target transcription factor and consisting of 8 to 30 nucleotides.

12. The ligand-binding nucleic acid complex according to claim 9, wherein the oligonucleotide is siRNA consisting of an antisense strand consisting of RNA and a nucleic acid strand complementary to the antisense strand.

13. The ligand-binding nucleic acid complex according to claim 9, wherein the nucleic acid strand of the nucleic acid molecule comprises nucleotides, modified nucleotides, and/or nucleotide analogs.

14. The ligand-binding nucleic acid complex according to claim 13, wherein the total number of nucleotides, modified nucleotides, and nucleotide analogs constituting the antisense strand of the nucleic acid molecule is 12 to 30.

15. The ligand-binding nucleic acid complex according to claim 13, wherein the nucleic acid molecule is HDO and the total number of nucleotides, modified nucleotides, and nucleotide analogs constituting the antisense strand and that constituting the complementary strand of HDO are each 12 to 30.

16. The ligand-binding nucleic acid complex according to claim 15, wherein the antisense strand is a gapmer and comprises a gap region comprising nucleotides and/or modified nucleotides and a wing region (or wing regions) comprising one or a plurality of nucleotide analogs and/or modified nucleotides provided on the 5' terminal side and/or 3' terminal side thereof.

17. The ligand-binding nucleic acid complex according to claim 15, wherein the antisense strand is a non-gapmer and comprises nucleotides, modified nucleotides, and/or nucleotide analogs.

18. The ligand-binding nucleic acid complex according to claim 17, wherein the complementary strand comprises nucleotides, modified nucleotides, and/or nucleotide analogs.

19. The ligand-binding nucleic acid complex according to claim 16, wherein the complementary strand comprises a center region comprising nucleotides and/or modified nucleotides and a wing region (or wing regions) comprising one or a plurality of nucleotide analogs and/or modified nucleotides provided on the 5' terminal side and/or 3' terminal side thereof.

20. The ligand-binding nucleic acid complex according to claim 13, wherein the modified nucleotides are nucleotides comprising a 2'-O-CH₃ group or a 2'-O-CH₂CH₂OCH₃ (MOE) group.

21. The ligand-binding nucleic acid complex according to claim 13, wherein the nucleotide analogs include bridged nucleotides selected independently from the group consisting of LNA, cEt-BNA, amide BNA (AmNA), and cMOE-BNA.

22. The ligand-binding nucleic acid complex according to claim 13, wherein the nucleotide analogs are selected independently from the group consisting of PNA, GNA, TNA, cEt, tcDNA, morpholino nucleic acid, BNA, GuNA, and scpBNA.

23. The ligand-binding nucleic acid complex according to claim 13, wherein at least one nucleotide or modified nucleotide in the nucleic acid molecule is phosphorothioated or boranophosphated.

24. A ligand-binding nucleic acid complex comprising an antisense strand to lower an expression level of a target transcription product in a subject's organ expressing the PTH1 receptor and a PTH1 ligand to deliver the antisense strand to the subject's organ, wherein the antisense strand comprises a base sequence capable of hybridizing to at least a part of a target transcription product and having antisense effects on the target transcription product.

25. The ligand-binding nucleic acid complex according to claim 24, wherein the nucleic acid complex is a double-stranded nucleic acid agent consisting of a first nucleic acid strand and a second nucleic acid strand, wherein the first nucleic acid strand comprises a base sequence capable of hybridizing to at least a part of a target transcription product and having antisense effects on the target transcription product, the second nucleic acid strand comprises a base sequence complementary to the first nucleic acid strand and binds to the PTH1 ligand, and the first nucleic acid strand is annealed to the second nucleic acid strand.

26. The ligand-binding nucleic acid complex according to claim 25, which is used for intravenous administration.
